# EUROPEAN PATENT SPECIFICATION

(11) **EP 1 594 434 B1**
(45) Date of publication and mention of the grant of the patent: **07.09.2016**
(21) Application number: 04702215.7
(22) Date of filing: 14.01.2004
(51) Int. Cl.: A61K 38/00, A61K 47/40, A61K 51/10

(54) **PARENTERAL FORMULATIONS OF PEPTIDES FOR THE TREATMENT OF SYSTEMIC LUPUS ERYTHEMATOSUS**
PARENTERALE PEPTIDFORMULIERUNGEN ZUR BEHANDLUNG VON SYSTEMISCHEM LUPUS ERYTHEMATODES
FORMULATIONS PARENTERALES DE PEPTIDES SERVANT A TRAITER UN LUPUS ERYTHEMATEUX SYSTEMIQUE

(30) Priority: 14.01.2003 US 439918 P
(43) Date of publication of application: 16.11.2005
(73) Proprietor: Yeda Research and Development Co., 76100 Rehovot (IL)
(72) Inventor: COHEN-VERED, Sharon, Kfar Saba (IL); NAFTALI, Esmira, Rosh Haayin 48056 (IL); WEINSTEIN, Vera, Mevaseret Zion 90805 (IL); GILBERT, Adrian, Ra'anana 43465 (IL); KLINGER, Ety, Tel Aviv 39930 (IL)
(74) Representative: D Young & Co LLP
(86) International application number: PCT/US2004/000948
(87) International publication number: WO 2004/064787

(56) References cited:
- WO-A-02/067848
- WO-A1-96/30057
- US-A- 5 134 127
- US-A- 5 730 969
- US-B1- 6 613 536
- UEDA H ET AL: "EVALUATION OF A SULFOBUTYL ETHER BETA-CYCLODEXTRIN AS A SOLUBILIZING/STABILIZING AGENT FOR SEVERAL DRUGS" DRUG DEVELOPMENT AND INDUSTRIAL PHARMACY, NEW YORK, NY, US, vol. 24, no. 9, 1 September 1998 (1998-09-01), pages 863-867, XP009040590 ISSN: 0363-9045

### SEQUENCE LISTING

<110> Cohen-Vered, et al., sharon
<120> PARENTERAL FORMULATIONS OF PEPTIDES FOR THE TREATMENT OF SYSTEMIC LUPUS
   ERYTHEMATOSUS
<130> 2609/68811-A
<140> 10/758,397
   <141> 2004-01-14
<160> 18
<170> PatentIn version 3.1
<210> 1
   <211> 20
   <212> PRT
   <213> Artificial Sequence
<220>
   <223> synthetic peptide based on CDR of mouse autoantibody
<400> 1
   Thr Gly Tyr Tyr Met Gln Trp Val Lys Gln Ser Pro Glu Lys Ser Leu
   1               5                   10                  15
   Glu Trp Ile Gly
               20
<210> 2
   <211> 20
   <212> PRT
   <213> Artificial sequence
<220>
   <223> Synthetic peptide based on CDR of mouse autoantibody
<400> 2
   Glu Ile Asn Pro Ser Thr Gly Gly Thr Thr Tyr Asn Gln Lys Phe Lys
   1               5                   10                  15
   Ala Lys Ala Thr
               20
<210> 3
   <211> 20
   <212> PRT
   <213> Artificial Sequence
<220>
   <223> Synthetic peptide based on CDR of mouse autoantibody
<400> 3
   Tyr Tyr Cys Ala Arg Phe Leu Trp Glu Pro Tyr Ala Met Asp Tyr Trp
   1               5                   10                  15
   Gly Gln Gly Ser
               20
<210> 4
   <211> 19
   <212> PRT
   <213> Artificial Sequence
<220>
   <223> Synthetic peptide based on CDR of mouse autoantibody
<400> 4
   Gly Tyr Asn Met Asn Trp Val Lys Gln Ser His Gly Lys Ser Leu Glu
   1               5                   10                  15
   Trp Ile Gly
<210> 5
   <211> 17
   <212> PRT
   <213> Artificial sequence
<220>
   <223> synthetic peptide based on CDR of mouse autoantibody
<400> 5
   Tyr Tyr Cys Ala Arg Ser Gly Arg Tyr Gly Asn Tyr Trp Gly Gln Thr
   1 5 10 15
   Leu
<210> 6
   <211> 19
   <212> PRT
   <213> Artificial sequence
<220>
   <223> Synthetic peptide based on CDR of mouse autoantibody
<400> 6
   Gly Tyr Tyr Trp Ser Trp Ile Arg Gln Pro Pro Gly Lys Gly Glu Glu
   1 5 10 15
   Trp Ile Gly
<210> 7
   <211> 22
   <212> PRT
   <213> Artificial Sequence
<220>
   <223> Synthetic peptide based on CDR of mouse autoantibody
<400> 7
   Tyr Tyr Cys Ala Arg Gly Leu Leu Arg Gly Gly Trp Asn Asp Val Asp
   1 5 10 15
   Tyr Tyr Gly Met Asp Val
   20
<210> 8
   <211> 7
   <212> PRT
   <213> Artificial sequence
<220>
   <223> Synthetic peptide based on CDR of mouse autoantibody
<400> 8
   Phe Ser Gly Tyr Tyr Trp Ser
   1 5
<210> 9
   <211> 16
   <212> PRT
   <213> Artificial Sequence
<220>
   <223> Synthetic peptide based on CDR of mouse autoantibody
<400> 9
   Glu Ile Asn His Ser Gly Ser Thr Asn Tyr Lys Thr Ser Leu Lys Ser
   1 5 10 15
<210> 10
   <211> 18
   <212> PRT
   <213> Artificial Sequence
<220>
   <223> Synthetic peptide based on CDR of mouse autoantibody
<400> 10
   Gly Leu Leu Arg Gly Gly Trp Asn Asp Val Asp Tyr Tyr Tyr Gly Met
   1 5 10 15
   Asp Val
<210> 11
   <211> 20
   <212> PRT
   <213> Artificial sequence
<220>
   <223> Synthetic peptide based on CDR of mouse autoantibody
<220>
   <221> MISC_FEATURE
   <222> (5)..(5)
   <223> x= Met, Ala or Val
<220>
   <221> MISC_FEATURE
   <222> (6)..(6)
   <223> X= Gln, Asp, Glu or Arg
<220>
   <221> MISC_FEATURE
   <222> (7)..(7)
   <223> X= Trp or Ala
<220>
   <221> MISC_FEATURE
   <222> (8)..(8)
   <223> X= Val or Ser
<220>
   <221> MISC_FEATURE
   <222> (9)..(9)
   <223> X= Lys, Glu or Ala
<400> 11
   Thr Gly Tyr Tyr Xaa Xaa Xaa Xaa Xaa Gln Ser Pro Glu Lys Ser Leu
   1               5                   10                  15
   Glu Trp Ile Gly
               20
<210> 12
   <211> 20
   <212> PRT
   <213> Artificial Sequence
<220>
   <223> Synthetic peptide based on CDR of mouse autoantibody
<220>
   <221> MISC_FEATURE
   <222> (9)..(9)
   <223> x= Thr, Val or Ala
<220>
   <221> MISC_FEATURE
   <222> (10)..(10)
   <223> X= Thr, val or Ala
<220>
   <221> MISC_FEATURE
   <222> (11)..(11)
   <223> X= Tyr or Phe
<220>
   <221> MISC_FEATURE
   <222> (12)..(12)
   <223> X= Asn or Asp
<220>
   <221> MISC_FEATURE
   <222> (13)..(13)
   <223> X =Gln or Glu
<220>
   <221> MISC_FEATURE
   <222> (14)..(14)
   <223> X= Lys or Glu
<220>
   <221> MISC_FEATURE
   <222> (15)..(15)
   <223> X= Phe or Tyr
<400> 12
   Glu Ile Asn Pro Ser Thr Gly Gly Xaa Xaa Xaa Xaa Xaa Xaa Xaa Lys
   1 5 10 15
   Ala Lys Ala Thr
   20
<210> 13
   <211> 20
   <212> PRT
   <213> Artificial sequence
<220>
   <223> Synthetic peptide based on CDR of mouse autoantibody
<220>
   <221> MISC_FEATURE
   <222> (6)..(6)
   <223> X= Phe, Thr or Gly
<220>
   <221> MISC_FEATURE
   <222> (7)..(7)
   <223> X= Leu, Ala or Ser
<220>
   <221> MISC_FEATURE
   <222> (8)..(8)
   <223> X= Trp or Ala
<220>
   <221> MISC_FEATURE
   <222> (9)..(9)
   <223> X= Glu or Lys
<220>
   <221> MISC_FEATURE
   <222> (13)..(13)
   <223> X= Met or Ala
<220>
   <221> MISC_FEATURE
   <222> (14)..(14)
   <223> X= Asp, Lys or Ser
<400> 13
   Tyr Tyr Cys Ala Arg Xaa Xaa Xaa Xaa Pro Tyr Ala Xaa Xaa Tyr Trp
   1 5 10 15
   Gly Gln Gly Ser
   20
<210> 14
   <211> 19
   <212> PRT
   <213> Artificial sequence
<220>
   <223> synthetic peptide based on CDR of mouse autoantibody
<220>
   <221> MISC_FEATURE
   <222> (4)..(4)
   <223> X= Met or Ala
<220>
   <221> MISC_FEATURE
   <222> (S)..(S)
   <223> X= Asn, Asp or Arg
<220>
   <221> MISC_FEATURE
   <222> (6)..(6)
   <223> X= Trp or Ala
<220>
   <221> MISC_FEATURE
   <222> (7)..(7)
   <223> X= Val or Ser
<220>
   <221> MISC_FEATURE
   <222> (8)..(8)
   <223> X= Lys or Glu
<220>
   <221> MISC_FEATURE
   <222> (9)..(9)
   <223> X= Gln or Ala
<220>
   <221> MISC_FEATURE
   <222> (13)..(13)
   <223> X= Lys or Glu
<220>
   <221> MISC_FEATURE
   <222> (14)..(14)
   <223> X= Ser or Ala
<400> 14
   Gly Tyr Asn Xaa Xaa Xaa Xaa Xaa Xaa Ser His Gly Xaa Xaa Leu Glu
   1               5                   10                  15
   Trp Ile Gly
<210> 15
   <211> 17
   <212> PRT
   <213> Artificial sequence
<220>
   <223> Synthetic peptide based on CDR of mouse autoantibody
<220>
   <221> MISC_FEATURE
   <222> (6)..(6)
   <223> X= Ser or Phe
<220>
   <221> MISC_FEATURE
   <222> (7)..(7)
   <223> X= Gly or Ala
<220>
   <221> MISC_FEATURE
   <222> (8)..(8)
   <223> X= Arg, Ala or Glu
<220>
   <221> MISC_FEATURE
   <222> (11)..(11)
   <223> X= Asn or Asp
<220>
   <221> MISC_FEATURE
   <222> (12)..(12)
   <223> X= Tyr or Phe
<220>
   <221> MISC_FEATURE
   <222> (13)..(13)
   <223> x= Trp, His or Ala
<400> 15
   Tyr Tyr Cys Ala Arg Xaa Xaa Xaa Tyr Gly Xaa Xaa Xaa Gly Gln Thr
   1 5 10 15
   Leu
<210> 16
   <211> 19
   <212> PRT
   <213> Artificial Sequence
<220>
   <223> Synthetic peptide basedon CDR of mouse autoantibody
<220>
   <221> MISC_FEATURE
   <222> (1)..(1)
   <223> X= Gly or Thr Gly
<220>
   <221> MISC_FEATURE
   <222> (8)..(8)
   <223> X= Arg or Lys
<220>
   <221> MISC_FEATURE
   <222> (10)..(10)
   <223> X= Pro or Ser
<220>
   <221> MISC_FEATURE
   <222> (12)..(12)
   <223> X= Gly or Glu
<220>
   <221> MISC_FEATURE
   <222> (13)..(13)
   <223> X= Lys or Asp
<220>
   <221> MISC_FEATURE
   <222> (15)..(15)
   <223> X= Glu, Leu or Ser
<400> 16
   Xaa Tyr Tyr Trp Ser Trp Ile Xaa Gln Xaa Pro Xaa Xaa Gly Xaa Glu
   1               5                   10                  15
   Trp Ile Gly
<210> 17
   <211> 22
   <212> PRT
   <213> Artificial Sequence
<220>
   <223> Synthetic peptide basedon CDR of mouse autoantibody
<220>
   <221> MISC_FEATURE
   <222> (6)..(6)
   <223> X= Gly or Phe
<220>
   <221> MISC_FEATURE
   <222> (9)..(9)
   <223> X= Arg or Ala
<220>
   <221> MISC_FEATURE
   <222> (10)..(10)
   <223> X= Gly or Ala
<220>
   <221> MISC_FEATURE
   <222> (11)..(11)
   <223> X= Gly or Ala
<220>
   <221> MISC_FEATURE
   <222> (12)..(12)
   <223> X= Trp or Ala
<220>
   <221> MISC_FEATURE
   <222> (13)..(13)
   <223> X= Asn or Ala
<220>
   <221> MISC_FEATURE
   <222> (18)..(18)
   <223> X= Tyr or Trp
<220>
   <221> MISC_FEATURE
   <222> (20)..(20)
   <223> X= Met or Gln
<400> 17
   Tyr Tyr Cys Ala Arg Xaa Leu Leu Xaa Xaa Xaa Xaa Xaa Asp Val Asp
   1               5                   10                  15
   Tyr Xaa Gly Xaa Asp Val
               20
<210> 18
   <211> 19
   <212> PRT
   <213> Artificial Sequence
<220>
   <223> Synthetic peptide of 19 amino acids based on the complementarity-determining region 1 (CDR1) of human anti-dsDNA mAb denoted 16/6 Id
<400> 18
   Gly Tyr Tyr Trp Ser Trp Ile Arg Gln Pro Pro Gly Lys Gly Glu Glu
   1               5                   10                  15
   Trp Ile Gly

## Description

### Background of the Invention

Systemic lupus erythematosus (SLE), or lupus, is a debilitating autoimmune disease characterized by the presence of an array of autoantibodies, including antibodies to dsDNA, to nuclear antigens and to ribonucleoproteins. SLE affects approximately 1 in 2000 individuals (U.S. 1 in 700 women). The disease primarily affects young women, with a female-to male ratio of approximately 9:1.

Systemic lupus can affect almost any organ or system of the body. Systemic lupus may include periods in which few, if any, symptoms are evident ("remission") and other times when the disease becomes more active ("flare"). Most often when people mention "lupus," they are referring to the systemic form of the disease.

Corticosteroids are the mainstay in treating systemic autoimmune disorders. Life threatening, severely disabling manifestations of SLE are treated with high doses of glucocorticoids (1-2 mg/kg/day). Undesirable effects of chronic glucocorticoids include an array of prominent adverse effects such as cushingoid habitus, central obesity, hypertension, infection, capillary fragility, hirsutism, accelerated osteoporosis, cataracts, diabetes mellitus, myopathy and psychosis. In addition to corticosteroid toxicity, patient compliance to a dosage regimen also poses a serious problem.

Cytotoxic agents are also used for controlling active disease, reducing the rate of disease flares, and reducing steroid requirements. Undesirable side effects of the latter include bone marrow depression, increased infections with opportunistic organisms, irreversible ovarian failure, alopecia and increased risk of malignancy.

SLE is an inflammatory disease for which to date there is no definitive treatment or cure. The disease results in acute and chronic complications. The only treatments available are palliative, aimed at relieving acute symptoms and preventing chronic complications, often with profound side effects. There is therefore an unmet need in this field, and both physicians and patients would welcome new treatments which could potentially eliminate or reduce the unwanted manifestations of the disease.

Despite the extensive research on the mechanisms underlying the induction of SLE, the information on the etiology of the disease is very limited. Studies on SLE have been performed until recently using peripheral blood lymphocytes (PBL) of patients at different clinical stages and under various treatment protocols. Alternatively, murine strains that develop spontaneous SLE-like disease were investigated as a model for SLE. This kind of analysis led to incomplete and confusing interpretations of the role of various immunological and non-immunological factors in either inducing or sustaining the disease, mainly due to the heterogeneity of patients on one hand and the inability to control the induction phase of the disease in murine SLE strains on the other hand.

Several years ago, an animal model of SLE was established. This model, based on the concept of the idiotypic network, developed a wide spectrum of lupus-related autoantibodies and clinical manifestations (Mendlovic, S. et al. Proc. Natl. Acad Sci. USA 85: 2260 (1988)). The induction was carried out by the immunization of mouse strains that do not develop any spontaneous autoimmune disorders, with a human anti-DNA monoclonal antibody (mAb) which bears a common idiotype termed 16/6 Id (Shoenfeld, Y. et al., J. Exp. Med. 158: 718 (1983)).

The human mAB 16/6 is an anti-DNA antibody originally of the IgM isotype and switched in culture to IgG1. The mAB was derived from a patient and expresses a common idiotype, the 16/6 Id (Shoenfeld et al., 1983; Mendlovic et al., 1988). The hybridoma cells secreting this mAB are routinely grown in culture, and the antibody is isolated from culture supernatants using an affinity column of Protein G coupled to Sepharose™. The human 16/6 anti-DNA mAB (IgG1/κ) was described in Shoenfeld, Y. et al., J. Clin. Invest. 70: 205-208 (1982) and in Waisman, A. et al., Int. Immunol. 7: 689-696 (1995).

Following immunization, the mice produced antibodies specific to the 16/6 Id, antibodies that bear the 16/6 Id and antibodies directed against different nuclear antigens (dsDNA, ssDNA, Sm, ribonucleoprotein (RNP), Ro, La and others). The serological findings were associated with leukopenia, elevated erythrocyte sedimentation rate, proteinuria, abundance of immune complexes in the kidneys and sclerosis of the glomeruli (Mendlovic et al., 1988), which are typical manifestations of SLE. It was further shown that the experimental disease could be induced by a murine anti-16/6 Id mAb (Mendlovic, S. et al., Eur. J. Immunol. 19: 729, (1989)) and by the mouse anti-anti 16/6 Id (16/6 Id+) mAb (Waisman, A. et al., Internatl. Immunol. 5:1293 (1993); Waisman, A. and Mozes, E. Eur. J. Immunol. 23: 1566, (1993)). The induction of the disease is genetically controlled, and thus is strain dependent (Mendlovic, S. et al., Immunology 69: 228 (1990)). This unique model for the induction of experimental SLE provides the appropriate tools to clearly dissect the different steps and the linked immune parameters involved in the induction and development of SLE.

SLE is a systemic autoimmune disease characterized by the formation of auto antibodies against self-antigens, such as DNA, Sm, Ro, La, RNP, cardiolipin and histones. The etiology of SLE is unknown, and understanding the mechanism by which these self-antibodies arise provided insight. Monoclonal autoantibodies that were capable of eliciting antibodies that bear the 16/6 Id or react with it were found to be pathogenic and thus capable of inducing experimental SLE (Fricke, H. et al., Internatl. Immunol. 2: 225 (1990); Sthoeger, Z.M. et al., J. Clin. Immunol. 13: 127 (1993)). The variable (V) regions of nine autoantibodies that bind either DNA or HeLa nuclear extract (NE), isolated from the C3H.SW mice with experimental SLE, were sequenced (Waisman and Mozes, 1993). Monoclonal antibodies with different specificity were analyzed in an attempt to determine the connections between the different autoantibodies. Three mAb were found to bind DNA, and were shown to exhibit sequence characteristics of pathogenic anti-DNA antibodies. One of these mAb, designated 2C4C2, was shown to use a heavy (H) chain V region gene (V_{H}) identical to the V_{H} of anti-DNA mAb isolated from other lupus-prone mice, namely (NZB xNZW)F₁. The light (L) chain V region gene (V_{L}) of mAb 2C4C2 is 98% homologous to the V_{L} of another anti-DNA mAb, also isolated from (NZB xNZW)F₁ mice. The other two anti-DNA mAb, designated 5G12-4 and 5G12-6, share 93% of their V_{H} sequences with that of mAb 2C4C2. Six mAb bound proteins of HeLa NE. The nine mAb use a total of five V_{H} and four V_{L} germ-line genes, demonstrating that the autoantibodies induced in mice with experimental SLE do not originate from one B cell clone. Three of the nine V_{H} and V_{L} were identical in sequence to germ-line genes, while at least three others had somatic mutations. The latter suggests that these autoantibodies arise in mice by both usage of existing (pre-immune) B cells, and through anantigen-driven process. Furthermore, it appears that autoantibodies found in mice with experimental SLE use genetic elements similar to those used by mAb that were isolated from mouse strains which develop lupus spontaneously.

T cells play an important role in the induction and development of experimental SLE. Thus, T cell lines and clones specific to the 16/6 Id were shown to induce experimental SLE in syngeneic recipients similarly to the 16/6 antibody. Therefore, following the inoculation of the activated cells of the lines, the mice developed both the serology and the renal damage which is typical to SLE (Fricke, H. et al., Immunology 73: 421 (1991)). Furthermore, a 16/6 Id specific T cell line of C3H.SW origin induced SLE in C57BL/6 mice that were shown to be resistant to the induction of the disease following injections with either the 16/6 Id or the anti-16/6Id mAb (Mendlovic et al., 1990).

In an attempt to identify the pathogenic region of the 16/6 Id, (Fab')₂ fragments were prepared of the 16/6 Id mAb and were found to retain the specificity and pathogenic capacity of the whole 16/6 Id molecule (Ruiz, P.J. et al., Immun. Let. 41: 79 (1994)).

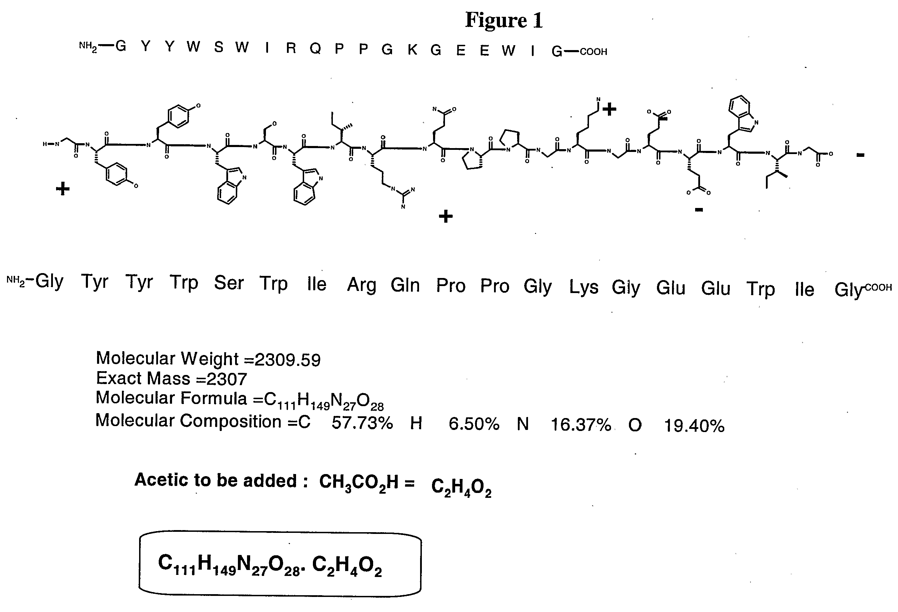

The mAb 5G12 that was isolated from mice with experimental SLE and was shown to bind DNA and bear the 16/6. Id, is capable of inducing experimental SLE in mice (Waisman, A. et al., Internatl. Immunol. 5:1293 (1993)). T cells that react specifically to mAb by proliferation, are probably reacting to peptides representing sequences from their complementarity-determining regions (CDR). It is very likely that the T cells recognize the V regions of the above antibodies since they do not react with other antibodies that carry the same constant region but have different specificities. Within the variable region, the regions with the highest probability to be recognized are the CDR, since those are the regions that differ the most between the various antibodies. The CDR regions of the VH sequences of the nine pathogenic murine mAb mentioned above that induce SLE in mice, are boxed in Fig. 1 of Waisman and Mozes, 1993, in which the complete nucleotide and deduced amino acid sequences for the VH of the nine mAb are presented.

In experimental SLE models - Balb/c mice and SLE-prone mice, i.e. (NZBxNZW)F1 mice - treatment with either mCDR based-peptides or Compound 1 significantly reduced the SLE related findings, notably immune complex deposits (ICD) in the kidney, proteinuria and leukopenia. The treatment had no effect on the 16/6 Id specific antibody response (Waisman, A., et al. "Modulation of murine systemic lupus erythematosus with peptides based on complementarity determining regions of pathogenic anti -DNA monoclonal antibodies." Proc. Natl. Acad. Sci. U.S.A. (1997), 94(4): 620; Eilat, E., et al., "Prevention of systemic lupus erythematosus-like disease in (NZBxNZW)F1 mice by treating with CDR1- and CDR3- based peptides of pathogenic autoantibody" J. Clin. Immunol. (2000), 20: 268; Eilat, E., et al., "The mechanism by which a peptide based on complementarity determining region-1 of pathogenic anti-DNA antibody ameliorates experimental SLE" (2001), Proc.Natl.Acad. Sci. U.S.A. 98: 1148).

Human CDR1, Compound 1, shown in Figure 1, is a synthetic peptide of 19 amino acids based on the complementarity-determining region 1 (CDR1) of the human anti-dsDNA mAb denoted 16/6 Id (Waisman, A., et al. "Modulation of murine systemic lupus erythematosus with peptides based on complementarity determining regions of pathogenic anti-DNA monoclonal antibodies." Proc. Natl. Acad. Sci. U.S.A. (1997), 94(4): 4620-4625).

WO02/067848 A2 discloses a pharmaceutical composition comprising synthetic peptides of at least 12 and at most 30 amino acids residues, including peptides having the sequences as denoted by SEQ ID NO: 6-10,16,17 with a pharmaceutically acceptable carrier for parenteral administration. It also discloses the use thereof for treating Systemic Lupus Erythematosus (SLE).

WO9630057 A1 discloses a pharmaceutical composition comprising synthetic peptides of at least 12 and at most 30 amino acids residues, including peptides having the sequences as denoted by SEQ ID NO: 1-5,11-15 with a pharmaceutically acceptable carrier for parenteral administration. It also discloses the use thereof for treating Systemic Lupus Erythematosus (SLE).

These peptides, like many peptides, are not very soluble. Therefore, formulations that improve the solubility of the peptides are provided herein.

### Summary of Invention

The subject invention provides a pharmaceutical composition comprising:
an aqueous carrier;
from 0.1 mg/ml to 20 mg/ml of the composition of a pharmaceutically acceptable salt of
   a peptide comprising consecutive amino acids having the sequence
      (i) TGYYX₁X₂X₃X₄X_{SQ}SPEKSLEWIG (SEQ ID NO:11) wherein X₁ is Met, Ala or Val; X₂ is Gln, Asp, Glu or Arg; X₃ is Trp or Ala; X₄ is Val or Ser; and X₅ is Lys, Glu or Ala;
      (ii) EINPSTGGX₆X₇X₈X₉X₁₀X₁₁X₁₂KAKAT (SEQ ID NO:12) wherein X₆ and X₇ are each Thr, Val or Ala; X₈ is Tyr or Phe; X₉ is Asn or Asp; X₁₀ is Gln or Glu; X₁₁ is Lys or Glu, and X₁₂ is Phe or Tyr;
      (iii) YYCARX₁₃X₁₄X₁₅X₁₆PYAX₁₇X₁₈YWGQGS (SEQ ID NO:13) Ser; X₁₅ is Trp or Ala; X₁₆ is Glu or Lys; X₁₇ is Met or Ala, and X₁₈ is Asp, Lys or Ser;
      (iv) GYNX₁₉X₂₀X₂₁X₂₂X₂₃X₂₄SHGX₂₅X₂₆LEWIG (SEQ ID NO:14) wherein X₁₉ is Met or Ala; X₂₀ is Asn, Asp or Arg; X₂₁ is Trp or Ala; X₂₂ is Val or Ser; X₂₃ is Lys or Glu; X₂₄ is Gln or Ala; X₂₅ is Lys or Glu, and X₂₆ is Ser or Ala;
      (v) YYCARX₂₇X₂₈X₂₉YGX₃₀X₃₁X₃₂GQTL (SEQ ID NO:15) wherein X₂₇ is Ser or Phe; X₂₈ is Gly or Ala; X₂₉ is Arg, Ala or Glu; X₃₀ is Asn or Asp; X₃₁ is Tyr or Phe, and X₃₂ is Trp, His or Ala;
      (vi) X₃₃YYWSWIX₃₄QX₃₅PX₃₆X₃₇GX₃₈EWIG (SEQ ID NO:16) wherein X₃₃ is Gly or Thr Gly; X₃₄ is Arg or Lys; X₃₅ is Pro or Ser; X₃₆ is Gly or Glu; X₃₇ is Lys or Asp; and X₃₈ is Glu, Leu or Ser;
      (viii) FSGYYWS (SEQ ID NO:8);
      (ix) EINHSGSTNYKTSLKS (SEQ ID NO:9); or
      (x) GLLRGGWNDVDYYYGMDV (SEQ ID NO:10),
      and a substituted β-cyclodextrin as a solubility enhancer, and,
   wherein both the peptide and the solubility enhancer are dissolved in the aqueous carrier; and
   wherein the composition has a pH between 4 and 9.

The subject invention also provides a method of alleviating symptoms of systemic lupus erythematosus (SLE) in a human subject comprising administering to the human subject any of the pharmaceutical compositions of the invention in an amount effective to alleviate the symptoms of SLE in the human subject.

### Brief Description of Figures

**Figure 1****.** Human CDR1 (Compound 1) as acetate salt - showing the molecular and structural formulas of hCDR1, the amino acid sequence, and physical parameters
**Figure 2****.** IL-2 Secretion from cells taken from mice treated with Compound 1 and Captisol® solution after the cells were subsequently activated with a solution of Compound 1 in PBS.
   -■- Compound 1 (RS) 50µg/mouse
   -▲- Compound 1 (RS) 200µg/mouse
   -□- DP 50µg/mouse
   -Δ- DP 200µg/mouse
   -●- Captisol® ampulized
**Figure 3****.** IFN-γ Secretion from cells taken from mice treated with Compound 1 solution after the cells were subsequently activated with a solution of compound 1 in EM-1 (2.5 x 10⁶ cells/well).
   **-◆-** Placebo
   -●- Compound 1 50 µg/mouse (treatment dose)
   -Δ- Compound 1 100 µg/mouse (treatment dose)
   -X- Compound 1 200 µg/mouse (treatment dose)
**Figure 4****.** IFN-γ Secretion from cells taken from mice treated with Compound 1 solution after the cells were subsequently activated with a solution of compound 1 in EM-1 (5 x 10⁶ cells/well).
   -◆- Placebo
   -□- Compound 1 25 µg/mouse
   -Δ- Compound 1 50 µg/mouse
   -X- Compound 1 100 µg/mouse
   -*- Compound 1 200 µg/mouse
**Figure 5****.** Anti-dsDNA antibodies in (NZBxNZW)F1 mice after 10 injections with Compound 1 in Captisol^{®} [OD=Optical Density; Compound 1 (C)= Compound 1 dissolved in Captisol^{®}]
   -□- Placebo
   -◊- Compound 1 50 µg/mouse
   -○- Compound 1 25 µg/mouse
**Figure 6****.** Kidney sections from (NZBxNZW)F1 mice showing intensity of Immune Complex Deposits. The top row sections are from a Captisol®-treated mouse, the mid-row sections are from a mouse treated with 50 µg/mouse Compound 1 and the bottom row sections are from a mouse treated with 25 µg/mouse Compound 1. *Magnification:* Left: x100, Right: x400. FITC immunohistology.
**Figure 7****.** Antibody titers in sera of SLE patients and healthy human controls by testing their sera for the ability to bind the peptides Ia, IIa and IIIa, or mAb 5G12 or a control peptide.
**Figure 8****.** Concentrations of the human anti-DNA 16/6 Id mAb required for optimal stimulation of PBL of SLE patients and of healthy controls. PBL were stimulated with various concentrations (0.1-40 µg/well) of the 16/6 Id mAb. The concentration yielding the highest stimulation index was defined as optimal for triggering a proliferative response.
**Figure 9****.** Proliferation of PBL from one SLE patient stimulated with the mitogen phytohemagglutinin (PHA) in the absence or presence of hCDR1 or hCDR3.
**Figure 10****.** Proliferation of PBL from one SLE patient stimulated with human 16/6I mAb in the absence or presence of the human peptides hCDR1 or hCDR3 or the murine peptide mCDR3.
**Figure 11****.** Proliferation of PBL from one SLE patient stimulated with Human 16/6I mAb in the absence or presence of the human peptides hCDR1 or hCDR3 or the murine reversed peptides revmCDR1 and revmCDR3.
**Figure 12****.** Inhibition of IL-2 secretion in PBL of SLE patients triggered by the human 16/6Id mAb in the absence or presence of hCDR1 or hCDR3.
**Figure 13****.** Up-regulation of TGF-β secretion in the PBL of one representative SLE patient stimulated with the human 16/6Id mAb in the absence or presence of hCDR1 or hCDR3.
**Figure 14****.** Representative gel showing activity of MMP-2 and MMP-9 in sera of SLE patients and healthy controls. Sera (5 µl) of 40 individual SLE patients and 25 healthy controls were analysed for their MMP-2 or MMP-9 activities by gel zymography. The figure shows representative results with serum samples of the two groups.
**Figure 15****.** Graph showing quantitative analysis of MMP-2 and MMP-9 activities in sera of SLE patients (dark columns) and healthy controls (white columns). Thirty-six serum samples of SLE patients and 15 serum samples of healthy controls were tested for MMP-2 or MMP-9 activity using specific activity assay kits. Results are expressed as the mean ± s.e.m. *P = 0.0302.
**Figures 16A-B.** Graphs showing MMP-9 activity levels and disease activity indices (SLEDAI) in patients with SLE. Thirty-five serum samples from 8 male (Fig. 16A) and 27 female (Fig. 16B) SLE patients were tested for MMP-9 activity by a specific activity assay kit. The distribution of MMP-9 activity according to the SLEDAI of the patients is presented. The dashed line represents the activity of MMP-9 in healthy controls.
**Figures 17A-B.** Graphs showing pattern of MMP-2 (white circles) and MMP-9 (black circles) activities in sera of two SLE patients sampled during 4-6 years of disease. The sera were tested for MMP-2 or MMP-9 activities by specific activity assay kits. The assays were performed in duplicate.

### Detailed description

The subject invention provides a pharmaceutical composition comprising:
an aqueous carrier;
from 0.1 mg/ml to 20 mg/ml of the composition of a pharmaceutically acceptable salt of a peptide comprising consecutive amino acids having the sequence
   (i) TGYYX₁X₂X₃X₄X₅QSPEKSLEWIG (SEQ ID NO:11)
      wherein X₁ is Met, Ala or Val; X₂ is Gln, Asp, Glu or Arg; X₃ is Trp or Ala; X₄ is Val or Ser; and X₅ is Lys, Glu or Ala;
   (ii) EINPSTGGX₆X₇X₅X₉X₁₀X₁₁X₁₂KAKAT (SEQ ID NO:12) wherein X₆ and X₇ are each Thr, Val or Ala; X₈ is Tyr or Phe; X₉ is Asn or Asp; X₁₀ is Gln or Glu; X₁₁ is Lys or Glu, and X₁₂ is Phe or Tyr;
   (iii) YYCARX₁₃X₁₄X₁₅X₁₆PYAX₁₇X₁₈YWGQGS (SEQ ID NO:13) wherein X₁₃ is Phe, Thr or Gly; X₁₄ is Leu, Ala or Ser; X₁₅ is Trp or Ala; X₁₆ is Glu or Lys; X₁₇ is Met or Ala, and X₁₈ is Asp, Lys or Ser;
   (iv) GYNX₁₉X₂₀X₂₁X₂₂X₂₃X₂₄SHGX₂₅X₂₆LEWIG (SEQ ID NO:14) wherein X₁₉ is Met or Ala; X₂₀ is Asn, Asp or Arg; X₂₁ is Trp or Ala; X₂₂ is Val or Ser; X₂₃ is Lys or Glu; X₂₄ is Gln or Ala; X₂₅ is Lys or Glu, and X₂₆ is Ser or Ala;
   (v) YYCARX₂₇X₂₈X₂₉YGX₃₀X₃₁X₃₂GQTL (SEQ ID NO:15) wherein X₂₇ is Ser or Phe; X₂₈ is Gly or Ala; X₂₉ is Arg, Ala or Glu; X₃₀ is Asn or Asp; X₃₁ is Tyr or Phe, and X₃₂ is Trp, His or Ala;
   (vi) X₃₃YYWSWIX₃₄QX₃₅PX₃₆X₃₇GX₃₈EWIG (SEQ ID NO:16) wherein X₃₃ is Gly or Thr Gly; X₃₄ is Arg or Lys; X₃₅ is Pro or Ser; X₃₆ is Gly or Glu; X₃₇ is Lys or Asp; and X₃₈ is Glu, Leu or Ser;
   (viii) FSGYYWS (SEQ ID NO:8);
   (ix) EINHSGSTNYKTSLKS (SEQ ID NO:9); or
   (x) GLLRGGWNDVDYYYGMDV (SEQ ID NO:10),
   and a substituted β-cyclodextrin as solubility enhancer, and,
wherein both the peptide and the solubility enhancer are dissolved in the aqueous carrier; and
wherein the composition has a pH between 4 and 9.

In one embodiment, at least 0.5 mg/ml of the composition is the pharmaceutically acceptable salt of the peptide.

In another embodiment, the peptide has a sequence selected from the group consisting of:
NH₂- Thr Gly Tyr Tyr Met Gin Trp Val Lys Gin Ser Pro Glu Lys Ser Leu Glu-Trp Ile Gly-COOH (SEQ ID NO:1);
NH₂- Glu Ile Asn Pro Ser Thr Gly Gly Thr Thr Tyr Asn Gln Lys Phe Lys Ala Lys Ala Thr-COOH (SEQ ID NO:2);
NH₂- Tyr Tyr Cys Ala Arg Phe Leu Trp Glu Pro Tyr Ala Met Asp Tyr Trp Gly Gln Gly Ser-COOH (SEQ ID NO:3);
NH₂- Gly Tyr Asn Met Asn Trp Val Lys Gln Ser His Gly Lys Ser Leu Glu Trp Ile Gly-COOH (SEQ ID NO:4);
NH₂- Tyr Tyr Cys Ala Arg Ser Gly Arg Tyr Gly Asn Tyr Trp Gly Gln Thr Leu -COOH (SEQ ID NO:5);
NH₂ - Gly Tyr Tyr Trp Ser Trp Ile Arg Gln Pro Pro Gly Lys Gly Glu Glu Trp Ile Gly-COOH (SEQ ID NO:6);
NH₂- Phe Ser Gly Tyr Tyr Trp Ser-COOH (SEQ ID NO: 8);
NH₂- Glu Ile Asn His Ser Gly Ser Thr Asn Tyr Lys Thr Ser Leu Lys Ser-COOH (SEQ ID NO:9); and
NH₂- Gly Leu Leu Arg Gly Gly Trp Asn Asp Val Asp Tyr Tyr Tyr Gly Met Asp Val-COOH (SEQ ID NO:10).

In one embodiment, the peptide comprises consecutive amino acids having the sequence
X₃₃YYWSWIX₃₄QX₃₅PX₃₆X₃₇GX₃₈EWIG (SEQ ID NO:16)
   wherein X₃₃ is Gly or Thr Gly; X₃₄ is Arg or Lys; X₃₅ is Pro or Ser; X₃₆ is Gly or Glu; X₃₇ is Lys or Asp; and X₃₈ is Glu, Leu or Ser.

In one embodiment, the substituted β-cyclodextrin is a hydroxypropyl, a sulfobutyl ether, or asulfopropyl ether substituted β-cyclodextrin.

In another embodiment, the substituted β-cyclodextrin is a substituted sulfobutyl ether β-cyclodextrin.

In a further embodiment of any of the above compositions, the concentration of peptide in solution is at least 1 mg/ml.

In a further embodiment, the concentration of peptide in solution is at least 2.5 mg/ml.

In one embodiment, the concentration of the salt of the peptide is from 0.5 mg/ml to 10 mg/ml.

In another embodiment, the concentration of the salt of the peptide is from 0.5 mg/ml to 2.5 mg/ml.

In another embodiment, the concentration of the salt of the peptide is from 2.5 mg/ml to 5 mg/ml.

In another embodiment, the concentration of the salt of the peptide is from 5 mg/ml to 7 mg/ml.

In another embodiment, the concentration of the salt of the peptide is from 7 mg/ml to 8.5 mg/ml.

In another embodiment, the concentration of the salt of the peptide is from 8.5 mg/ml to 10 mg/ml.

In another embodiment, the concentration of the salt of the peptide is from 9 mg/ml to 10 mg/ml.

In another embodiment, the concentration of the salt of the peptide is from 10 mg/ml to 15 mg/ml.

In another embodiment, the concentration of the salt of the peptide is from 15 mg/ml to 20 mg/ml.

In another embodiment, the concentration of the salt of the peptide is 1.0 mg/ml.

In another embodiment, the concentration of the salt of the peptide is 2.5 mg/ml.

In another embodiment, the concentration of the salt of the peptide is 5 mg/ml.

In another embodiment, the concentration of the salt of the peptide is 10 mg/ml.

In another embodiment, the concentration of the salt of the peptide is 15 mg/ml.

In another embodiment, the concentration of the salt is from 0.1 mg/ml to 0.5 mg/ml.

In another embodiment, the concentration of the salt is from 0.1 mg/ml to 0.2 mg/ml.

In another embodiment, the concentration of the salt is from 0.2 mg/ml to 0.3 mg/ml.

In another embodiment, the concentration of the salt is from 0.3 mg/ml to 0.4 mg/ml.

In another embodiment, the concentration of the salt is from 0.4 mg/ml to 0.5 mg/ml.

In a further embodiment, the composition has a pH between 6.5 and 8.5.

In a further embodiment, the composition has a pH between 7.5 and 8.5.

In a further embodiment, the composition has a pH between 4 and 5.

In a further embodiment, the composition has a pH between 5 and 6.

In a further embodiment, the composition has a pH between 6 and 7.

In a further embodiment, the composition has a pH between 7 and 8.

In a further embodiment, the composition has a pH between 8 and 9.

In another embodiment of any of the above pharmaceutical compositions, the pharmaceutically acceptable salt is an acetate salt.

In a further embodiment, the pharmaceutically acceptable salt is an acetate salt, and the substituted β-cyclodextrin is hepta-(sulfobutyl ether)-β-cyclodextrin.

In another embodiment, the composition further comprises a pharmaceutically acceptable buffer in an amount and of a type suitable to make the pH of the pharmaceutical composition in the range of 4-9. The buffer may be acetate buffer, citrate buffer, or sodium carbonate.

The subject invention also provides a method of alleviating symptoms of systemic lupus erythematosus (SLE) in a human subject comprising administering to the human subject any of the above pharmaceutical compositions in an amount effective to alleviate the symptoms of the SLE in the human subject.

The subject invention also provides any of the above pharmaceutical compositions for use in treating SLE in a human subject.

The subject invention also provides a process for manufacturing any of the above pharmaceutical compositions comprising the steps of:
a) preparing a solution of substituted β-cyclodextrin in an aqueous carrier at a predetermined concentration;
b) adding a predetermined amount of a pharmaceutically acceptable salt of
   a peptide comprising amino acids having the sequence
      (i) TGYYX₁X₂X₃X₄X₅QSPEKSLEWIG (SEQ ID NO:11) wherein X₁ is Met, Ala or Val; X₂ is Gln, Asp, Glu or Arg; X₃ is Trp or Ala; X₄ is Val or Ser; and X₅ is Lys, Glu or Ala;
      (ii) EINPSTGGX₆X₇X₉X₉Xₗ₀XₙX₁₂KAKAT (SEQ ID NO:12) wherein X₆ and X₇ are each Thr, Val or Ala; X₈ is Tyr or Phe; X₉ is Asn or Asp; X₁₀ is Gln or Glu; X₁₁ is Lys or Glu, and X₁₂ is Phe or Tyr;
      (iii) YYCARX₁₃X₁₄X₁₅X₁₆PYAX₁₇X₁₈YWGQGS (SEQ ID NO:13) wherein X₁₃ is Phe, Thr or Gly; X₁₄ is Leu, Ala or Ser; X₁₅ is Trp or Ala; X₁₆ is Glu or Lys; X₁₇ is Met or Ala, and X₁₈ is Asp, Lys or Ser;
      (iv) GYNX₁₉X₂₀X₂₁X₂₂X₂₃X₂₄SHGX₂₅X₂₆LEWIG (SEQ ID NO:14) wherein X₁₉ is Met or Ala; X₂₀ is Asn, Asp or Arg; X₂₁ is Trp or Ala; X₂₂ is Val or Ser; X₂₃ is Lys or Glu; X₂₄ is Gln or Ala; X₂₅ is Lys or Glu, and X₂₆ is Ser or Ala;
      (v) YYCARX₂₇X₂₈X₂₉YGX₃₀X₃₁X₃₂GQTL (SEQ ID NO:15) wherein X₂₇ is Ser or Phe; X₂₈ is Gly or Ala; X₂₉ is Arg, Ala for Glu; X₃₀ is Asn or Asp; X₃₁ is Tyr or Phe, and X₃₂ is Trp, His or Ala;
      (vi) X₃₃YYWSWIX₃₄QX₃₅PX₃₆X₃₇GX₃₈EWIG (SEQ ID NO:16) wherein X₃₃ is Gly or Thr Gly; X₃₄ is Arg or Lys; X₃₅ is Pro or Ser; X₃₆ is Gly or Glu; X₃₇ is Lys or Asp; and X₃₈ is Glu, Leu or Ser;
      (viii) FSGYYWS (SEQ ID NO:8);
      (ix) EINHSGSTNYKTSLKS (SEQ ID NO:9); or
      (x) GLLRGGWNDVDYYYGMDV (SEQ ID NO:10),
c) adjusting the pH of the solution of step b) until the peptide dissolves in the solution; and
d) if necessary, adjusting the pH of the solution of step c) to a pH of 4-9, thereby manufacturing the pharmaceutical composition.

In one embodiment, the predetermined amount of peptide is such which results in a final concentration of peptide in the pharmaceutical composition of at least 0.1 mg/ml.

In another embodiment, the predetermined amount of peptide is such which results in a final concentration of peptide in the pharmaceutical composition of at least 0.5 mg/ml.

In a further embodiment, the predetermined amount of peptide is such which results in a final concentration of peptide in the pharmaceutical composition of 2.5mg/ml, 2.0mg/ml, 1.0mg/ml, 0.5 mg/ml or 0.1 mg/ml.

In another embodiment, the predetermined amount of peptide is such which results in a final concentration of peptide in the pharmaceutical composition is 5 mg/ml, 10 mg/ml or 15 mg/ml.

In one embodiment of the process, the resulting final concentration of the substituted β-cyclodextrin in the pharmaceutical composition is from 70 mg/ml to 170 mg/ml.

In one embodiment of the process, the predetermined concentration of the substituted β-cyclodextrin is such which results in a final concentration of substituted β-cyclodextrin in the pharmaceutical composition of from 80 mg/ml to 170 mg/ml.

In one embodiment of the process, the predetermined concentration of the substituted β-cyclodextrin is such which results in a final concentration of substituted α-cyclodextrin in the pharmaceutical composition of from 90 mg/ml to 170 mg/ml.

In one embodiment of the process, the predetermined concentration of the substituted β-cyclodextrin is such which results in a final concentration of substituted β-cyclodextrin in the pharmaceutical composition of from 100 mg/ml to 170 mg/ml.

In one embodiment of the process, the predetermined concentration of the substituted β-cyclodextrin is such which results in a. final concentration of substituted β-cyclodextrin in the pharmaceutical composition of from 110 mg/ml to 170 mg/ml.

In one embodiment of the process, the predetermined concentration of the substituted β-cyclodextrin is such which results in a final concentration of substituted β-cyclodextrin in the pharmaceutical composition of from 120 mg/ml to 170 mg/ml.

In one embodiment of the process, the predetermined concentration of the substituted β-cyclodextrin is such which results in a final concentration of substituted β-cyclodextrin in the pharmaceutical composition of from 130 mg/ml to 170 mg/ml.

In one embodiment of the process, the predetermined concentration of the substituted β-cyclodextrin is such which results in a final concentration of substituted β-cyclodextrin in the pharmaceutical composition of from 140 mg/ml to 170 mg/ml.

In one embodiment of the process, the predetermined concentration of the substituted β-cyclodextrin is such which results in a final concentration of substituted β-cyclodextrin in the pharmaceutical composition of from 150 mg/ml to 170 mg/ml.

In one embodiment of the process, the predetermined concentration of the substituted β-cyclodextrin is such which results in a final concentration of substituted β-cyclodextrin in the pharmaceutical composition of from 160 mg/ml to 170 mg/ml.

In one embodiment of the process, the predetermined concentration of the substituted β-cyclodextrin is such which results in a final concentration of substituted β-cyclodextrin in the pharmaceutical composition is 120 mg/ml.

In a further embodiment, step b) further comprises mixing the solution for 1 hour.

In a further embodiment, in step c) the pH is adjusted using HCl or NaOH 1.0N.

In a further embodiment, the process further comprises filtering the solution of step d) through a cellulose acetate filter.

In a further embodiment,
the predetermined amount of peptide is such which results in a final concentration of peptide in the pharmaceutical composition of 2.5mg/ml, 2.0mg/ml, 1.0mg/ml, 0.5 mg/ml or 0.1 mg/ml;
step b) further comprises mixing the solution for 1 hour; and
in step c) the pH is adjusted using HCl or NaOH 1.0N, further comprising filtering the solution of step d) through a cellulose acetate filter.

The subject invention also provides a composition prepared by any of the above processes.

The subject invention also provides a lyophilized pharmaceutical composition comprising from 0.1 mg/ml to 20 mg/ml of the composition of a pharmaceutically acceptable salt of
a peptide comprising consecutive amino acids having the sequence
   (i) TGYYX₁X₂X₃X₄X₅QSPEKSLEWIG (SEQ ID NO:11) wherein X₁ is Met, Ala or Val; X₂ is Gln, Asp, Glu or Arg; X₃ is Trp or Ala; X₄ is Val or Ser; and X₅ is Lys, Glu or Ala;
   (ii) EINPSTGGX₆X₇X₈X₉X₁₀X₁₁X₁₂KAKAT (SEQ ID NO:12) wherein X₆ and X₇ are each Thr, Val or Ala; X₈ is Tyr or Phe; X₉ is Asn or Asp; X₁₀ is Gln or Glu; X₁₁ is Lys or Glu, and X₁₂ is Phe or Tyr;
   (iii) YYCARX₁₃X₁₄X₁₅X₁₆PYAX₁₇X₁₈YWGQGS (SEQ ID NO:13) wherein X₁₃ is Phe, Thr or Gly; X₁₄ is Leu, Ala or Ser; X₁₅ is Trp or Ala; X₁₆ is Glu or Lys; X₁₇ is Met or Ala, and X₁₈ is Asp, Lys or Ser;
   (iv) GYNX₁₉X₂₉X₂₁X₂₂X₂₃X₂₄SHGX₂₅X₂₆LEWIG (SEQ ID NO:14) wherein X₁₉ is Met or Ala; X₂₀ is Asn, Asp or Arg; X₂₁ is Trp or Ala; X₂₂ is Val or Ser; X₂₃ is Lys or Glu; X₂₄ is Gln or Ala; X₂₅ is Lys or Glu, and X₂₆ is Ser or Ala;
   (v) YYCARX₂₇X₂₈X₂₉YGX₃₀X₃₁X₃₂GQTL (SEQ ID NO:15) is Arg, Ala or Glu; X₃₀ is Asn or Asp; X₃₁ is Tyr or Phe, and X₃₂ is Trp, His or Ala;
   (vi) X₃₃YYWSWIX₃₄QX₃₅PX₃₆X₃₇GX₃₈EWIG (SEQ ID NO:16) wherein X₃₃ is Gly or Thr Gly; X₃₄ is Arg or Lys; X₃₅ is Pro or Ser; X₃₆ is Gly or Glu; X₃₇ is Lys or Asp; and X₃₈ is Glu, Leu or Ser;
   (viii) FSGYYWS (SEQ ID NO:8);
   (ix) EINHSGSTNYKTSLKS (SEQ ID NO:9); or
   (x) GLLRGGWNDVDYYYGMDV (SEQ ID NO:10),
   and
a solubility enhancer being a substituted β-cyclodextrin.

In one embodiment of the lyophilized pharmaceutical composition, at least 0.5 mg/ml of the composition is the pharmaceutically acceptable salt of the peptide.

The subject invention also provides a process of lyophilizing any of the above pharmaceutical compositions comprising the steps of:
a) lowering the temperature of the pharmaceutical composition to -40°C;
b) holding the temperature at -40°C for a predetermined time;
c) raising the temperature of the solution to 20°C;
d) holding the temperature at 20°C for a predetermined time; and
e) reducing the pressure and holding the temperature at 20°C for a predetermined time, thereby lyophilizing the pharmaceutical composition.

In one embodiment, step a) is performed within 2 hours.

In another embodiment, step b) is performed within 3 hours.

In another embodiment, step c) is performed over 13 hours.

In another embodiment, step c) is performed at a pressure of 110µbar.

In another embodiment, step d) is performed over 13 hours.

In another embodiment, step d) is performed at a pressure of 110µbar.

In another embodiment, in step e) the pressure is reduced to 10µbar.

In another embodiment, step e) is performed over 5 hours.

In another embodiment,
step a) is performed within 2 hours;
step b) is performed within 3 hours;
step c) is performed over 13 hours and at a pressure of 110µbar;
step d) is performed over 13 hours and at a pressure of 110µbar; and
step e) is performed over 5 hours and the pressure is reduced to 10µbar.

The subject invention also provides a lyophilized pharmaceutical composition prepared by any of the above processes.

The subject invention also provides a process of lyophilizing any of the above pharmaceutical compositions comprising the steps of:
a) lowering the temperature of the pharmaceutical composition to -45°C;
b) holding the temperature at -45°C for a predetermined time;
c) raising the temperature of the solution to -20°C;
d) raising the temperature of the solution to 25°C; and
e) holding the temperature at 25°C for a predetermined time, thereby lyophilizing the pharmaceutical composition.

In one embodiment, step a) is performed within 6 hours.

In another embodiment, step b) is performed within 3 hours.

In another embodiment, step c) is performed over 19 hours.

In another embodiment, step c) is performed at a pressure of 150µbar.

In another embodiment, step d) is performed over 13 hours.

In another embodiment, step d) is performed at a pressure of 150µbar.

In another embodiment, step e) is performed over 8 hours.

In another embodiment, step e) is performed at a pressure of 150µbar.

In another embodiment,
step a) is performed within 6 hours;
step b) is performed within 3 hours;
step c) is performed over 19 hours and at a pressure of 150µbar;
step d) is performed over 13 hours and at a pressure of 150µbar; and
step e) is performed over 8 hours and at a pressure of 150µbar.

The subject invention also provides a lyophilized pharmaceutical composition prepared by any of the above processes.

The subject invention also provides the above lyophilized pharmaceutical composition wherein the water content of the composition is less than 5%.

In one embodiment, the water content of the composition is less than 4.0%.

In another embodiment, the water content of the composition is less then 3.5%.

The subject invention also provides a packaged pharmaceutical composition comprised of:
a packaging material; and
a predetermined amount of any of the above lyophilized pharmaceutical compositions.

In another embodiment, the peptide has the formula
NH₂-Gly Tyr Tyr Trp Ser Trp Ile Arg Gln Pro Pro Gly Lys Gly Glu Glu Trp Ile Gly-COOH (SEQ ID NO:6).

The synthetic peptides of this invention are based on the CDR of monoclonal pathogenic autoantibodies isolated from mice with experimental SLE. Such monoclonal antibodies are obtained from supernatants of hybridomas produced by fusion, for example, of spleen cells of C3H.SW mice immunized with an anti-16/6 Id mAb, with X63.653 plasmacytoma cells (Waisman and Mozes, 1993).

Examples of such peptides are those of formulas Ia to Va herein, based on, respectively, the CDR1, CDR2 and CDR3 regions of the heavy chain of mAb 5G12 and the CDR1 and CDR3 regions of the heavy chain of mAb 2C4C2 (Waisman and Mozes, 1993), and analogs thereof.

Peptides of the present invention are intended to include analogs of peptides Ia-Va including substitution, deletion and addition analogs as described herein. Substitution analogs have amino acid substitutions at different positions, these substitutions being made based on the volume, hydrophobic-hydrophilic pattern and charge of the amino acids.

Amino acids may be divided along the lines of volume, hydrophobic-hydrophilic pattern and charge. With respect to volume, those of ordinary skill in the art understand that the amino acids with the largest volume are Trp, Tyr, Phe, Arg, Lys, Ile, Leu, Met and His, while those with the smallest volumes are Gly, Ala, Ser, Asp, Thr and Pro, with others being in between.

With respect to hydrophobic-hydrophilic pattern, it is well known that the amino acids Gly, Ala, Phe, Val, Leu, Ile, Pro, Met and Trp are hydrophobic, whereas all of the remaining amino acids are hydrophilic. Among the hydrophilic amino acids, Ser, Thr, Gln, and Tyr have no charge, while Arg, Lys, His and Asn have a positive charge and Asp and Glu have negative charges.

In selecting peptides to be tested for their potential in inhibiting the proliferative response of T lymphocytes of mice that are high responders to SLE-inducing autoantibodies, it is important that the substitutions be selected from those which cumulatively do not substantially change the volume, hydrophobic-hydrophilic pattern and charge of the corresponding portion of the unsubstituted parent peptide. Thus, a hydrophobic residue may be substituted with a hydrophilic residue, or vice-versa, as long as the total effect does not substantially change the volume, hydrophobic-hydrophilic pattern and charge of the corresponding unsubstituted parent peptide.

It should be understood that other modifications of the peptides are also contemplated by the present invention. Thus, the peptide of the present invention is intended to include a "chemical derivative" thereof which retains at least a portion of the function of the peptide which permits its utility in preventing or inhibiting T cell proliferative responses and autoimmune disease.

A "chemical derivative" of a peptide of the present invention contains additional chemical moieties not normally a part of the peptide. Covalent modifications of the peptide are included within the scope of this invention. Such modifications may be introduced into the molecule by reacting targeted amino acid residues of the peptide with an organic derivatizing agent that is capable of reacting with selected side chains or terminal residues. Many such chemical derivatives and methods for making them are well known in the art.

Also included in the scope of the invention are salts of the peptides of the invention. As used herein, the term "salts" refers to both salts of carboxyl groups and to acid addition salts of amino groups of the peptide molecule. Salts of a carboxyl group may be formed by means known in the art and include inorganic salts, for example, sodium, calcium, ammonium, ferric or zinc salts, and the like, and salts with organic bases such as those formed for example, with amines, such astriethanolamine, arginine, or lysine, piperidine, procaine, and the like. Acid addition salts include, for example, salts with mineral acids such as, for example, hydrochloric acid or sulfuric acid, and salts with organic acids, such as, for example, acetic acid or oxalic acid. Such chemical derivatives and salts are preferably used to modify the pharmaceutical properties of the peptide insofar as stability, solubility, etc., are concerned.

The synthetic peptides and analogs thereof according to the invention may be selected from the group consisting of peptides having the sequences I to V herein, wherein:
(i) the peptide of sequence I has the formula (SEQ ID NO:11):

   **T G Y Y X₁ X₂ X₃ X₄ X₅ Q S P E K S L E W I G** **[I]**

   wherein X₁ is Met, Ala or Val; X₂ is Gln, Asp, Glu or Arg; X₃ is Trp or Ala; X₄ is Val or Ser; and X₅ is Lys, Glu or Ala.
   In one embodiment, the peptide of sequence I has the formula (Ia)(SEQ ID NO: 1):

   **TGYYMQWVKQ S PE K S L E W I G** **(Ia)**
(ii) the peptide of sequence II has the formula (SEQ ID NO:12):

   **E I N P S T G G X₆ X₇ X₈ X₉ X₁₀ A₁₁ X₁₂ K A K A T** [**II]**

   wherein X₆ and X₇ are each Thr, Val or Ala; X₈ is Tyr or Phe; X₉ is Asn or Asp; X₁₀ is Gln or Glu; X₁₁ is Lys or Glu, and X₁₂ is Phe or Tyr.
   In one embodiment, the peptide of sequence II has the formula (IIa) (SEQ ID NO:2):

   **EINPST GGT T YN Q KF KA K A T** **(IIa)**
(iii) the peptide of sequence III has the formula (SEQ ID NO:13):

   **Y Y C A R X₁₃ X_{I4} X₁₅ X₁₆ P Y A X₁₇ X₁₈ Y W G Q G S** **[III]**

   wherein X₁₃ is Phe, Thr or Gly; X₁₄ is Leu, Ala or Ser; X₁₅ is Trp or Ala; X₁₆ is Glu or Lys; X₁₇ is Met or Ala, and X₁₈ is Asp, Lys or Ser.
   In one embodiment, the peptide of sequence III has the formula (IIIa) (SEQ ID NO:3):

   **Y Y CA R F L W E P Y A M D Y W G Q G S** **(IIIa)**
(iv) the peptide of sequence IV has the formula (SEQ ID N0:14):

   **G Y N X₁₉ X₂₀ X₂₁ X₂₂ X₂₃ X₂₄ S H G X₂₅ X₂₆ L E W I G** **[IV]**

   wherein X₁₉ is Met or Ala; X₂₀ is Asn, Asp or Arg; X₂₁ is Trp or Ala; X₂₂ is Val or Ser; X₂₃ is Lys or Glu; X₂₄ is Gln or Ala; X₂₅ is Lys or Glu, and X₂₆ is Ser or Ala.
   In one embodiment, the peptide of sequence IV has the formula (IVa) (SEQ ID NO:4):

   **GYNMNWVKQ S H G K S LEW I G** **(IVa)**
(v) the peptide of sequence V has the formula (SEQ ID NO:15):

   **Y Y C A R X₂₇ X₂₈ X₂₉ Y G X₃₀ X₃₁ X₃₂ G Q T L** **[V]**

   wherein X₂₇ is Ser or Phe; X₂₈ is Gly or Ala; X₂₉ is Arg, Ala or Glu; X₃₀ is Asn or Asp; X₃₁ is Tyr or Phe, and X₃₂ is Trp, His or Ala.

In one embodiment, the peptide of sequence V has the formula (Va) (SEQ ID NO:5):

**YYCARSGRYG N Y W GQ T L** **(Va).**

Peptides Ia to IIIa are based on the CDR1, CDR2 and CDR3 regions, respectively, of the V_{H} chain of mAb5G12, and peptides IVa and Va are based on the CDR1 and CDR3 regions, respectively, of the V_{H} chain of mAb 2C4C2 (Waisman and Mozes, 1993).

Once a peptide in accordance with the present invention is produced, its ability to inhibit the proliferative response of T lymphocytes of mice that are high responders to SLE inducing autoantibodies may be readily determined by those of ordinary skill in the art without undue experimentation using tests such as those described herein. One test which may be readily conducted is for the ability of substituted peptides to inhibit *in vitro* the proliferative responses of certain T cell lines and clones specific to SLE-inducing autoantibodies. The T cell lines and clones may, for example, be the T cell lines and clones specific to the 16/6 Id mAb (Fricke et al., 1991) established from immunized lymph node cells of mice by previously described methodology (Axelrod, O. and Mozes, E.

Immunobiology 172: 99 (1986)). Cells are exposed to the stimulating antibody presented on irradiated syngeneic spleen cells in the presence of enriched medium every two weeks. The T cell lines are cloned by the standard limiting dilution technique. The proliferative responses of these T cell lines and clones are tested, for example, by the method described in Materials and Methods herein.

Another test which can be conducted in order to select peptides having the desired activity is to test for the ability of the substituted peptides to inhibit the ability of the T cell lines and clones to provide help to peptide-specific B cells in the presence of the parent peptide. The substituted peptides may also be tested for their ability to bind directly, following biotinylation, to MMC Class II products on antigen-presenting cells of the relevant strains. For this purpose, N-terminal biotinylation of the relevant peptides is performed at 0ºC with an excess of biotin-N-hydroxysuccinimide in aqueous solution (Mozes, E. et al., EMBO J. 8: 4049 (1989)). Mouse splenic adherent cells or human peripheral blood lymphocyte (PBL)-adherent cells (1x10⁶/sample) are incubated with biotinylated peptides in PBS containing 0.1% bovine serum albumin (PBS/BSA) at 37ºC for 20 hr, followed by incubation with phycoerythrin-streptavidin for 30 min at 4ºC. After each incubation, the cells are washed twice with the above solution. Thereafter, the cells are analyzed by flow cytometry using FACScan. In each analysis, a minimum of 5000 cells are examined (for above procedures, see, for example, Mozes et al., 1989; Zisman et al., 1991).

A further test which can be conducted is to test for the ability of the peptides to inhibit cytokine secretion by the T cell line or by T lymphocytes of mice that are high responders to SLE-inducing autoantibodies. The cytokines are detected as follows: IL-1 activity is assessed either by ELISA using a pair of capture and detecting antibodies (as described below for IL-4, IL-6, IL-10) or using the LBRM-33(1A5) assay (Conlon, P.J. J. Immune. 134:1280 (1983))in which 1A5 cells are stimulated in the presence of phytohemagglutinin (PHA), with either supernatants or recombinant IL-1 at various concentrations to secrete IL-2. Following an overnight incubation, supernatants of 1A5 cells are transferred to the IL-2 dependent cytotoxic T lymphocyte (CTLL) line. Stimulation of the CTLL line by IL-2 is measured after 24 hr by incorporation of ³[H]-thymidine. IL-2 is directly detected using the IL-2 dependent CTLL line or by ELISA. Levels of IL-4, IL-6, IL-10, INFγ and TNFα in the supernatants are determined by ELISA using antibodies to the various cytokines (Phamingen, San Diego, Ca., USA) according to the manufacturer's instructions.

Peptides which test positive in one or more of these *in vitro* tests will provide a reasonable expectation of *in vivo* activity. However, in vivo tests can also be conducted without undue experimentation. Thus, for example, adult mice may be injected with the candidate peptide at either day -3 or day 0. The mice are then immunized with the disease-inducing autoantibody or with the peptide. Ten days later, lymph node cells of the mice are tested for their ability to proliferate to the immunogen in order to find out the inhibitory capacity of the candidate peptide.

Another such *in vivo* animal test consists in measuring the therapeutic activity directly in the murine model *in vivo* for the production of SLE as described above. The peptides can be injected into the mice in which experimental SLE is induced by different routes at different dosages and at different time schedules. In order to determine the pharmacokinetic parameters of the peptides, including volume of distribution, uptake into antigen-presenting cells and clearance, one can use biotinylated derivatives of the peptides. The concentration of the soluble fraction of the peptides in the various body fluids can be determined by ELISA, using avidin-coated plates and specific anti-peptide antibodies. Cell bound peptides can be analyzed by FACS, using fluorochromo-conjugated avidin or streptavidin. Furthermore, the treated mice can be tested periodically in order to determine the effect of the peptides on the autoantibody responses and on disease manifestations elicited in the mice by the SLE-inducing autoantibody.

Another *in vivo* procedure consists in tolerizing newborn mice with the candidate peptide followed by immunization of the mice with the pathogenic autoantibody, such as 16/6 Id+, or with the same peptide, and following the disease manifestations, such as serological findings associated with leukopenia, elevated erythrocyte sedimentation rate, proteinuria, abundance of immune complexes in the kidneys and sclerosis of the glomeruli. It can thus be seen that, besides the preferred embodiments which have been shown to be operable in the examples herein, those of ordinary skill in the art will be able to determine additional peptides which will also be operable following the guidelines presented herein without undue experimentation.

A relatively simple *in vitro* test can also be conducted in order to assay for the expected therapeutic efficacy of any given substituted peptide on any given SLE patient. In order to assess the ultimate goal of producing peptides that will bind with high affinity to the appropriate MHC Class II molecules but will not lead to further activation of T cells and will therefore have a therapeutic effect on SLE patients, the peptides may be assayed, following biotinylation, for their ability to bind directly to HLA Class II products on antigen-presenting cells in the peripheral blood lymphocytes of the SLE patients. Healthy control donors and control peptides may be used in such assays-to verify their specificity.

In one embodiment, the therapeutic agent of the invention is a peptide selected from the group of peptides of formulas I to V herein, including peptides Ia to Va and substitution and/or deletion analogs thereof.

In another embodiment, the therapeutic agent in accordance with the present invention is the form of a multi-epitope single peptide. Thus, in a preferred embodiment, dual petides consisting of two different peptides selected from the group of peptides of formulas I-V herein, are covalently linked to one another, such as by a short stretch of alanine residues or by a putative site for proteolysis by cathepsin. See, for example, U.S. Patent 5,126,249 and European Patent 495,049 with respect to such sites. This will induce sitespecific proteolysis of the preferred form into the two desired analogs. Alternatively, a number of the same or different peptides of the present invention may be formed into a peptide polymer, such as, for example, polymerization of the peptides with a suitable polymerization agent, such as 0.1% glutaraldehyde (Audibert et al. (1981), Nature 289:593). The polymer will preferably contain from 5 to 20 peptide residues. Such peptide polymers may also be formed by crosslinking the peptides or attaching multiple peptides to macromolecular carriers. Suitable macromolecular carriers are, for example, proteins, such as tetanus toxoid, and linear or branched copolymers of amino acids, such as a linear copolymer of L-alanine, L-glutamic acid and L-lysine and a branched copolymer of L tyrosine, L-glutamic acid, L-alanine and L-lysine (T,G)-A-L-, or multichain poly-DLalanine (M. Sela et al. 1955, J. Am. Chem. Soc. 77:6175). The conjugates are obtained, for example, by first coupling the peptide with a water-soluble carbodiimide, such as 1-ethyl-3-(3'-dimethylaminopropyl) carbodiimide hydrochloride, and then performing the conjugation with the macromolecular carrier as described by Muller, G.M. et al. (1982) Proc. Natl.Acad. Sci. USA 79:569. The contents of the coupled peptide in each conjugate are determined by amino acid analysis, in comparison to the composition of the carrier alone.

According to one embodiment of the present invention, one or more active peptides may be attached to a suitable macromolecular carrier or may be polymerized in the presence of glutaraldehyde.

The peptides, polymers thereof or their conjugates with suitable macromolecular carriers, will be given to patients in a form that insures their bioavailability, making them suitable for treatment. If more than one peptide is found to have significant inhibitory activity, these peptides will be given to patients in a formulation containing a mixture of the peptides.

The invention further includes pharmaceutical compositions comprising at least one synthetic peptide according to the invention, a conjugate thereof with a suitable macromolecular carrier or a polymer thereof optionally with a pharmaceutically acceptable carrier.

Any suitable route of administration is encompassed by the invention, including oral, intravenous, subcutaneous, intraarticular, intramuscular, inhalation, intranasal, intrathecal, intraperitoneal, intradermal, transdermal or other known routes, including the enteral route.

The dose ranges for the administration of the compositions of the present invention should be large enough to produce the desired effect, whereby, for example, an immune response to the SLE-inducing autoantibody, as measured by T cell proliferation in vitro, is substantially prevented or inhibited, and further, where the disease is significantly treated.

The doses should not be so large as to cause adverse side effects, such as unwanted cross reactions, generalized immunosuppression, anaphylactic reactions and the like.

Effective doses of the peptides of this invention for use in treating SLE are in the range of 1 µg/kg to 1 mg/kg body weight. The dosage administered will be dependent upon the age, sex, health, and weight of the recipient, kind of concurrent treatment, if any, frequency of treatment, and the nature of the effect desired.

The synthetic peptides of the invention, particularly those of sequences I to V herein, are aimed at inhibiting or suppressing specific antigen responses of SLE patients, without affecting all other immune responses. This approach is of the utmost importance since most diagnosed patients are young women that have to be treated for many years and the currently accepted treatment for SLE involves administration of immunosuppressive agents, such as corticosteroids and/or cytotoxic drugs, that are both non-specific and have multiple adverse side effects.

The preparations of the present invention may be given parenterally, topically, or rectally. They are of course given by forms suitable for each administration route. For example, they are administered by injection, inhalation, ointment, suppository, etc. administration by injection, infusion or inhalation; topical by lotion or ointment; and rectal by suppositories.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systematically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

Details of general formulation procedures and information on additional excipients may be found in Remington: The Science and Practice of Pharmacy, 20th Edition.

Synthetic peptides can be produced as described in PCT International Publication No. WO 02/067848, or in PCT International Publication No. WO 96/30057.

This invention will be better understood from the Experimental Details which follow. However, one skilled in the art will readily appreciate that the specific methods and results discussed are merely illustrative of the invention as described more fully in the claims which follow thereafter.

### Experimental Details

The synthesis of peptides I to V and the testing showing the effectiveness of the synthesized peptides for treating SLE in animal models is described in PCT International Publication No. WO 96/30057. Additional animal testing is described in PCT International Publication No. WO 02/067848.

### Example 1: Formulation Development for Compound 1

The peptides of the subject application are described in PCT International Publication No. WO 02/067848, published September 6, 2002, and can be prepared by methods well known in the art, (see, for example, Peptides: Synthesis, Structure and Applications, ed. by B. Gutte, Academic Press, 1995; Peptide Synthesis Protocols, ed. By M. Pennington and B. Dunn, Humana Press, 1994; Schnolzer, M. et al., "In situ neutralization in Boc-chemistry solid phase synthesis. Rapid, High yield assembly of difficult sequences." Int. J. Pept. Protein Res. (1992) 40: 180-193).

Compound 1 is a synthetic polypeptide composed of 19 amino acids. It is provided as an acetate salt. The aqueous solubility of the peptide has been determined to be less than 0.5 mg/ml. Figure 1 shows compound 1 as an acetate salt.

In order to develop a formulation with peptide concentration exceeding 2 mg/ml, preferably up to 10 mg/ml, experiments with several solubility enahancers were performed. The preliminary experiments indicated that a concentration of 2 mg/ml cannot be easily attained. In order to develop a formulation for subcutaneous injection, it is also desirable that the pH be in the range of 4 to 9 and that the solution be iso-osmotic.

Based on an extensive literature survey, a few principal approaches were adopted in order to produce a formulation with maximal solubility. The factors considered were:
- pH adjustment and buffers
- Solvents
- Co-solvents
- Solubilizing agents

### Methods

Compound 1 was dissolved in the chosen solubility enhancer solution either separately or in combination with other excipients and the solutions were stirred for at least an hour. The pH was adjusted if needed. The solutions were visually examined to estimate the solubility and sent for analytical assay determination. For a few chosen formulations, biological activity was also tested.

### Results

Table 1 presents the type of solubility enhancers used for the formulation development. Tables 2 and 3 summarize the experiments that were performed with the various solubility enhancers. Table 2 summarizes the initial screening performed with peptide concentrations in the range of 5 to 10 mg/ml. The experimental work that was performed with higher peptide concentration was then repeated with the lower doses (see table 3).

Initial tests indicated that Compound 1 was more soluble at the limits of the desired pH levels, both acidic and basic, but was less stable at the basic pH range. Thus, several buffers and pH adjustment agents were tested, including acetate buffer, citrate buffer and sodium carbonate. None of the initially tested buffers achieved the desired peptide solubility level.

Only above pH 9.2 and below pH 3.0 were solubility levels of 2 mg/ml observed. Nevertheless, at the initial stage, formulations with acetate buffer and citrate buffer (with Mannitol as a tonicity agent) were selected for initial toxicology studies. These formulations were tested for biological activity and proven active

Non aqueous solvents (see table 1) such as Ethanol, Glycerin, Propylene glycol, Chremophore and their combinations were tested but did not increase the solubility of Compound 1. A solution of 30% DMA (dimethyl-acetamide) yielded solubility in the desired ranges (5 to 9 mg/ml), but was not suitable for a pharmaceutical formulation due to its toxicity profile. Improved solubility was also observed using 30% (w/w) PEG 400 (5 to 9 mg/ml). This latter formulation was chosen for the toxicology studies, but it has proved to be both inactive in the biological assay, and may have been the cause of some adverse effects in a mouse toxicity study. Thus, it was decided not to further pursue this formulation. In view of the preliminary experiments non-aqueous solvents were not used in the subject formulations.

Several amino acids (see table 1) including L-Arginine, L-Glutamic acid, L-Glycine and L-Lysine were tested to improve the protein solubility. The solubility of the peptide in L-Arginine was at the desired level but the resulting pH was above 9. An attempt to decrease the pH or use an Arginine HCl salt resulted in precipitation of the peptide. Human Serum Albumin was also tested and improved the solubility of the peptide at low peptide concentrations (1 mg/ml) (see table 3). However, due to its potential immunogenicity and the low peptide solubility, it was not utilized in further experiments.

Bulking agents (see table 1) including Mannitol, Sorbitol and Dextran were tested alone and in combination with other excipients, but did not improve the solubility of the peptide in solution.

Co-solvents (see table 1), including Polysorbate 20 and Polysorbate 80 were tested alone and in combination with other excipients. While lower concentrations of Polysorbates (up to 6%) did not improve the solubility of the peptide, higher concentrations (up to 10% - see table 2) improved the solubility of the peptide up to 2 mg/ml. However, such high concentrations of Polysorbates were deemed unsuitable for pharmaceutical formulations.

Two types of cyclodextrins, both approved for use in marketed parenteral products, were also tested: Hydroxypropyl-β-cyclodextrin and Sulfobutylether-β-cyclodextrin (Captisol). Both markedly increased the solubility of the peptide (concentrations in the levels of 10 mg/ml for Hydroxypropyl-β-cyclodextrin and 2.5 for Captisol). The biological activity of the two cyclodextrin formulations was tested and was found to be equal to the activity of the peptide alone.

CAPTISOL® is a commercially available polyanionic β-cyclodextrin derivative with a sodium sulfonate salt separated from the hydrophobic cavity by a butyl ether spacer group, or sulfobutylether (SBE). CAPTISOL® is the trade name for CyDex Inc.'s hepta-substituted sulfobutylether β-cyclodextrin (SBE7-β-CD) preparation (www.captisol.com). The structure of CAPTISOL® allows drug molecules to fit in the hydrophobic cavity, thereby isolating the drug molecule from the aqueous solvent. Because the outer surface of CAPTISOL® is hydrophilic, the solubility of the complexed drug molecule is thereby enhanced. The use of cyclodextrins to enhance the solubility of drug molecules is disclosed in U.S. Patent Nos. 5,134,127 and 5,376,645.

According to the literature of CyDex Inc., CAPTISOL® is safe when administered parenterally and does not exhibit the nephrotoxicity associated with beta-cyclodextrin. Relative to beta-cyclodextrin, CAPTISOL® provides comparable or higher complexation characteristics and superior water solubility in excess of 90 grams/100ml - a 50-fold improvement.

### Conclusions

Several solubility enhancers were found to match the desired solubility range: DMA, PEG-400, dimethyl-acetamide, polyethylene glycol, polyoxylated castor oil, N-methyl-2-pyrrolidinone, 1-ethenyl-2-pyrrolidinone, Polysorbate 20, Polysorbate 80, Hydroxypropyl-β-cyclodextrin and Sulfobutylether-β-cycldextrin (Captisol®). Of these solubility enhancers both cyclodextrins have proven to be superior with respect to solubility, biological activity and stability. Thus, it was decided to select Captisol® as the solubility enhancer for use in Example 5 formulations and to further study both cyclodextrin formulations. The final formulation for the Example 5 clinical studies consists of: 120 mg/ml of Captisol in water with the desired amount of peptide (0.5, 1.0 or 2.5 mg/ml), and HCl and NaOH for pH adjustment.

**Table 1: Solubility enhancers used for Compound 1 formulation development**

| **Solubility enhancer classification** | **Solubility Enhancers** |
|---|---|
| Solvents | Cremophor EL, CMC Ethanol, DMA, Gycerin, Propylene Glycol, PEG 400, Monotioglycerol |
| Co-solvents | Polysorbate 20, Ploysorbate 80 |
| Solubilizing.agents | Argenine, HSA, Glycine, Creatinine, Glutamic acid, Lysine (acetate salt and free base), Captisol, Hydroxypropyl-β-cyclodextrin, |
| Bulking agents | Mannitol, Sorbitol, Dextrose, Lactose Dextran |
| pH Adjustment Agents | Citrate buffer, Acetate buffer, Sodium Carbonate |

**Table 2. List of Cosolvents and Stabilizers evaluated in Compound 1 Peptide Formulations.**

| Solubility Enhancer * | ***% Used*** | ***% of Standard* amoun*t from the literature*** | ***Amount of peptide added (mg*/*ml)*** | ***Assay, %*** | ***pH of formulation*** | ***Remarks*** |
|---|---|---|---|---|---|---|
| Albumin (HSA), | 1.5 | 0.4-5.0 | 5 | | 6.0 | Insoluble |
| Dextrose | 1.5 | | | | Adjust, to 4.1 | |
| Albumin (HSA), | 1.0 | 0.4-5.0 | 5 | - | 5.8 | |
| Polysorbate 80, | 0.6 | 0.8-4.0 | | | Adjust. to 4.1 | Insoluble |
| Glycine | 2.0 | 0.2-2.1 | | | | |
| Arginine | 1.5 | 0.8-1.6 | 15 | 93 | 9.8 | Clear solution |
| Arginine HCI | 2.0 | 0.8-1.6 | 5 | - | 3.5 | Insoluble |
| Arginine | 1.5 | 0.8-1.6 | 15 | 93 | 9.8 | When the pH was lowered below 8.5 the peptide |
| Lactose | 1.5 | | | | | precipitated and the solution turned into gel |
| Captisol | 10.0 | Up to 30.0 | 10 | 86 | 4.9 | Turbid solution |
| | 20.0 | | | 89 | Adjust. to 4.4 | |
| CMC (carboxy methyl cellulose) in acetate | 0.2 0.05M | | 5 | 90 | 5.0 | For toxicology studies |
| Creatanine | 0.8 | Up to 0.6 | 5 | - | 6.1 Adjust. to 4.1 | Insoluble |
| Cremophor EL | 15.0 | -10.0 | 5 | - | 4.0 | Very turbid |
| Ethanol | 10.0 | 0.6-32.9 | | | | |
| Dimethylacetamide | 6.0 | 0.012-6.0 | | | | |
| Dextran | 4.0 to 15.0 | 3.0-30.0 | 5 | - | 3.9 | Insoluble |
| Dimethylacetamide (DMA) | 6.0-20.0 | 0.012-6.0 | 5 | - | 4.6 | Insoluble |

| **Solubility Enhancer *** | ***% Used*** | ***% of Standard amount*** | ***Amount of peptide added (mg*/*ml)*** | ***Assay, %*** | ***pH of formulation*** | ***Remarks*** |
|---|---|---|---|---|---|---|
| Dimethylacetamide (DMA) | 25.0 | 0.012-6.0 | 5 | 87 | 5.1 | Clear solution |
| Dimethylacetamide (DMA) | 30.0 | 0.012-6.0 | 10 | 93 | 5.1 | Clear solution |
| Ethanol | 10.0 | 0.6-32.9 | 5 | - | - | Insoluble |
| Glutamic acid | 2.0 | precipitated | 5 | - | 3.7 | When the pH was increased above 4 the peptide precipitated and the solution turned into gel |
| Glycerin | 1.5 | 1.6-32.5 | 5 | 37 | 4.5 | Insoluble |
| Glycerin | 30.0 | 1.6-32.5 | 5 | - | 3.7 | Insoluble |
| Glycerin, | 10.0 | 1.6-32.5 | 5 | 12 | 6.5 | Insoluble |
| Polysorbate 80 | 0.6 | 0.8-4 | | | Adjust. to 4.5 | |
| Glycine | 0.4 | 0.2-2.1 | 5 | - | 4.6 | Insoluble |
| Hydroxypropyl β-cyclodextrins | 20.0 | Up to 30.0 | 10 | 99 | 4.6 | Clear solution |
| Lysine Acetate Salt | 2.0 | | 5 | - | 3.8 | Insoluble |
| Lysine Free base | 2.0 | | 5 | - | 9.2 | When the pH was lowered below 8 the peptide precipitated and the solution turned into gel |
| Mannitol in Citrate buffer | 4.0 | 2.0-10.0 | 5 | 38 | 3.4 | For toxicology studies |
| | 0.035M | | | | | |
| Mannitol | 4.0 | 2.0-10.0 | 5 | 32 | 4.3 | For toxicology studies |
| in acetate buffer | 0.05M | | | | | |
| Mannitol, | 20.0 | 2.0-10.0 | 5 | 14 | 6.4 | Insoluble |
| Glycine | 0.4 | 0.2-2.1 | | | Adjust. to 4.5 | |
| Mannitol, | 20.0 | 2.0-10.0 | 5 | 22 | 6.5 | Insoluble |
| Polysorbate 20 | 0.6 | - | | | Adjust. to 4.5 | |
| Monothioglycerol | 1.0 | 0.1-10.0 | 5 | - | 4.5 | Turbid solution |
| PEG 400 | 30.0 | Up to 30.0 | 5 | 88 | 4.2 | Slightly opalescent |

| Solubility Enhancer * | **% *Used*** | ***% of Standard amount from the literature*** | ***Amount of peptide added (mg*/*ml)*** | ***Assay,%*** | ***pH of formulation*** | ***Remarks*** |
|---|---|---|---|---|---|---|
| PEG 400 | 30.0 | Up to 30.0 | 10 | 89 | 4.2 | Turbid solution |
| PEG 400 | 30.0 | Up to 30.0 | 5 | 94 | 4.2 | Clear solution |
| with DMA | 6.0 | 0.012-0.6 | | | | |
| PEG 400 | 10.0 | 6.0-18.0 | 5 | 58 | 4.2 | Insoluble |
| PEG 400 | 10.0 | Up to 30.0 | 5 | - | 4.3 | Insoluble |
| DMA | 10.0 | 0.012-0.6 | | | | |
| PEG 400 | 10.0 | Up to 30.0 | 5 | - | 4.1 | Insoluble |
| Propylene glycol PG | 10.0 | 10.0 | | | | |
| PEG 400 | 18.0 | Up to 30.0 | 5 | 100 | 4.2 | Clear solution |
| Propylene glycol | 50.0 | 10.0 | | | | |
| Polysorbate 80 | 1.6 | 0.8-4.0 | 5 | 24 | 7.2 | Insoluble |
| | | | | | Adjust. to 4.5 | |
| Polysorbate 80 | 6.0 | 0.8-4.0 | 5 | - | 3.9 | Insoluble |
| Polysorbate 80 | 6.0 | 0.8-4.0 | 5 | - | 4.0 | Insoluble |
| Creatanine | 0.6 | up to 0.6 | | | | |
| Propylene glycol PG | 10.0 | 10.0 | 5 | - | 4.2 | Insoluble |
| DMA | 10.0 | 0.012-0.6 | | | | |
| Propylene glycol PG | 10.0, 30.0 | 10.0 | 5 | - | 4.2 | Insoluble |
| Sodium Carbonate | 1.5 | | 5 | - | 11.4 | When the pH was lowered below 8.5 the peptide precipitated and the solution turned into gel |
| Sorbitol | 5.0 | 10.0-25.0 | 5 | - | 6.9 | Turbid solution |
| | | | | | Adjust. to 4.5 | |

**Table 3: Compound 1 formulations at low Peptide concentrations**

| Solubility Enhancer * | ***% Used*** | ***% of Standard amount from the literature*** | ***Amount of peptide added (mg*/*ml)*** | ***Assay,%*** | ***pH of formulation*** | ***Rema**rk**s*** |
|---|---|---|---|---|---|---|
| Albumin (HSA), | 5.0 | 0.4-5.0 | 1.0 | 90 | 6.9 | Clear solution |
| | | | 2.5 | - | Adjust. to 4.5 | Turbid solution |
| Arginine | 1.5 | 0.8-1.6 | 1.0 | 24 | 10.6 | When the pH was lowered below 8.5 the peptide |
| | | | | | Adjust. to 8.5 | precipitated and the solution turned into gel |
| Captisol | 12.0 | Up to 30.0 | 1.0 | 106 | 5.3 | Clear solution |
| | | | 2.5 | 100 | 6.5 to 8.5 | |
| Dextran | 20.0 | 3.0-30.0 | 1.0 | 69 | 4.8 | Turbid solution |
| | | | | | Adjust. to 4.0 | |
| Glycerin | 30.0 | 1.6-32.5 | 1.0 | - | 4.8 | Turbid solution |
| | | | | | Adjust. to 4.0 | |
| Mannitol | 4.0 | 0.8-4.0 | 1.0 | 64 | 4.7 | Turbid solution |
| | | | | | Adjust. to 4.0 | |
| Polysorbate 20 | 10.0 | - | 1.0 | 95 | 5.8 | Clear solution |
| | | | 2.5 | 88 | Adjust. to 4.8 | Clear with small amount of precipitation |
| Polysorbate 20 | 10.0 | - | 2.5 | 115 | 5.1 | Clear solution |
| Mannitol | 2.0 | 2.0-10.0 | | | Adjust. to 4.3 | |
| Polysorbate 80 | 4.0 | 0.8-4.0 | 1.0 | 91 | 5.5 | Clear solution |
| | 6.0-10.0 | | 2.5 | 89 | Adjust. to 5.0 | Slightly turbid solution |
| Polysorbate 80 | 4.0 | 0.8-4.0 | 2.5 | 88 | 5.1 | Slightly turbid solution |
| Mannitol | 2.0 | 2.0-10.0 | | | Adjust. to 4.4 | |
| Propylene glycol PG | 10.0 | 10.0 | 1.0 | 78 | 5.0 | Turbid solution |
| | | | | | Adjust. to 4.4 | |
| Sorbitol | 20.0 | 10.0-25 | 1.0 | 52 | 4.5 | Turbid solution |

### Example 2: Preparation protocol for solution of Compound 1 in Captisol®

Standard dissolution methods, such as mixing dry Compound 1 and dry Captisol® into water or adding Compound 1 to a prepared solution of Captisol® and water did not result in complete dissolution at the desired concentrations. Several different concentrations of both Compound 1 and Captisol® were tested at various pH levels. However, the following method for producing a solution of Compound 1 in Captisol® resulted in complete dissolution at the desired concentrations.

### Materials: Captisol®, Compound 1 and water

Method:
1. Weigh the appropriate amount of Captisol® to give a final concentration of 120 mg/ml.
2. Add 80% of the final amount of water and mix for 10 minutes with a magnetic stirrer.
3. Weigh Compound 1 to give a final concentration of 2.5 mg/ml, 2.0 mg/ml, 1.0 mg/ml, 0.5 mg/ml or 0.1 mg/ml.
4. Add the peptide to the Captisol® solution. Mix for 1 hour.
5. Raise the pH to obtain clear solution (in the 2.0 mg/ml formulation there might be a need to raise the pH slightly above 9). pH should be adjusted using HCl 1.0 N and NaOH 1.0 N. Mix for 10 minutes.
6. Correct the pH to the range of 7.5 to 8.5 if needed (using either HCl or NaOH 1.0 N).
7. Add water to final volume.
8. Filter the solution through a 0.2µ cellulose acetate filter.
9. Record final pH.
10. Dispense into aliquots and store at the proper temperature.

### Example 3: Lyophilization of Compound 1 and Captisol® solution

The current lyophilization process differs from other lyophilization processes in that the percentage of solids in the formulation is high (12%) whereas lyophilized products normally contain between 5 and 10% solids.

### Equipment

The freeze drier used was an Edwards lyophilizer Lyoflex 0.6. The equipment IQ/OQ was performed and checked for compliance by quality assurance prior to the process development.

Solutions of Compound 1 and Captisol® at concentrations of 0.5 mg/ml, 1.0mg/ml and 2.5mg/ml of Compound 1 were prepared. The fill-volume was adjusted 1 ml (1.05 gr).

### Main process steps:

1. Freezing
2. Holding (at low temperature)
3. Drying under vacuum in two stages:
   3.1. Primary drying - shelf warming to an upper hold temperature, controlling shelf temperature at the upper hold level.
   3.2. Secondary drying - Pressure reduction to a minimal value at the upper hold shelf temperature.

### Batches 1-3

Freezing - Freezing was from room temperature to -40°C within 2 hours. Shelves were held at -40°C for 3 hours.

Drying - Drying was performed at 110 µbar pressure. Shelf temperature was increased to 20°C over 13 hours and held at that temperature for additional 13 hours.

Total process time was 31 hours.

### Results:

Water content results were:
Batch no. 1: 3.8%
Batch no. 2: 4.0% and
Batch no. 3: 4.9%

### Batches 4 and 5

Since the water content results of the processes leading to batches 1, 2 and 3 were higher then the desired value, it was decided to add a secondary drying step at the same temperature and at low pressure.

Drying - Drying was performed at 110µbar pressure. Shelf temperature was increased to 20°C over 13 hours and held at that temperature for additional 13 hours (Batch 4) or 8 hours (Batch 5). Pressure was decreased to 10µbar for additional 5 hours.

Total process time was 36 hours.

### Results:

Water content results were
Batch 4: Placebo: 3.0%,
   1 mg/ml: 3.9%.
Batch 5: Placebo: 4.1%

### Conclusions

As shown, a satisfactory lyophilization process for Compound 1 with Captisol® was developed. Due to the high percentage of solids and hence the condensed cake, the developed process is longer then the currently available lyophilization cycles for peptides and it exhibits an additional secondary drying stage. Table 4 summarizes the developed process.

**Table 4**

| Step | Compound 1 (Peptide) with Captisol® |
|---|---|
| Loading | 5°C |
| Freezing | 2 hours to -40°C |
| Hold at low temp. | 3 hours to -40°C |
| Primary Drying: | |
| Warm to 20°C | 13 hours pressure 110µbar |
| Hold at 20°C | 13 hours pressure 110µbar |
| Secondary drying: | |
| Hold at 20°C | 5 hours pressure 10µbar |
| Storage at | -20°C |
| Process time | 36 hours |

### Example 4.

### Examination of the in-vivo biological activity of the lyophilized compound solution (DP, 1 mg/vial, 12% captisol®)

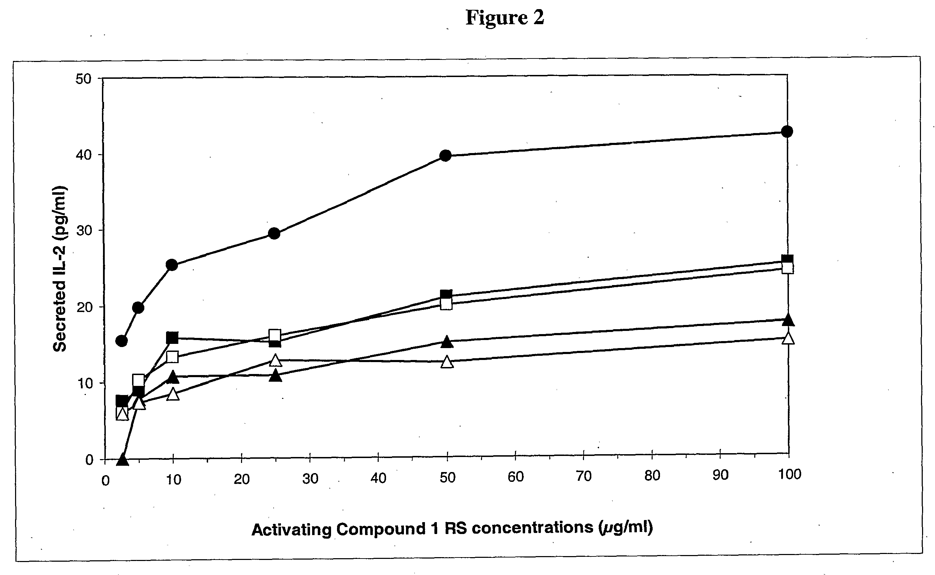

The biological activity was monitored by inhibition of IL-2 secretion from Compound 1 reference standard (RS) specific T-cells following subcutaneous (s.c.) treatment with the lyophilized compound solution, i.e. the drug product (DP), at two concentrations. The results of the treatment are compared to those of treating mice with Compound 1 (RS) in phosphate buffered saline (PBS). The results are shown in the tables below and in Figure 2.

### Experimental design:

1. Immunization Day 0 (Compound 1 RS emulsified with CFA,
   at all four footpads)
2. Treatment
   (s.c. at the back of the neck, in 200 µl solution) Day 0
3. In-vitro activation with: Day 10
   a. Compound 1 RS at concentrations of 0; 0.5; 1; 2.5; 5; 10; 25; 50 and 100 µg/ml
   b. a peptide with the reverse order of amino acids of Compound 1 (negative control).
   c. Con A (positive control).
4. Incubation of culture for 20 hrs at 37°C in a humidified 5% CO₂ incubator.
5. IL-2 measurement by ELISA.

**Table of experimental Groups:**

| **Group** | **Immunization with** | **Treatment** |
|---|---|---|
| **A** | 50 µg Compound 1 RS | 50µg Compd. 1 RS in PBS |
| **B** | | 200µg Compd. 1 RS in PBS |
| **C** | | 50µg DP(batch 2) |
| **D** | | 200µg DP (batch 2) |
| **F** | | Placebo (12 % captisol) |

**IL-2 Secretion from Compound 1(DP) Treated Mice Following in-vitro Activation with Compound 1 RS (pg/ml)**

| | | **Treated with:** | | | | | | | | |
|---|---|---|---|---|---|---|---|---|---|---|
| | **Group** | **F** | **A** | | **B** | | **C** | | **D** | |
| Activator | Concentration of activator (pg/ml) | 12% captisol® Ampulized | Compd. 1 RS 50 µg/mouse | % inhib. | Compd. 1 RS 200 µg/mouse | % inhib. | DP 50 µg/mouse | % inhib. | DP 200 µg/mouse | % inhib. |
| Con A | 2.5 | 5,825 | 6,215 | | 5,403 | | 3,537 | | 4,069 | |
| Compd. 1 RS | 0 | BQL | BQL | | BQL | | BQL | | BQL | |
| Compd. 1 RS | 0.5 | 11 | 9 | | 10 | | 8 | | BQL | |
| Compd. 1 RS | 1 | 10 | BQL | | BQL | | BQL | | BQL | |
| Compd. 1 RS | 2.5 | 15 | 8 | 51 | BQL | NA | 6 | 61 | 6 | 62 |
| Compd. 1 RS | 5 | 20 | 9 | 55 | 8 | 60 | 10 | 48 | 7 | 63 |
| Compd. 1 RS | 10 | 25 | 16 | 38 | 11 | 58 | 13 | 48 | 8 | 67 |
| Compd. 1 RS | 25 | 29 | 15 | 48 | 11 | 63 | 16 | 45 | 13 | 56 |
| Compd. 1 RS | 50 | 40 | 21 | 47 | 15 | 62 | 20 | 50 | 12 | 69 |
| Compd. 1 RS | 100 | 42 | 25 | 41 | 18 | 58 | 24 | 43 | 15 | 64 |
| | | | | | | | | | | |
| | Average inhibition (%) (at the range of 5-100 µg/ml) | | | **45.6** | | **60.4** | | **46.8** | | **63.7** |

| | | | | | | | | | | |
|---|---|---|---|---|---|---|---|---|---|---|
| BQL = Below Quantitation Limit NA = Not Applicable Rows 1-4 not included in the curve | | | | | | | | | | |

### Example 5: Evaluation of Optimal Dose for Treatment

The following abbreviations are used in the following description:
- CFA: Complete Freund's adjuvant
- Con A: Concanavalin A
- DP: Drug Product
- DS: Drug Substance
- EM-1: Enriched DCCM-1 Medium
- EM-3: Enriched RPMI-1640 + fetal calf serum medium
- FCS: Fetal Calf Serum
- IFN-γ: Interferon-gamma
- LN: Lymph Node
- PBS: Phosphate Buffered Saline
- RS: Reference Standard
- s.c.: Subcutaneous
- TB: Trypan Blue
- TGF-β: Transforming Growth Factor-beta
- WFI: Water for Injection

### Introduction

A group of 20 mice were immunized with 50 µg/mouse of Compound 1 RS. The immunized mice were allocated to five treatment groups as follows: placebo, 25, 50, 100 and 200µg/mouse of Compound 1 DP (subcutaneous administration). Ten days post immunization and treatment, LN was extracted and single cell suspension was prepared. The *in-vitro* secretion of IFN-γ and TGF-β by the cultured cells in response to activation with several concentrations of Compound 1 RS was then measured.

### Experimental design

1. Immunization -Day 0
2. Treatment with Compound 1 DP -Day 0
3. In-vitro activation of LN cells from treated mice -Day 10
4. Collection of culture media (for IFN-γ determination) -Day 12
5. Collection of culture media (for TGF-β determination) -Day 13
6. ELISA for IFN-γ
7. ELISA for TGF-β

**Table 7: Experimental Groups**

| **Exp. Group** | **Treatment** | | **In-vitro activation** | |
|---|---|---|---|---|
| | **Article** | **Mice/group** | **Cells/well** | **Compound 1 RS concentration** |
| **A1** | Control 12% Captisol® | 4 | 2.5 × 10⁶ | Compound 1 RS 0-100 µg/ml |
| | | | 5 × 10⁶ | |
| **A2** | 25 5 µg/mouse | 4 | 2.5 × 10⁶ | |
| | | | 5 × 10⁶ | |
| **A3** | 50 µg/mouse | 4 | 2.5 × 10⁶ | |
| | | | 5 × 10⁶ | |
| **A4** | 100 µg/mouse | 4 | 2.5 × 10⁶ | |
| | | | 5 × 10⁶ | |
| **A5** | 200 µg/mouse | 4 | 2.5 × 10⁶ | |
| | | | 5 × 10⁶ | |

### Materials and Reagents

### Animals

Mice: 20 female BALB/c mice, supplied by Harlan animals breeding center, Rehovot.
Age at immunization (week+days): 10
Average weight of mice included in the experiment: 19.01 gr.

### Materials

### General reagents

70% ethanol was prepared from 96% ethanol by diluting with purified H₂O.

### Preparation of Compound 1 solutions for immunization

CFA-Compound 1 RS emulsion (500 µg/ml, 50 µg/mouse) was prepared as follows:
1. 1.874 mg of Compound 1 was dissolved in 1.87 ml of WFI to yield a solution of 1 mg/ml.
2. The solution was tested with a pH indicator strip and found to have a pH of 5.
3. 1.5 ml of the solution were emulsified with 1.5 ml CFA resulting in a final concentration of 500 µg/ml.

### Preparation of solutions for Treatment

Treatment was by a s.c. injection of 200 µl solution.

### Preparation of 12% captisol® solution

1.2 gr of captisol® were dissolved in 10 ml of WFI to yield a solution of 12% captisol®.

### Experimental procedure

### Mice weighing

Mice were weighed before immunization. Average mice weight: 19.01 ± 0.97 gr

### Immunization

The immunization was performed by injecting 100 microliters of the immunization emulsion (50 microliters into each hind footpad).

### Treatment

Following the immunization step the mice were treated by s.c. injection of 200 µl from the designated Compound 1 DP or 12% captisol® treatment solutions, at the back of their neck.

### In-vitro culture

Mice were sacrificed by cervical dislocation. LN were extracted from the hind legs and were transferred to a sterile petri dish containing about 5 mL RPMI. The cells were extracted by gentle squeezing of the tissue against a 200 micrometer mesh stainless steel net. The cells were collected and centrifuged at 300 G for 10 minutes at RT.

Single cell suspensions were prepared from pooled LN of each experimental group.

2.5 and 5.0 million cells/ml/well suspensions were cultured with Compound 1 RS (0-100 µg/ml) in EM-1.

Secretion of IFN-γ and TGF-β, as indication of cellular response, were determined by ELISA of culture media (48hrs for IFN-γ and 72hrs for TGF-β).

**Table 8: The in-vitro experimental groups**

| **Experimental Group** | **Treatment** | **In-vitro activation** | |
|---|---|---|---|
| | **Article** | **Cells/well** | **Activation substance concentration** |
| **A1-2.5** | Control 12% Captisol® | 2.5 × 10⁶ | Compound 1 RS 0; 3.125; 6.25; 12.5; 50 and 100 µg/ml Con A 2.5 µg/ml |
| **A1-5** | | 5 × 10⁶ | |
| **A2-2.5** | DP 25 µg/mouse | 2.5 × 10⁶ | |
| **A2-5** | | 5 × 10⁶ | |
| **A3-2.5** | DP 50 µg/mouse | 2.5 × 10⁶ | |
| **A3-5** | | 5 × 10⁶ | |
| **A4-2.5** | DP 100 µg/mouse | 2.5 × 10⁶ | |
| **A4-5** | | 5 × 10⁶ | |
| **A5-2.5** | DP | 2.5 × 10⁶ | |
| **A5-5** | 200 µg/mouse | 5 × 10⁶ | |

### Preparation of cell suspensions

**Table 9: Results of cell counting and preparation of cell suspensions (10 x 10⁶/ml)**

| **Grp** | **Vol. (ml)** | **Dilutn. factor** | **Viable cells** | **Dead cells** | **%Viable cells** | **%Dead cells** | **Average viable cells** | **Total viable cells (× 10⁶)** | **EM-1 to add (ml) for suspension of 10 × 10⁶ cells/ml** |
|---|---|---|---|---|---|---|---|---|---|
| **A1** | 10 | 16 | 115 | -- | 100 | -- | 112 | 179.2 | 17.9 |
| | | | 109 | -- | 100 | -- | | | |
| **A2** | 10 | 16 | 50 | 4 | 92.6 | 7.4 | 47.5 | 76 | 7.6 |
| | | | 45 | 2 | 95.7 | 4.3 | | | |
| **A3** | 10 | 16 | 80 | 4 | 95.2 | 4.8 | 80.5 | 128.8 | 12.8 |
| | | | 81 | 5 | 94.2 | 5.8 | | | |
| **A4** | 10 | 16 | 87 | -- | 100 | -- | 89 | 142 | 14.2 |
| | | | 91 | -- | 100 | -- | | | |
| **A5** | 10 | 16 | 120 | 2 | 98.4 | 1.6 | 112.5 | 180 | 18 |
| | | | 105 | 2 | 98.1 | 1.9 | | | |

### Preparation of cell suspensions (5 x 10⁶/ml)

The 10 x 10⁶ cells/ml suspensions were diluted 1:2 by adding 5 ml EM-1 to 5 ml cells suspension.

### Incubation of LN cells cultures in 48 wells plates

3 tissue culture plates were prepared. The following was added to each plate.

### Background control (cells incubated with culture media)

0.5 ml of cells suspension
0.5 ml of culture media (EM-1)

### System positive control (cells stimulated with Con A)

0.5 ml of cells suspension
0.5 ml of Con A 5 µg/ml in EM-1 (final conc. 2.5 µg/well)

### Cells incubated with Compound 1 activation solutions (samples)

0.5 ml of cells suspension
0.5 ml of Compound 1 RS 6.25 - 200 µg/ml (final conc. 3.125 - 100 µg/ml/well)

### Incubation of LN cells cultures in 96 wells plates

After the 48-wells plates were prepared, 96-wells plates were prepared by applying 100 µl from the cell suspension and 100 µl from the activation solutions.

The culture plates were incubated at 37°. C in a humidified 5% CO₂ incubator, for either 48 or 72 hrs.

### Supernatants Collection

The cultured plates were centrifuged at 300 g for 10 minutes at RT. Supernatants (850 µl from each well) were transferred either to mirror plates or to tubes. The supernatant was then divided into working aliquots (two aliquots of 200 and one aliquot of 450 µl), in order to avoid repeated freeze/thawing of the samples. Each tube was labeled with the following details:
1. Experimental code and time post incubation.
2. Group and sample number
3. Activator and concentration.
4. Date of sup collection

The supernatants were stored at -20°C until used for ELISA.

### Results

**Table 10: Summary of Groups**

| **Experimental Groups:** | | | | |
|---|---|---|---|---|
| **Immunization** | | **Treatment** | | **In-vitro** |
| **Exp.Groups** | **Immunization dose** | **Sub group** | **Article** | **activation** |
| | | **A1** | 12% Captisol® Placebo control | |
| | | **A2** | Compound 1 25µg/M | |
| **A** | 50µg/mouse | **A3** | Compound 1 50µg/M | Compound 1 RS 3.125-100µg/ml |
| | | **A4** | Compound 1 100µg/M | |
| | | **A5** | Compound 1 200µg/M | |

**Table 11-A: Final cytokine concentrations**

| **Final cytokine (pg/ml)(2.5 million cells/well)** | | | | |
|---|---|---|---|---|
| Compound 1 concentration | **Placebo** | **50µg/M** | **100µg/M** | **200µg/M** |
| 3.125µg/ml | 321.3 | 54.1 | 64.5 | 103.9 |
| 6.25µg/ml | 238.6 | 81.8 | 116.1 | 126.1 |
| 12.5µg/ml | 397.1 | 123.1 | 180.9 | 129.0 |
| 25µ/ml | 655.5 | 215.1 | 262.8 | 240.3 |
| 50µg/ml | 573.9 | 292.5 | 518.3 | 378.1 |
| 100µg/ml | 926.0 | 531.8 | 582.7 | 524.1 |
| Con A | 322.6 | 356.2 | 337.4 | BQL |

**Table 11-B: Final cytokine concentrations**

| **Final cytokine (pg/ml) (5 million cells/well)** | | | | | |
|---|---|---|---|---|---|
| Compound 1 concentration | **Placebo** | **25µg/M** | **50µg/M** | **100µg/M** | **200µg/M** |
| 3.125µg/ml | 522.3 | BQL | 76.2 | 90.8 | 204.4 |
| 6.25µg/ml | 634.8 | BQL | 109.2 | 157.8 | 244.1 |
| 12.5µg/ml | 962.8 | 41.9 | 179.5 | 257.1 | 466.1 |
| 25µ/ml | 967.4 | 70.0 | 277.9 | 421.7 | 660.5 |
| 50µg/ml | 1338.8 | 104.2 | 373.4 | 739.7 | 922.5 |
| 100µg/ml | 2010.2 | 185.2 | 547.0 | 995.5 | 1006.2 |
| Con A | 6839.8 | 2995.3 | 4837.0 | 10126.8 | 7722.8 |

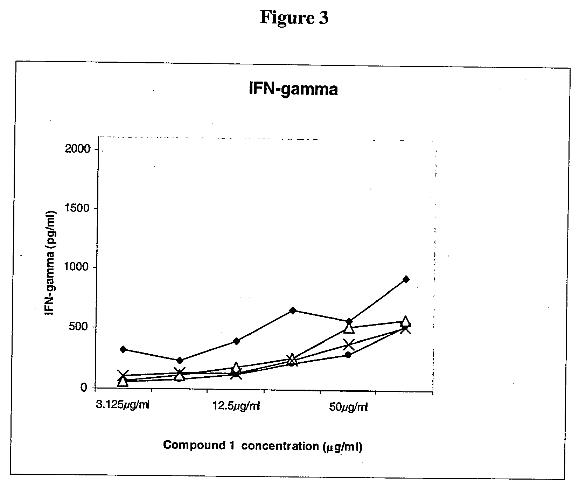

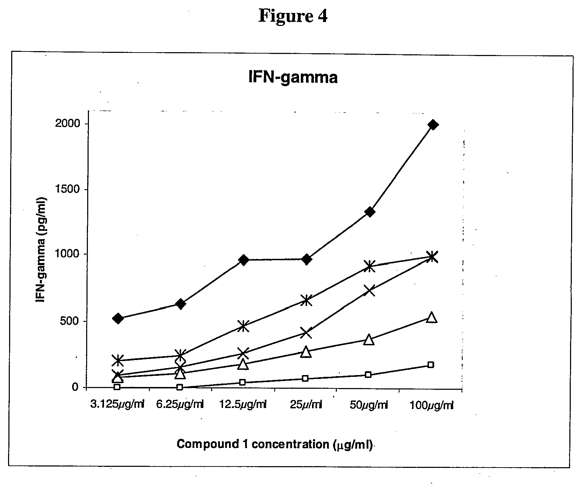

The results are also presented in Figures 3-4.

### Observations

### IFN-γ secretion

1. In the placebo group, a linear dose response upon Compound 1 activation in-vitro was shown. This graph resembles the graph obtained for the Ex-vivo model with the same immunization dose (50µg/mouse) and culturing medium (EM-1).
2. There was a dose response upon Compound 1 activation in vitro within all the tested groups.
3. Significant inhibition of IFN-γ secretion was seen with all the doses used for treatment (an average of 95% inhibition with treated dose of 25µg/mouse). A reverse correlation between the dose served for treatment and % inhibition can be found, mainly when 5x10⁶ cells/well were used. When 2.5x10⁶ cells/well were used, treatment of animals with 50µg/mouse gave better inhibition than 100 or 200µg. The point of 25µg is missing (lack of cells).
4. A better inhibition was seen when 5x10⁶ cells/well were used instead of 2.5x10⁶ cells/well.
5. In the linear range of the graphs, SD of % inhibition was low.
6. A technical problem with Con A is apparent when 2.5x10⁶ cells/well were used.

### TGF-β secretion

1. In the placebo group, no dose response upon in vitro activation with compound 1 was seen. TGF-β secreted level was below the detection limit of the ELISA in all other treatment groups.

### Example 6: Optimizations of freeze drying cycle with Compound 1 and Captisol® for injection (0, 0.5, 1.0 and 2.5 mg/vial)

### Purpose

The purpose of this study was to optimize the freeze drying cycle for Compound 1 with captisol® for injection to improve the shape of the lyophilization cake and avoid collapse and cracking. Thus it was decided to improve and optimize the lyophilization cycle. This cycle is transferred to the production lyophilizers for the manufacturing of the phase I batches.

### Process optimization

Batches of peptide at concentrations of 0.5 mg/ml 1.0mg/ml, 2.5mg/ml and Placebo were prepared and several freeze drying cycles were performed. The freeze drier used was an Edwards lyophilizer Lyoflex 0.6.

Solubility, water content and cake appearance were tested. According to the obtained results a new lyophilization cycle for Compound 1 was selected. Due to the high percentage of solids (12%) and hence the condensed cake, the new process is longer than the lyophilization cycle in Example 3 and exhibits an additional primary drying stage. Table 12 summarizes the differences between the processes.

**Table 12**

| Step | Lyoph. cycle for Compound 1 and Captisol® of Example 3 | **New Lyoph. cycle for Compound 1 and Captisol®** |
|---|---|---|
| Loading | 5°C | **5°C** |
| Freezing | 2 hours to -40°C | **6 hours to -45°C** |
| Hold at low temp. | 3 hours to -40°C | **3 hours to -45°C** |
| Primary Drying: | to 20°C | **to -20°C** |
| Stage I | 13 hours pressure 110µbar | **19 hours pressure 150µbar** |
| **Stage II** | - | **to 25°C** |
| | | **13 hours pressure 150µbar** |
| Hold at 20°C **(25°C)** | 13 hours pressure 110µbar | **8 hours pressure 150µbar** |
| Secondary drying: | | |
| Hold at 20°C | 5 hours pressure 10µbar | - |
| Storage at | 5°C | **5°C** |
| Process time | 36 hours | **49 hours** |

### Example 7: Effect of Compound 1 (administered in Captisol®) on lupus symptoms in the SLE-prone (NZBxNZW)F1 female mouse

Patients participating in clinical trials are to be treated with Compound 1 using Captisol^{®} (sulfobutyl ether beta-cyclodextrin sodium) as the excipient. For this reason, it was important to determine whether treatment of (NZBxNZW)F1 mice with the formulation of Compound 1 given in Captisol^{®} would have the same beneficial effects on lupus symptoms as observed when this strain of mice was treated with Compound 1 in PBS.

To this end, (NZBxNZW)F1 female mice (about 8 months old) were divided into 3 groups that were treated subcutaneously once a week for 10 weeks either with Captisol^{®} alone (n=8) or with 25 or 50 µg/mouse Compound 1 in Captisol® (n=9 and 10, respectively). These doses were selected since prior studies indicated tHat doses in this range were more effective in ameliorating SLE symptoms than the higher doses tested (100 and 200 µg/mouse). The same batch of drug substance was used in this study and in the first Phase I clinical trial with Compound 1.

The mice were followed for anti-dsDNA antibodies and for proteinuria. When the mice were sacrificed, the intensity of ICD was determined in kidneys.

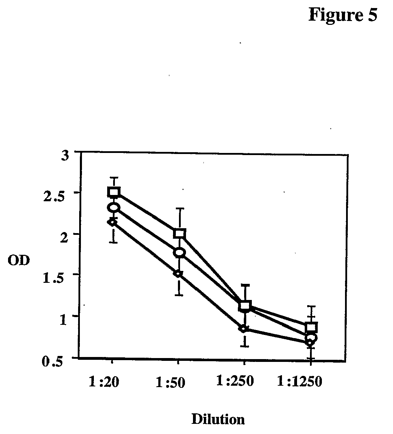

As can be seen in Figure 5, no significant differences between groups could be observed in the levels of dsDNA-specific antibodies after 10 treatment injections.

Table 13 also shows that the beneficial effect of treatment with Compound 1 could be observed starting from the 5^{th} injection and it was sustained up to the 10^{th} injection. The mean levels of proteinuria in the Captisol^{®} control group were consistently higher than in the Compound 1 -treated groups. Table 13 also shows that a reduction in the intensity of ICD was observed in kidneys of both Compound 1 dose groups. There was an overall trend showing that the lower dose (25 µg/mouse) was more effective than the higher dose (50 µg/mouse) in reducing the clinical symptoms of SLE in these mice.

**Table 13. Clinical Symptoms of SLE in (NZBxNZW)F1 Mice Treated with 25 or 50 µg/mouse Compound 1 (in Captisol^{®})**

| **Study Group** | **Mean Proteinuria ± SEM (g/L)** | | | | **ICD^{a} (Mean ± SEM)** |
|---|---|---|---|---|---|
| | **Number of Weeks Following Treatment Initiation** | | | | |
| | **5** | **7** | **8** | **10** | |
| Captisol^{®} | 1.81 ± 1.22 (n=8) | 5.74 ± 3.13 (n=8) | 4.5 ± 2.92 (n=7)^{b} | 4.46 ± 2.93 (n=7)^{b} | 2.29 ± 0.28 (n=7) |
| Compound 1 (50 µg/mouse) | 0.75 ± 0.3 (n=10) | 0.81 ± 0.3 (n=10) | 1.09 ± 0.4 (n=10) | 1.29 ± 0.3 (n=10) | 1.90 ± 0.23 (n=10) |
| Compound 1 (25 µg/mouse) | 0.16 ± 0.05 (n=9) | 1.26 ± 1.09 (n=9) | 0.5 ± 0.31 (n=9) | 0.56 ± 0.3 (n=9) | 1.22^{c} ± 0.32 (n=9) |

| | | | | | |
|---|---|---|---|---|---|
| ^{a} ICD=Immune Complex Deposits. ICD intensity scale: 0=none; 1=moderate; 2=severe; 3=severe/extremely intense. ^{b} The death of one animal with a high level of proteinuria resulted in a lower group mean. ^{c} p<0.05 (compared to Captisol^{®}-treated control mice; Mann-Whitney). | | | | | |

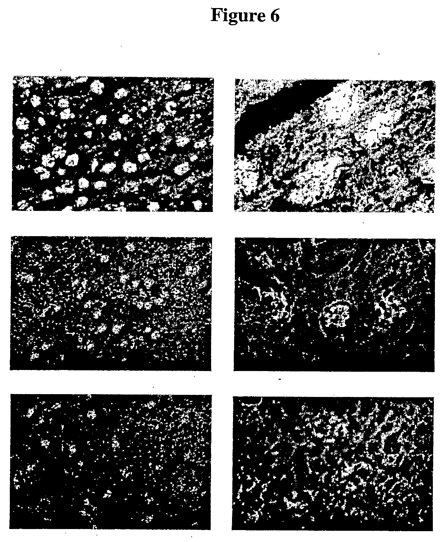

Figure 6 shows representative sections of one kidney from each treatment group. The top row sections are from a Captisol^{®}-treated mouse, the mid-row sections are from a mouse treated with 50 µg/mouse Compound 1 and the bottom row sections are from a mouse treated with 25 µg/mouse Compound 1. It can be seen that the intensity of immune complex deposits observed in kidney sections of mice treated with Compound 1 (dissolved in Captisol^{®}) at either dose level was much lower than that observed in the control group.

### Example 8: Phase I Clinical Study

### A Phase I, Multicenter, Randomised, Double-Blind, Placebo Controlled, Single Dose, Four-Arm Study to Assess the Tolerability and Safety of Compound 1 in Captisol® Subcutaneous Injection in SLE Subjects.

This was the first clinical study with Compound 1 in captisol® in humans, conducted in France. Its main objective was to evaluate tolerability and safety of Compound 1 in captisol®, administered as a single sc injection to SLE subjects. Its secondary objective was to evaluate immunological responses following a single sc dose of Compound 1 in captisol® in these subjects.

Thirty-six (36) subjects participated in the study. To be eligible for inclusion in the study, SLE patients must have fulfilled at least four criteria used for the diagnosis of lupus by the American College of Rheumatology. Patients must also have had stable, mild/moderate disease and score less than or equal to 10 on the SLE Disease Activity Index, SLEDAI.

Each patient received a single sc injection of reconstituted Compound 1 for injection or its matching placebo (Captisol^{®}) according to the following group assignment:
▪ ***Group A*:** Placebo (Captisol^{®})
▪ ***Group B*:** 0.5 mg Compound 1 in Captisol^{®}
▪ ***Group C*:** 1 mg Compound 1 in Captisol^{®}
▪ ***Group D*:** 2.5 mg Compound 1 in Captisol^{®}

A standard battery of safety tests, including blood and urine collection for laboratory tests, was performed at screening, during the day of dosing at 24 hours post-dose and at 2, 4 and 8 weeks following dosing. Prior to dosing, and on scheduled follow-up visits, blood samples were withdrawn for SLE-related immunological tests, anti-Compound 1 antibodies and PBL proliferation assay. The following immunology tests were performed:
▪ Coomb's (direct and indirect)
▪ C3, C4 and CH50
▪ Total IgG, IgM and IgA
▪ ANA, anti-dsDNA (Farr assay), anti-ssDNA
▪ Anti-ENA (including anti-La, anti-Ro, anti-RNP, anti-Sm)
▪ Anti-cardiolipin antibodies
▪ VDRL
▪ FTA antibodies
▪ Rheumatoid factor

The safety and tolerability of Compound 1 in captisol® in the subject population was evaluated on the basis of the following criteria:
▪ Occurrence of AEs, including SLE flare
▪ Vital signs
▪ 12-lead ECG
▪ Changes in physical examination
▪ Routine clinical laboratory tests
▪ SLEDAI score
▪ Immunological test results

### Phase Ia clinical study details

**Study Principal Investigators and Respective Study Sites:** Six (6) study centers in France:Prof. Jean Charles Piette (Hopital La Pitie Salpetriere, Paris), Prof Oliver Meyer (Hopital Bichat, Paris), Prof. Jean Revuz (Hopital Henri Mondor, Creteil), Prof. Loic Guillevin (Hopital Avicenne, Bobigny), Prof. Eric Hachulla (Hopital Claude Huriez, Lille Cedex), Prof.- Xavier Mariette (Hopital Bicetre, Kremlin Bicetre).

### Compound 1 (in captisol®), Placebo, Water for Injection-Ampoules, Dose and Mode of Administration:

Vials of Compound 1 in Captisol^{®} (120mg/vial) were injected subcutaneously as a single dose per subject in the following dosages:
0.5 mg Compound 1/vial in Captisol^{®}, 1mg Compound 1/vial in Captisol^{®} and 2.5 mg Compound 1/vial in Captisol^{®}.

Placebo for Compound 1: 120 mg Captisol^{®}/vial (identical in appearance to vials of Compound 1 in Captisol^{®}).

### Methodology

This was a multi-center, randomized; double blind, placebo-controlled, four-arm study, using a single subcutaneous injection of Compound 1 or placebo. SLE patients were screened up to 21 days prior to baseline procedures. Each eligible subject was randomized to one of the 4 treatment groups: subcutaneous injection of either 0.5, 1 or 2.5 mg Compound 1 or its matching placebo. All subjects were admitted to the clinic on pre-dosing day. Each subject received a single dose of one of the above listed treatments. Subjects were discharged from the clinic 24 hours after dosing. Subjects were further monitored at weeks 2, 4 and 8 following dosing. Blood samples (serum and whole blood) for safety laboratory tests were withdrawn at Screening, Dosing Day (pre-dose), Day 2 (post dose), at Weeks 2, 4 and 8 (Termination visit). Blood samples for immunological tests were withdrawn at: Screening, Dosing Day (pre-dose) and at Weeks 4 and 8. Peripheral blood lymphocytes (PBL) proliferation was evaluated at Dosing Day (pre-dose) and at Weeks 2, 4 and 8.

### Number of Subjects (total and for each treatment):

Thirty six (36) subjects were randomized into this study as follows; 9 subjects into the 0.5 mg treatment group, 9 subjects into 1 mg treatment group, 10 subjects into the 2.5 mg treatment group, and 8 subjects into the placebo treatment group.

### Diagnosis and Main Criteria for Inclusion:

Eligible subjects for this study were SLE patients who fulfilled at least four diagnostic criteria of the American College of Rheumatology (ACR). Their disease condition had to be stable, mild to moderate with a score equal to or less than 10 on the SLE disease activity index, year 2000 updated (SLEDAI 2K).

Excluded from participation were SLE patients who reported unstable or severe asthma, stroke, acute myocardial infarction, unstable angina, cerebral hemorrhage and pulmonary embolism during the six months prior to study screening. SLE patients wno had any clinically significant or unstable medical or surgical conditions, diabetes mellitus, liver disease (cirrhosis, active hepatitis, portal hypertension, and/or ascites), clinically significant hypertension, a medical history of any malignancy, dialysis, or chronic obstructive pulmonary disease (COPD) were also excluded from study participation.

Also excluded from study participation were SLE patients who underwent plasmapheresis or were treated during the three months prior to screening with one of the drugs listed below: prednisone 30mg/day or greater (or an equivalent dose of another corticosteroid), intravenous corticosteroids, intravenous immunoglobulin G (IgG), oral anticoagulants and any cytotoxic agents (e.g. azathioprine, chlorambucil, cyclophosphamide, mycophenolate mofetil, methothrexate, tacrolimus.

In addition, SLE patients initiating treatment with corticosteroids (more than ± 10 mg/day prednisone, or an equivalent dose of another corticosteroid) and/or anti-malarials, during the last 3 months prior to screening were excluded from the study.

While an effort was made to retain baseline SLE medical treatments throughout the course of the study, investigators could nevertheless change participant medical treatment at any time during the study to maintain and optimize patient welfare.

### Criteria for Evaluation

### Safety:

The following safety parameters were assessed at Screening, during the hospitalization and at follow-up visits including Termination visit: vital signs (systolic blood pressure, diastolic blood pressure, pulse, oxygen saturation, temperature and weight), 12-lead ECG, change in physical examination and clinical routine laboratory safety tests. Adverse events were recorded at the Dosing Day and at each visit thereafter.

### Immunology:

SLE-related immunological tests were performed at Screening, during the hospitalization and at follow-up visits including Termination visit.

Drug-related immunological responses were followed by using the PBL proliferation assay and anti-Compound 1 antibodies assay at the Dosing Day and at follow-up visits including Termination visit.

### Disease Activity:

Disease activity assessment using the SLE disease activity index score, year 2000 updated (SLEDAI 2K) was assessed at Screening, during the hospitalization and at follow-up visits including Termination visit.

### Statistical Methods:

SAS® version 9.0 software was used to analyze and present data collected during this study. No power calculation was performed and no formal hypothesis testing was conducted for this Phase Ia study.

### Adverse Experiences

The incidence and the frequency of adverse experiences was presented by System Organ Class and preferred terminology according to MedDRA dictionary version 5.0. The data is tabulated by treatment group.

### Clinical Laboratory Data

Descriptive statistics of laboratory values including number of observations, mean, standard deviation, minimum and maximum were determined for Screening, Day 1 (pre dose), Day 2, Week 2, 4 and 8 are presented by treatment group. Changes from baseline to each time point/visit are also presented for each visit by treatment assignment. Percent of abnormal results (low and high, where applicable) are presented on a parameter basis, by treatment group and visit/time point. Shift analyses from baseline to 24-hours post dose and from baseline to termination visit were performed.

### Vital Signs

Descriptive statistics for vital signs including number of observations, mean, standard deviation, median, minimum and maximum values were determined for Screening, Day 1 (pre and post dose, and at each time point) Day 2, Weeks 2, 4 and 8 are tabulated by the assigned treatment. Changes from baseline to each time point/visit is presented in by visit and treatment assignment.

### Weight

Descriptive Statistics of Weight (kg) at baseline, termination and change from baseline is presented by treatment group.

### ECG

Descriptive statistics of ECG parameters at baseline, termination and changes from baseline are presented. Shift analysis is presented as tables of shift from baseline to termination between normal/abnormal or present/absent ECG parameters. Potentially clinically significant (PCS) QTc (Bazett) measurements were identified according to the predefined criteria. Tables of shift analysis between PCS and non-PCS Absolute QTc (Bazett) and incidence table of PCS change in QTc (Bazett) from baseline to any visit are presented.

### Physical Examination

Physical examination results are analyzed by incidence of subjects with abnormal or normal findings for each body system at Baseline and Termination visit. Shitt analysis between normal to abnormal and vice versa was also applied. When no change from baseline occurred, it was defined as "other".

### Compound 1 related immunological tests

For immunological parameters, descriptive statistics, including number of observations, mean, standard deviation, median, minimum and maximum values were calculated and are presented by treatment group and visit. Change from baseline to each follow-up visit is also presented by treatment group. Where applicable, number and percent of subjects with negative/positive results is presented by treatment group and visit.

### SLEDAI 2K

Descriptive statistics, including mean, standard deviation, median, minimum and maximum values of SLEDAI 2K are presented.

### Results of Phase Ia clinical study:

### Subject Disposition and SLE Characteristics

Thirty six (36) study subjects entered and completed this study per protocol. The majority of subjects (34) in all treatment groups were female (94.4%) and Caucasian (30, 83.3%). The mean age for all treatment groups was 35.6 years (range of means from 32 to 39 years). Most of the subjects (91.7%) had between 4 to 6 American College of Rheumatology (ACR) diagnostic criteria and a mean group SLEDAI 2K score ranged from 2.1 to 4.1.

### Safety Results

There was no prominent difference between study drug treatment groups and the placebo group in the incidence of AEs. The most common AEs in all groups were headache, classified as mild or moderate in nature and injection site reaction classified as mild in nature. Dose response was not seen. No serious adverse event (SAE) or AE classified as severe occurred during the study.

No clinically significant effect attributable to study drug was seen for hematology, biochemistry or urinalysis values.

No clinically significant effect attributable to the study drug was seen for vital signs parameters (systolic blood pressure, diastolic blood pressure, pulse, oxygen saturation).

No clinically significant effect attributable to the study drug was seen for temperature and weight.

No differences of clinical significance were seen between Compound 1 -treated groups and placebo for categorical ECG measurements and digitized ECG parameters. No PCS QTc absolute value and no QTc change from baseline > 60 msec was recorded. A similar number of subjects in Compound 1 -treated and placebo groups had QTcB change from baseline between 30 and 60 msec.

No clinically significant effects of Compound 1 on physical exam were noted.

### Immunology Results

Evaluation of serum samples from all subjects indicated that a single subcutaneous administration of Compound 1 at the dose levels of 0.5, 1 and 2.5 mg/patient did not induce the development of anti-Compound 1 specific antibodies. Seven subjects had a response to Compound 1 above the cut-off. These elevated levels of antibodies were already present prior to dosing. No increase in the levels of antibodies was observed in the follow up period (two months) of the study. The sera of these subjects were analyzed for the isotype of the reactive antibodies. The response in two of the subjects was associated with the IgM isotype and with the IgG isotype in two others. None of the seven had specific IgE antibodies.

The peripheral blood lymphocytes (PBL) assay showed that 50 % of the subjects (18) were classified as responders (SI>2) with similar distribution in all treatment groups. The T cell response was relatively low and no association between Compound 1 treatment dose or concentration used in the assay and responder non-responder status could be detected, taking into consideration that only a single SC dose of the study drug was administered. Also, no indication of increased incidence of responder status over time was observed. The tetanus toxoid (TTX) assay that serves as a safety control shows that the response to TTX was preserved throughout the study period in all treatment groups indicating that Compound 1 in captisol® did not change the immunological response to TTX recall antigen.

The immunological findings are the result of the administration of only a single dose of the study drug Compound 1.

### Disease Activity Results

No clinically significant effects of Compound 1 on the SLEDAI score (change of ≥ 3, ≤ 12 points) were noted during the study except for one subject in the 0.5 mg treatment group for whom a change in the SLEDAI score of 2 to 10 points was recorded between baseline and week 4 on the basis of an urinalysis showing pyuria. This urinalysis finding was not confirmed by the investigator as a lupus flare per protocol definition and was resolved with no treatment change.

### Conclusions

This Phase Ia study showed that a single subcutaneous injected dose of Compound 1 of 0.5, 1 or 2.5 mg in 120 mg Captisol ® was safe and well tolerated and allows continuation to a phase Ib multiple dose study.

### Example 9: Phase Ib Clinical Study

### A Phase I, Multicenter, Bi-National, Randomized, Double-Blind, Four-Arm, Placebo Controlled, Multiple Dose Study to Assess the Tolerability and Safety of Compound 1 in Captisol® Subcutaneous Injections in SLE Subjects

This study is being performed in order to evaluate the safety and tolerability of repeated Compound 1 sc administration to SLE subjects. The study's secondary objective is to evaluate immunological responses following repeated sc administration of Compound 1 in Captisol® in SLE subjects.

Compound 1 is given in doses of 0.5, 1.0 or 2.5 mg in Captisol^{®}. The investigational product is administered every other day (excluding weekends) for a total of 12 sc injections, i.e. 3 doses a week for 4 weeks. Subjects are monitored on planned visits scheduled at 2, 4, 8 and 12 weeks after start of dosing. Safety and tolerability are evaluated using tests similar to those described in the Phase Ia Clinical Study above.

### Results

This Phase Ib study shows that multiple subcutaneous injected doses of Compound 1 of 0.5, 1 or 2.5 mg in 120 mg Captisol ® are safe and well tolerated.

### Example 10. Detection of antibodies against peptides Ia, IIa and IIIa, and anti-1616 Id antibodies in the sera of SLE patients and healthy controls

Human SLE patients (32 patients) were bled and their sera were tested by ELISA for their ability to bind the peptides Ia, IIa and IIIa, a control peptide p195-212 (a myasthogenic peptide described in PCT publication No. WO 94/00148) or mAb 5G12.

Detection of the antibodies was conducted on plates that were coated with 10µg/ml of peptides Ia, IIa, IIIa, p195-212 or mAb 5G12, in PBS for 2 hr, washed and blocked with 1% ovalbumin in PBS for an additional 2 hr. ELISA was continued as described after blockage in Example 2 of PCT International Publication No. 96/30057, using goat anti-human IgG polyclonal antibody conjugated to peroxidase.

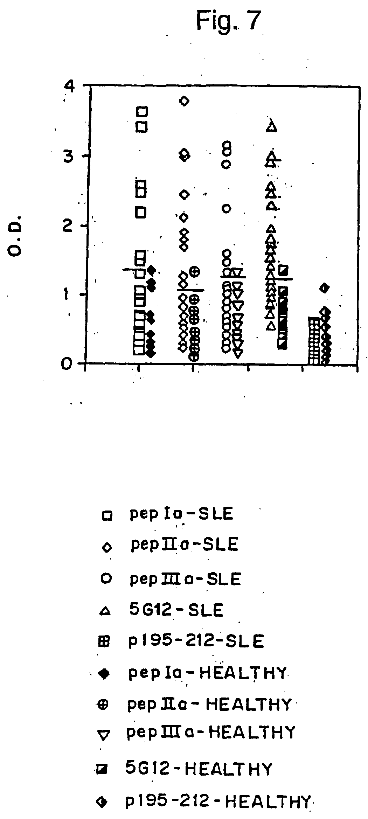

As shown in Fig. 7, SLE patients exhibited significantly higher levels of antibodies that bind either peptide Ia (open squares), IIa (open diamonds), IIIa (open circles), or mAb 5G12 (open triangles), in comparison to healthy controls (peptide Ia-healthy = closed diamonds; peptide IIa-healthy = crossed circles; peptide IIIa-healthy = inverted open triangles; 5G12-healthy = half filled squares). No binding could be observed when either sera of patients or controls were tested on plates coated with the non-relevant peptide p195-212 (p195-212-SLE = crossed squares; p195-212-healthy = half filled diamonds). The results indicate a correlation between the whole antibody molecule and the CDR-based peptides on the level of antibody titers.

### Example 11. Proliferation of PBL from SLE patients and healthy controls in the presence of human 16/6Id mAb and peptides

Peripheral blood lymphocytes (PBL) were isolated from the blood of SLE patients or healthy controls using ficol gradient. Thereafter, the PBL were incubated in the presence of different concentrations of the peptides Ia, IIa or IIIa, or the human 16/6 Id mAb for 24 hr, when a sample was taken for IL-2 measurement. The assay was continued for a total of 7 days, and ³H-thymidine was added for the last 16 hr. Proliferation was detected by reading the amount of radioactivity incorporated into the DNA of the cells.

As is seen in Table 14, a lower proportion of the PBL taken from SLE patients reacted to the peptides or to the 16/6 Id mAb, when compared to the healthy controls. The results are expressed in percentage of responder (34% in the first line) and the actual number of patients (11 out of 32: 11/32).

Similar results were obtained when the levels of the IL-2 produced by the PBL in the presence of the peptides or the 16/6 Id mAb were tested, as shown in the next example.

**Table 14. Proliferation of PBL from SLE Patients and Healthy Controls in Presence of mAb 16/6 Id and Peptides Ia-IIIa EMI29.1**

| | | SLE Patients | | Healthy Controls |
|---|---|---|---|---|
| 16/6 Id | 34% | 11/32 | 72% | 5 |
| pep Ia | 21% | 7/32 | 44% | 11/25 |
| pep IIa | 9% | 3/32 | 28% | 7/25 |
| pep IIIa | 31% | 10/32 | 60% | 15/25 |

### Example 12. Production of IL-2 by PBL of SLE patients and healthy controls in the presence of human mAb 16/6 Id and peptides

PBL were isolated from blood of SLE patients or healthy controls using ficol gradient, and were incubated as in Example 11. A sample of 50µl was removed 24 hr after the assay was started, and incubated in the presence of IL-2 sensitive cells (CTLD) for 24 hr, after which ³H-thymidine was added for 16 hr, and the plates were harvested and counted on a beta counter.

As in Table 14, it can also be seen from Table 15 that a lower proportion of the PBL taken from SLE patients reacted to the peptides or to the 16/6 Id mAb, when compared to the healthy controls, thus indicating that the response to the peptide corresponds to that of T cells of the patient to the pathogenic human autoantibody.

**Table 15: Production by PBL of SLE Patients and Healthy Controls in Presence of mAb 16/6 Id and Peptides Ia-IIIa**

| | | SLE Patients | | Healthy Controls |
|---|---|---|---|---|
| 16/6 Id | 31% | 10/32 | 66% | 17/25 |
| pep Ia | 16% | 5/32 | 56% | 14/25 |
| pep IIa | 90/o | 3/32 | 32% | 8/25 |
| pep IIIa | 16% | 5/32 | 64% | 16/25 |

### Example 13.

### Synthesis of the human peptides hCDR1 and hCDR3

The human hCDR1 (SEQ ID NO: 6) and hCDR3 (SEQ ID NO: 7) are shown below.
GYYWSWIRQPPGKGEEWIG (hCDR1)
YYCARGLLRGGWNDVDYYGMDV (hCDR3)

The peptides were prepared by methods well-known in the art, for example, by chemical solid phase or solution phase synthesis using an automated synthesizer by using the manufacturer's protocols for t-butyloxycarbonyl (t-Boc), fluorenylmethoxycarbonyl (Fmoc) or other alpha-amino acid protecting group procedure essentially as described (see, for example, Peptides: Synthesis, Structure and Applications, ed. By B. Gutte, Academic Press, 1995; Peptide Synthesis Protocols, ed. by M. Pennington and B. Dunn, Humana Press,1994 ; Schnolzer M. et al., In situ neutralization in Bocchemistry solid phase peptide synthesis. Rapid, high yield assembly of difficult sequences. Int. J. Pept. Protein Res. 40: 180-193,1992).

### Example 14

### Peptides hCDR1 and hCDR3 inhibit the proliferative response of PBL of SLE patients to the human16/6Id mAb.

Sixty-two patients, 9 males (14.5%) and 53 females (85.5%) with SLE participated in the study. The mean age at diagnosis was 32.95 ±12.92 (range 12-61) years and the mean follow-up period was 10.98 ± 10.76 (range 1-32) years. All patients fulfilled at least 4 of the American College of Rheumatology (ACR) revised diagnostic criteria for SLE (Tan E.M. et al., Arthritis Rheum 25: 1271-77 (1982)). Patients were recruited from three Israeli Medical Centers (Kaplan, Rehovot; Ichilov, Tel Aviv; Asaf-Harofeh, Rishon Lezion). Disease activity was determined according to the SLEDAI lupus activity index (Bombardier C. et al., Arthritis Rheum 35: 630-40 (1992)). A control group of 36 sex- and age-matched healthy control volunteers was studied concomitantly with the SLE patients. The study was approved by the Ethical Committee of the Medical Center.

It was of interest to investigate whether the peptides hCDR1 and hCDR3, which are based on the CDR1 and CDR3 of the human 16/6Id mAb, are capable of inhibiting the specific proliferative responses of PBL of SLE patients to the human 16/6Id mAb. To this end, we first had to identify the patients whose PBL could be stimulated to proliferate by the human 16/6Id mAb (responders).

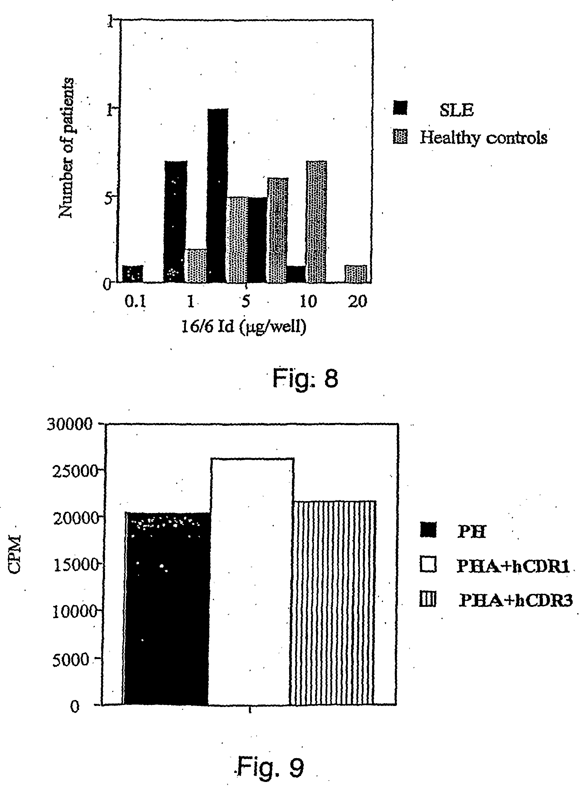

Therefore, PBL of 62 consecutive SLE patients were cultured in the presence of the human 16/6 Id and their proliferative responses and ability to secrete IL-2 were determined. PBL of 24 out of the total of 62 (39%) and of 23 out of 55 (42%) SLE patients tested responded (SI ≥ 2, range 2-5.6) by proliferation and by IL-2 secretion (SI ≥ 2, range 2-60), respectively. The frequency of responders in the group of SLE patients was lower than that observed in the group of healthy donors that was tested as control. Thus, PBL of 21 out of a total of 36 (58%) healthy donors responded by proliferation to the 16/6 Id. The extent of proliferation (SI levels) was similar for the SLE patients and for the healthy controls who responded to the 16/6 Id. However, as shown in Figure 8, the optimal response to the 16/6 Id of PBL of the control donors was observed at higher concentrations of 16/6 Id as compared to the SLE patients.

No differences could be demonstrated between gender and age of SLE patients that responded to the 16/6 Id and of the non-responder group of patients. However, the patients whose PBL proliferated in response to the 16/6 Id were sick for a shorter period of time (a mean of 9.78±8.36 vs. 11.73±12.06 years for responders and non-responders, respectively; P ≤ 0. 036). Table 16 summarizes the clinical characterization of the 16/6 Id responder and non-responder groups of SLE patients. As can be seen in the Table, both groups were similar in most SLE related clinical manifestations. The SLE disease activity score (SLEDAI) and the number of SLE diagnostic criteria were also similar in the two groups. Nevertheless, a higher frequency of neurological (both seizures and psychosis) and hematological involvement and a lower rate of renal involvement were noted in the responder group of patients in comparison to the group of non-responders. However, probably because of low number of patients in the relevant subgroups, the above differences did not reach statistical significance. Moreover, relatively less responder patients were determined between those treated with either steroids or cytotoxic agents at the time of the study. It is noteworthy that significantly more patients who never received steroids responded to the 16/6 Id in comparison to the non-responder group (54% vs 21% ; P=0.023).

It is noteworthy that the efficacy of the CDR-based peptides to inhibit the proliferative responses of PBL of healthy donors to the 16/6 Id was much lower than that observed for PBL of SLE patients (not shown).

**Table 16. Clinical and laboratory characterization of SLE patients.**

| **A: Diagnostic Criteria*** | | | |
|---|---|---|---|
| | All Patients | Responders | Non-responders |
| Number of Patients (%) | 62(100) | 24(39) | 38(61) |
| Malar rash | 19/62 (30.1) | 8/24 (33.3) | 11/38(29) |
| Discoid rash | 9/62 (15) | 3/24 (12.5) | 6/38 (16) |
| Photosensitivity | 21/62 (34) | 9/24 (37.5) | 12/38 (32) |
| Mucosal ulcers | 17/62 (27.4) | 8/24 (33.3) | 9/38 (23.7) |
| Arthritis | 46/62 (74.2) | 19/24 (79.2) | 27/38 (71) |
| Serositis | 14/62 (22.6) | 5/24 (20.8) | 9/38 (23.7) |
| Neurologic | 5/62(8.1) | 4/24 (16.7) | 1/38 (2.7) |
| disorders ^{‡} | | | |
| Renal disorder ^{‡} | 24/62 (38.8) | 7/24 (29.2) | 17/38 (44.8) |
| Hematological disorders ^{‡} | 44/62 (71) | 19/24 (79.2) | 25/38 (65.8) |
| ANA | 61/62 (98.4) | 24/24 (100) | 37/38 (92.1) |
| A-dsDNA | 54/62 (87.1) | 19/24 (79.2) | 35/38 (92.1) |
| APLA | 35/62 (56.5) | 12/24 (50.0) | 23/38 (60.53) |
| **B: Disease Activity** | | | |
| SLEDAI Score | 6.65±5.12 | 7.29±1.06 | 6.24±0.84 |
| Number of ACR | 5.44±1.39 | 5.54±0.33 | 5.34±0.2 |
| diagnostic criteria | | | |

| **C: Current Treatment** | | | |
|---|---|---|---|
| NSAIDS | 17/62 (27.4) | 6/24 (25) | 11/38 (29) |
| Anti-Malarial | 37/62 (59.7) | 15/24 (62.5) | 22/38 (57.9) |
| Steroids^{‡} | 33/62 (53.2) | 11/24 (45.8) | 22/38 (57.9) |
| Cytotoxic^{‡} | 10/62 (16.1) | 2/24 (8.3) | 8/38(21) |

| | | | |
|---|---|---|---|
| *Clinical involvement was defined according to the ACR revised criteria. Antinuclear antibodies (ANA) and anti-dsDNA antibodies were determined by Hep2 cells and *Crithidia luciliae,* respectively. Anti-phospholipid antibodies (APLA) were defined as activity in one or more of the following assays: false positive VDRL, lupus anti-coagulant (LAC) or ELISA for anticardiolipin antibodies. † The anti-malarial agent, hydroxychloroquine, was used at a dose of 200-400 mg/day ; Steroid treatment was defined as a daily dose: 5mg of prednisone ; cytotoxic agents used were cyclophosphamide (0.75-1.0g/m² ; monthly) or azathioprine (100-150 mg/day). ‡ Parameters for which tendency was observed towards differences between the two groups of responder and non-responder SLE patients. | | | |

To test the ability of the peptides hCDR1 and hCDR3 to inhibit the proliferative response of PBL of SLE patients to the human 16/6Id mAb, PBL (2x10⁵/well) of SLE patients were stimulated in vitro in triplicates with different concentrations (0.1-20µg/well ) of the human 16/6Id mAb in the absence or presence of the peptides hCDR1 and hCDR3 (either 50 or 100 µg/well). Following 6 days of incubation, ³H-thymidine (0.5 µCi of 5 Ci/mmol) was added to each well for additional 18 hours of incubation. Cells were then harvested and radioactivity was counted using a β-counter. Results were expressed as mean counts per minute (cpm) of triplicate cultures. Stimulation indices (the ratio of mean cpm at the optimal concentration of 16/6Id to mean cpm without 16/6Id) were then calculated. A stimulation index (SI)≥2 was considered positive.

PBL of 24 out of the total of 62 (39%) SLE patients were found to proliferate to the 16/6Id mAb. The ability of the peptides hCDR1 and hCDR3 to inhibit the proliferative responses to the whole molecule of the 16/6Id autoantibody was tested on PBL of 19 responders SLE patients.

Table 17 shows the results of these experiments. Inhibition of above 50% of the proliferative capacity was considered positive. The Table represents the highest positive inhibition capacity for each peptide. It can be seen that the human hCDR1 and hCDR3 inhibited the proliferation of PBL of 16/19 (84.2%) and 15/19 (78.9%), respectively, of the 19 responders tested. Both peptides inhibited the proliferation of PBL of 18/19(95%) of responders tested. It can also be seen in the Table that the magnitudes of inhibitions were similar for both peptides. Thus, it can be concluded that peptides based on CDR1 and CDR3 of the human 16/6Id mAb are efficient inhibitors of the proliferation of PBL of SLE patients to the human 16/6Id mAb.

**Table 17**

| **Inhibition of proliferation of PBL of SLE patients by peptides hCDR1 and hCDR3.** | | |
|---|---|---|
| **Number** | | **Percent Inhibition** |
| | **hCDR1** | **hCDR3** |
| **1.** | **62** | **<50** |
| **2.** | **70** | **75** |
| **3.** | **69** | **<50** |
| **4.** | **<50** | **<50** |
| **5.** | **88.5** | **87.5** |
| **6.** | **80** | **80** |
| **7.** | **76** | **70.4** |
| **8.** | **58** | **56** |
| **9.** | **69.5** | **65** |
| **10.** | **68.2** | **71.8** |
| **11.** | **<50** | **72** |
| **12.** | **82** | **86** |
| **13.** | **63** | **64** |
| **14.** | **56** | **74** |
| **15.** | **63** | **69** |
| **16.** | **<50** | **68** |
| **17.** | **70.5** | **77.8** |
| **18.** | **51.5** | **<50** |
| **19.** | **63** | **60.8** |
| **Mean±SD** | **68.12±9.57** | **71.82±8.44** |

### Example 15

### Specificity of the inhibitory capacity of hCDR1 and hCDR3

It is important to demonstrate that the inhibitory effects of the hCDR-based peptides are specific to SLE-associated responses. To this end the peptides hCDR1 or hCDR3 were added to cultures of PBL of SLE patients that were stimulated with the mitogen phytohemagglutinin (PHA,2 µg/ml). The results of such an experiment performed with PBL of one SLE patient is shown in Figure 9. The peptides hCDR1 and hCDR3 could not inhibit the proliferative responses (expressed in cpm) of the PBL to the mitogen PHA and the proliferative responses were similarly high in the absence (black column) or presence of either hCDR1 or hCDR3.

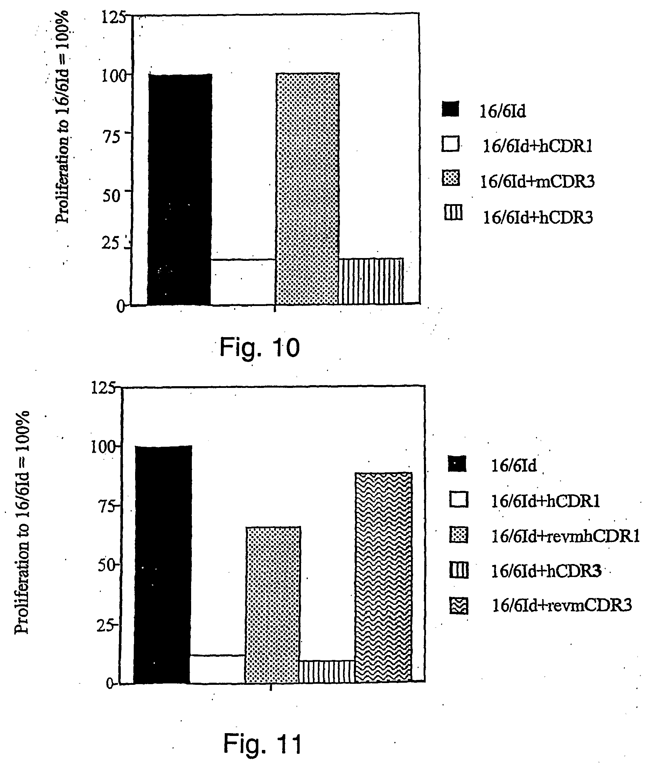

In another experiment, cultures of PBL of SLE patients were stimulated with the human 16/6Id mAb and then incubated with the human peptides hCDR1 or hCDR3 or with the peptide IIIa as a control. The results of such an experiment performed with PBL of one SLE patient are shown in Figure 10. As shown in Figure 10, whereas both peptides hCDR1 and hCDR3 based on the human autoantibody inhibited efficiently the proliferative responses of PBL to the human 16/6Id mAb, the peptide mCDR3 based on the CDR3 of the murine antibody (i.e. peptide IIIa) did not inhibit the proliferation.

Two additional control peptides were used in these experiments, namely peptides synthesized at the reversed order of the Ia and IIIa peptides, and the results are shown in Figure 11. It can be seen that the two reversed peptides failed to inhibit significantly the proliferative responses of the PBL of the SLE patient to human 16/6Id mAb while peptides hCDR1 and hCDR3 did inhibit efficiently the proliferation, demonstrating that the inhibition of proliferation by the human hCDR-based peptides is specific to the peptides and to the SLE-associated T cell responses.

### Example 16

### Down-regulation of the secretion of IL-2 by PBL of SLE patients in the presence of the peptides hCDR1 and hCDR3

It was of interest to find out whether the hCDR peptides are capable of inhibiting IL-2 secretion by PBL of SLE patients following stimulation with the human 16/6Id mAb. Such inhibition might also suggest that the human CDR-based peptides inhibit the proliferative responses to the 16/6Id mAb at least partially by down-regulating IL-2 secretion. To this end, PBL of SLE patients were incubated with the human 16/61d mAb in the absence or presence of the peptides hCDR1 or hCDR1 Supernatants of the cultures were collected following 48 hours of incubation. Assays to determine levels of IL-2 in the supernatants were performed using the CTLL IL-2 dependent line. Briefly, cells of the CTLL line (2x10⁴/well) were incubated in the presence of the different supernatants for 24 hours, followed by the addition of ³H-thymidine for an additional 18-hour incubation period. Cells were then harvested and radioactivity counted using a β-counter. Results were calculated based on recombinant human IL-2 used as a standard. The ability of the peptides to inhibit the IL-2 secretion of PBL of 23 responders stimulated by the human 16/6 Id was tested. The results, summarized in Table 18, show that hCDR1 and hCDR3 inhibited the secretion of IL-2 by PBL of 21/23 and 19/23 patients, respectively. Inhibition of proliferative responses of PBL directly correlated with IL-2 inhibition by the CDR-based peptides. Thus, inhibition of IL-2 secretion was observed in all cases where inhibition of proliferation were determined.

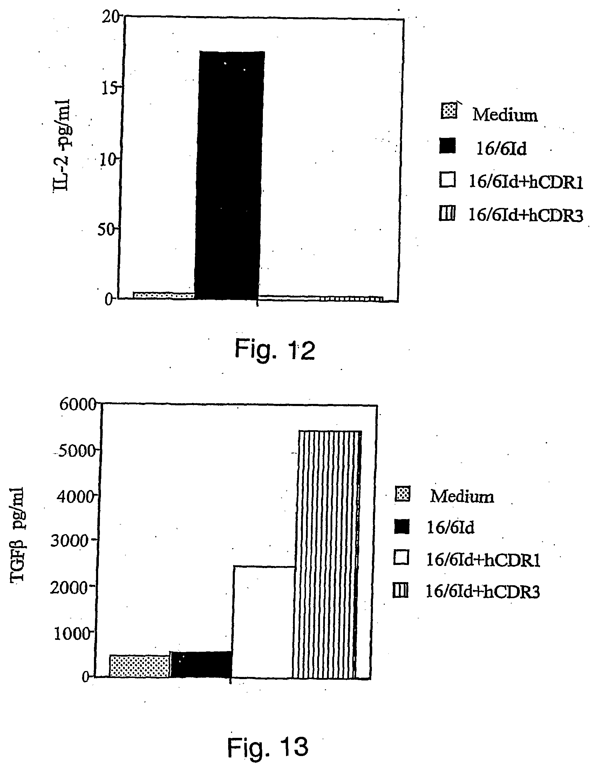

The results obtained with PBL of one SLE patient represented in Figure 12 (secretion of IL-2 is expressed in pg/ml) show that both hCDR1 and hCDR3 inhibited 100% of the IL-2 secretion by PBL of a SLE patient triggered by the human 16/6Id mAb.

**Table 18. Inhibition of IL-2 secretion by hCDR1 and hCDR3**

| Peptide | Inhibitory | Maximum |
|---|---|---|
| | Activity* | inhibition |
| | % | % |
| hCDR1 | 91 (21/23) | 84±31 |
| hCDR3 | 83 (19/23) | 78±34 |

| | | |
|---|---|---|
| *IL-2 secretion in the presence of 16/6 Id alone was considered as 100%. Inhibition of 50% or more was considered significant. | | |

### Example 17

### Up-regulation of the secretion of the immunosuppressive cytokine TGF-β by CDR-based peptides

In attempts to shed light on the mechanisms by which the human CDR-based peptides inhibit the proliferative responses to the human monoclonal anti-DNA 16/6Id antibody, the levels of the immunosuppressive cytokine TGF-β in the supernatants of the cell cultures were determined. The rationale behind these experiments is based on our previous findings of elevated levels of TGF-β in cultures of splenocytes of mice with SLE either induced with the human anti-DNA 16/6Id mAb or spontaneous {(NZB x NZW) F1 mice} following treatment with the peptides based on mouse CDR (Eilat, E. et al., Proc. Natl. Acad. Sci. U. S. A., 98, 1148 (2001)). The elevation in the levels of TGF-β correlated with amelioration of disease manifestations in the treated mice.

For this purpose, supernatants were removed from cultures of PBL of various SLE patients following 48 hours incubation with the human 16/6Id mAb in the absence or presence of the peptides hCDR1 or hCDR3. TGF-β was determined by ELISA according to the manufacturer's instructions. Briefly, Maxisorb plates (Nunc) were coated with recombinant human TGFβsRII/Fc chimera (R & D Systems) diluted in PBS (100 ng/ml). After blocking, cell supernatants were added. After 18 hours incubation the detecting biotinylated anti-human TGF-β antibody (R & D Systems) was added. The substrate solution used was the TMB Colour Reagent (Helix Diagnostics) and enzyme activity was evaluated by the MRX ELISA reader using the 570 nm and 630 nm filters. The results are summarized in Table 19.

The results in Figure 13 demonstrate that peptides hCDR1 and hCDR3 triggered a significant up-regulation in the secretion ofTGF-ss (expressed in pg/ml) by the PBL of one representative SLE patient that were stimulated with the pathogenic human 16/6Id mAb.

**Table 19**

| **Up-regulation of TGF-β secretion of 16/6 Id-induced stimulation of PBL of SLE patients with hCDR1 and hCDR3 peptides.** | | |
|---|---|---|
| Peptide | Up-Regulation of TGF-β % | Maximum Up-regulation % |
| hCDR1 | 100(19/19) | 305±221 |
| hCDR3 | 100 (19/19) | 338±242 |

Secretion of TGF-β in the presence of 16/6 Id alone (mean63625 pg/ml) was considered as 100%. Results are expressed as percent secretion above that in the presence of16/6Id alone.

### Example 18

### Activity of MMP-9 (but not of MMP-2) is elevated in sera of SLE patients

In the present example, we determined the levels of MMP-9 and MMP-2 in sera of 40 patients with SLE and we demonstrate that MMP-9 but not MMP-2 activity is significantly elevated in sera of SLE patients compared to healthy controls. High MMP-9 activity correlated with the presence of discoid rash, Raynaud phenomenon, pneumonitis, mucosal ulcers and the presence of anti phospholipid antibodies (APLA). In addition, elevated levels of MMP-9 correlated with SLE' activity in the group of male patients.

### Materials and Methods

***Patients*.** Forty patients, 32 females and 8 males with SLE participated in this study. All patients revealed at least four of the revised diagnostic criteria of the American College of Rheumatism (ACR) for the diagnosis of SLE (Winchester RJ. Systemic lupus erythematosus pathogenesis. In: Koopman WJ, ed. Birmingham. Alabama: William and Wilkins,pp. 1361-91 (1996)). Twenty-five sex-and age-matched healthy volunteers served as a control group in our studies. The mean age of patients at diagnosis was 29±9.7 (range 15-48) years and the mean follow-up period was 11±10 (range 1-32) years. Disease activity was determined according to the SLEDAI lupus activity index (Bombardier et al., 1992) and by the BILAG index (Hay E.M. et al., Q. J. Med. 86: 447-58 (1993)). The study was approved by the ethic committee of the Kaplan Medical Center, Rehovot, Israel.

***Measurement of MMP-2 and MMP*-*9 by activit assay kits.*** Activities of MMP-2 and MMP-9 were measured by specific Biotrak MMP-2 or MMP-9 activity assay kits (Amersham Pharmacia Biotech UK Limited, UK) according to the manufacturer's instructions. Sera were diluted 1:100 and 1:32 for the determination of MMP-2 and MMP-9 activities, respectively. The appropriate standards were added in each assay. In order to measure the total content of the MMPs, activation of the pro form of the MMPs was performed using p-aminophenylmercuric acetate (APMA).

***Measurement of MMP-2 and MMP-9 activities by gel zymography.*** MMP-2 and MMP-9 activities were tested by gelatin zymography. A 5µl sample of serum was separated by an 8% SDS-PAGE gel polymerized with 1 mg/ml gelatin. Gels were washed once for 30 min in 2.5% Triton X-100 to remove the SDS, and once for 30 min in the reaction buffer containing 50 mM Tris-HCI, 200 mM NaCl, 10 mM CaCl₂ and 0.02%(w/v) Brij 35 (pH 7.5). The reaction buffer was changed to a fresh one, and the gels were incubated at37°C for 24 h. Gelatinolytic activity was visualized by staining the gels with 0.5% Coomassie brilliant blue and was quantified by densitometry.

***Statistical analyses.*** The data were evaluated using chi-square or Fisher exact tests, unpaired t-test and two tailed P-values. Pearson, Spearman and multivariate analyses were also used.

### Example 18 (i)

### Activity of MMP-9 but not of MMP-2 is elevated in SLE

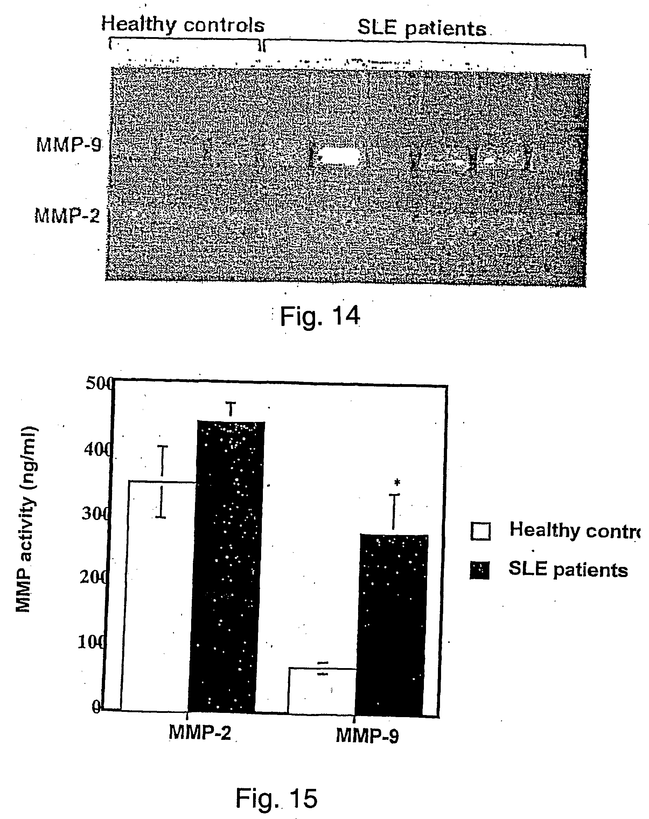

As described above and in PCT International Publication No. WO 02/067848, MMP-9 was shown to be involved in several autoimmune diseases as well as in animal models of SLE. Thus, we were interested in studying whether MMP-9 is also elevated in sera of SLE patients. For this purpose, we examined sera of 40 SLE patients and of 25 healthy controls by gel zymography, in which both MMP-9 and MMP-2 activities can be visualized. A representative gel is shown in Figure 14. As can be seen in this figure, levels of MMP-9 are elevated in the sera of SLE patients when compared to healthy controls. Densitometric analysis of the zymograms of sera of 40 SLE patients and 25 healthy controls indicated that the mean MMP-9 activity for SLE patients was 109±5.6 densitometry units and for the healthy controls, 76.5±4.2 densitometry units (P=0.0001). Activity values of above 85 densitometry units (mean of healthy controls + 2 s.e.) were considered high. The results demonstrated high activity levels of MMP-9 in 68% of the SLE patients. Only 3% of healthy controls exhibited high MMP-9 activity (P=0.001). Densitometric analysis of MMP-2 levels in the same serum samples revealed that the differences in MMP-2 activity between sera of SLE patients and of healthy controls were not significant. Thus values of 109±7 and of 123±5. (mean activity densitometry units ± s.e.) were determined for healthy controls and SLE patients, respectively (P=0.0531). To quantify the activity levels of MMP-9 and MMP-2 in the serum further, we used activity assay kits.

Figure 15 shows that the activity of MMP-9 is elevated by threefold in sera of SLE patients compared with sera of healthy controls, and this elevation is statistically significant (P=0.0302). In contrast, the differences in the levels of MMP-2 between the two groups are not significant (P=0.1254).

Since we, as well as others (Ebihara I. et al., Am J Kidney Dis 32: 544-50 (1998); Ebihara I. et al., Nephron 83: 169 (1999) detected high MMP-9 levels in sera of patients with non-SLE chronic renal failure (e. g. diabetes mellitus, hypertension) probably due to the retention of the enzyme, we analysed the correlation between levels of MMP-9 and kidney function in the group of SLE patients tested. No correlation was observed between creatinine levels and MMP-9 levels (r²=0.01), indicating that the elevated levels of MMP-9 in SLE patients were not the result of retention of the enzyme due to renal impairment.

### Example 18 (ii)

### Correlation of MMP-9 activity with clinical and laboratory parameters

The elevation in the activity levels of MMP-9 in sera of SLE patients prompted us to look for possible correlation between clinical and laboratory parameters, and serum MMP-9 levels. Statistical analysis (chi-square or Fisher exact tests) was performed by investigating the number of patients with high and normal MMP-9 levels for each clinical manifestation (Table 20) as well as by taking into consideration the actual mean activity levels of MMP-9 for patients with or without a certain clinical symptom. The results were similar by both analyses. It is noteworthy that for all clinical symptoms, the percent of patients with elevated MMP-9 levels is much higher than that in the group of healthy controls. Levels of MMP-9 did not correlate with gender, duration of disease or the age of its onset (Pearson, Spearman).

Table 20 shows the clinical and laboratory characteristics of the SLE patients according to their MMP-9 activity levels (lower or equal to healthy controls=normal). High levels of MMP-9 correlated significantly with the presence of Raynaud phenomenon (P=0.0138) and APLA (P=0.041). A strong correlation could be observed with pneumonitis, discoid rash, neurological disorders and mucosal ulcers. However, the number of patients with the latter manifestations was too small to perform a statistical analysis. Multivariate analysis revealed that Raynaud phenomenon and low complement (C3, C4) levels are positively correlated with high MMP-9 levels (P=0.0001 and 0.0137, respectively). In contrast, photosensitivity, arthritis and hematological disorders are negatively correlated with MMP-9 activity levels (P=0.0381, 0.0014 and 0.0065, respectively).

**Table 20**

| **Clinical characteristics of SLE patients with high and normal MMP-9 activities according to their MMP-9 levels.** | | | |
|---|---|---|---|
| **MMP-9 LEVELS (%)** | | | |
| | | **High** | **Normal** |
| **Number of Patients (%)** | 40(100) | 27 (68) | 13 (32) |
| **Photosensistivity** | 13 | 8 (62) | 5(38) |
| **Mucosal ulcers** | 9 | 8(89) | 1 (11) |
| **Malar rash** | 9 | 7(78) | 2(22) |
| **Discoid rash** | 5 | 5(100) | 0(0) |
| **Raynaud phenomenon** | 8 | 8(100) | 0(0) |
| **Vasculitis** | 18 | 14 (78) | 4 (22) |
| **Arthritis** | 31 | 21 (68) | 10 (32) |
| **Serositis** | 9 | 7(78) | 2(22) |
| **Pneumonitis** | 4 | 4(100) | 0(0) |
| **Neurological disorders** | 4 | 4 (100) | 0(0) |
| **Renal disorder** | 16 | 11(69) | 5(31) |
| **Hematological Disorders** | 29 | 18 (62) | 11 (38) |
| **ANA** | 40 | 27 (68) | 13 (32) |
| **αds-DNA** | 36 | 24 (67) | 12 (33) |
| **APLA** | 25 | 20 (80) | 5 (20) |
| **Low complement (C3,C4)** | 30 | 21 (70) | 9(30) |

Clinical involvement was defined according to the ACR revised criteria (Winchester, 1996). Anti-nuclear antibodies (ANA) and anti-ds DNA antibodies were determined by using Hep2 cells and *Crithidia lucilie*, respectively. Antiphospholipid antibodies (APLA) were defined as reactivity with one or more of the following assays: false positive VDR, lupus anti-coagulant (LAC) or ELISA for anticardiolipin antibodies.

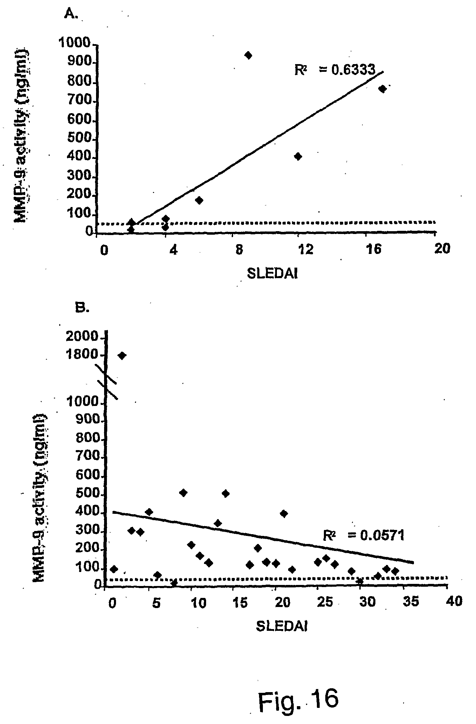

We also looked for a possible correlation between SLEDAI and MMP-9 activity in male (Figure 16A) and female patients (Figure 16B). Interestingly, the correlation coefficient was significant and positive for men (r² = 0.6333) but insignificant and negative for women (r² = 0.0571). Similar results were obtained using the BILAG scoring system. Thus, a positive correlation coefficient between MMP-9 activity and BILAG scores was observed for men (r² = 0.6442) and an insignificant one for women.

It was also of interest to determine whether a correlation exists between the use of various treatment modalities by the patients and MMP-9 activity. As can be seen in Table 21(A), there was no significant correlation between the current treatment of the patients and MMP-9 activity. However, when we looked at treatment of patients at any time during their disease course (Table 21(B)), high MMP-9 levels were associated with usage of cytotoxic agents (82%).

**Table 21**

| **Treatment modalities of SLE patients according to their MMP-9 levels.** | | | |
|---|---|---|---|
| | **Total Number of Patients** | **MMP-9 Levels (%)** | |
| | | **High** | **Normal** |
| **A. Current treatment.** | | | |
| **Cytotoxic agents** | 8 | 6 (75) | 2 (25) |
| **Steroids** | 23 | 17 (74) | 6 (26) |
| **Anti-Malarial** | 21 | 14 (67) | 7 (33) |
| **NSAID** | 7 | 5 (71) | 2 (29) |
| **B. Treatment along the follow up period.** | | | |
| **Cytotoxic agents** | 17 | 14 (82) | 3 (18) |
| **Steroids** | 29 | 19 (66) | 10 (34) |
| **Anti-Malarial** | 26 | 16 (62) | 10 (38) |
| **NSAID** | 18 | 12 (67) | 6 (33) |

The anti-malarial agent hydroxychloroquine was used at dose of 200-400 mg/day. Steroid treatment was defined as a daily dose ≥ mg of prednisone. Cytotoxic agents used were cyclophosphamide (0.5-1g/m² monthly) or azathioprine (100-150 mg/day).

### Example 18 (iii)

### Variations in MMP-9 activity in serum samples taken from individual SLE patients at different time points

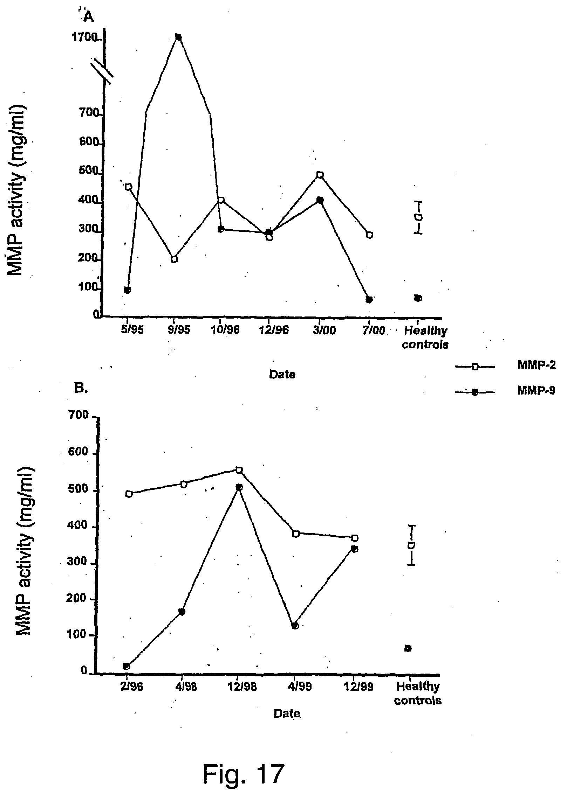

Since disease activity varies over time, we measured the activity levels of MMP-9 and MMP-2 in the serum of individual patients that were sampled during 4-6 years of follow-up. Sera of nine patients taken at different time points were analysed. Levels of MMP-2 did not vary significantly between patients and healthy controls. In 5 out of the 9 patients tested, variations in MMP-9 activity in serum samples of individual patients could be observed with time. The results for 2 representative SLE patients are shown in Figures 17A-B. As can be seen, MMP-9 activity, but not MMP-2 activity, has been changing with time in the same patients. These changes were not associated with disease activity indices as determined by either the SLEDAI or BILAG systems. Changes in MMP-9 activity were not detected in sera of 5 healthy controls that were sampled at different time points (data not shown). In 4 other SLE patients, no substantial changes in MMP-9 or MMP-2 activity were observed with time, and MMP-9 activity levels remained either high or low, depending on the individual patient.

### Discussion

The present study demonstrates for the first time the involvement of MMP-9 in human SLE. We show that the activity of MMP-9, but not MMP-2, is significantly elevated in sera of 68% of SLE patients compared with healthy controls. High MMP-9 levels correlated with Raynaud phenomenon, pneumonitis, neurological disorders, discoid rash and the presence of APLA. Changes in MMP-9 activity were observed in serum of the same patient at different periods of the disease. MMP-9 activity levels did not correlate with disease activity index (SLEDAI, BILAG) in female patients, but correlated with SLE activity in the group of male patients.

The present study shows that activity levels of MMP-2 are not elevated significantly in sera of SLE patients. These results are compatible with those reported previously (Zucker, S. J Rheumatol. 26,78 (1999)) that MMP-2 levels were not increased in SLE. Levels of MMP-2 were also constitutive and unchanged in other pathological conditions (like optic neuritis and multiple sclerosis) in which levels of MMP-9 were elevated relatively to the healthy controls (Gijbels K et al., J. Neuroimmunol. 41: 29-34 (1992).; Paemen, L. et al., Eur. J. Neurol. 1: 55-63 (1994)).

Involvement of an additional MMP, namely, MMP-3 was suggested in the pathogenesis of SLE, since it was significantly increased in sera of patients with SLE (Kotajima, L. et al., Clin. Exp. Rheum. 16: 409-415 (1998)). The frequency of SLE patients with elevated MMP-9 activity (68%) shown in the present example, resembles the frequencies reported (Kotajima et al., 1998) for high MMP-3 levels in SLE (76%) and in RA (82%) patients. Furthermore, the MMP-3 transcript was shown to increase significantly with the progression of nephritis in (NZB x NZW) F1 mice (Nakamura, T. et al., Clin. Sci. 85:295-301 (1993)).

The origin of the elevated MMPs in sera of SLE patients is not known. MMP-9 has been shown to be secreted by peripheral blood cells such as T cells, neutrophils, and macrophages (for review, see Goetzl, E. J. et al., J. Immunol. 156: 1-4 (1996)). The fact that no correlation was found between MMP-9 activity levels and the number of peripheral blood cells in the patients may suggest that MMP-9 was not secreted by peripheral blood immune cells but rather, by SLE-affected organs like kidneys or lungs/pleura. The observation that all SLE patients with pneumonitis exhibited high MMP-9 activity levels may suggest the diseased lung as a source of high MMP-9 levels. Moreover, the association between cytotoxic treatment, which represents the severity of SLE-related organ impairment, and high levels of MMP-9 in the sera may also support the notion that the diseased organs are the source of MMP-9 activity in SLE patients. Nevertheless, the possibility still exists that less peripheral blood lymphocytes secreted higher activity levels of MMP-9.

TNF-α and IL-1 were shown to play an important role in the pathogenesis of SLE both in the human disease (Dean G.S. et al., Ann Rheum Dis 59: 243-51 (2000)) and in murine models (Segal R. et al., J Immunol 158: 3009-16 (1997); Theofilopoulos A.N. et al., Ann Rheum Dis 58 (suppl): 149-55 (1999); Eilat et al., 2001). It has been shown in several systems that these cytokines induce MMP-9 production (Guedez, L. et al., Crit. Rev. Oncogenesis 7: 205-225 (1996)), and thus, it is possible that the induction of the latter MMPs is part of the pathogenic effect of these cytokines in SLE. It has been reported that levels of MMP-9, that are secreted spontaneously by peripheral blood monocytes of healthy individuals, were upregulated upon exposureto TNF-α and IL-1β (Saren P. et al., J Immunol 157: 4159-65 (1996)). In addition, MMPs of both T cells and macrophages facilitate secretion of TNF-α by cleavage of the membrane-bound form (Gearing A.J.H. et al., Nature 370:555-7 (1994)). Thus, these examples demonstrate the mutual regulatory effects of MMP on the proinflammatory cytokines and vice versa. Nevertheless, the fact that in the sera of some of the patients the activity levels of MMP-9 remained within the normal range during the follow-up period, whereas high activity levels of MMP-9 were measured in the sera of most patients, may suggest the involvement of genetic factors in the regulation of the latter.

The results herein indicate that MMP-9 might play a role in the pathogenesis of SLE, and that measurement of plasma/serum activity levels of this metalloproteinase may provide important information when monitoring patients treated with drugs that interfere with MMP-9 activity.

## Claims

1. A pharmaceutical composition comprising:
an aqueous carrier;
from 0.1 mg/ml to 20 mg/ml of the composition of a pharmaceutically acceptable salt of
a peptide comprising consecutive amino acids having the sequence
(i) TGYYX₁X₂X₃X₄X₅QSPEKSLEWIG (SEQ ID NO:11) wherein X₁ is Met, Ala or Val; X₂ is Gln, Asp, Glu or Arg; X₃ is Trp or Ala; X₄ is Val or Ser; and X₅ is Lys, Glu or Ala;
(ii) EINPSTGGX₆X₇X₈X₉X₁₀X₁₁X₁₂KAKAT (SEQ ID NO:12) wherein X₆ and X₇ are each Thr, Val or Ala; X₈ is Tyr or Phe; X₉ is Asn or Asp; X₁₀ is Gln or Glu; X₁₁ is Lys or Glu, and X₁₂ is Phe or Tyr;
(iii) YYCARX₁₃X₁₄X₁₅X₁₆PYAX₁₇X₁₈YWGQGS (SEQ ID NO:13) wherein X₁₃ is Phe, Thr or Gly; X₁₄ is Leu, Ala or Ser; X₁₅ is Trp or Ala; X₁₆ is Glu or Lys; X₁₇ is Met or Ala, and X₁₈ is Asp, Lys or Ser;
(iv) GYNX₁₉X₂₀X₂₁X₂₂X₂₃X₂₄SHGX₂₅X₂₆LEWIG (SEQ ID NO:14) wherein X₁₉ is Met or Ala; X₂₀ is Asn, Asp or Arg; X₂₁ is Trp or Ala; X₂₂ is Val or Ser; X₂₃ is Lys or Glu; X₂₄ is Gln or Ala; X₂₅ is Lys or Glu, and X₂₆ is Ser or Ala;
(v) YYCARX₂₇X₂₈X₂₉YGX₃₀X₃₁X₃₂GQTL (SEQ ID NO:15) wherein X₂₇ is Ser or Phe; X₂₈ is Gly or Ala; X₂₉ is Arg, Ala or Glu; X₃₀ is Asn or Asp; X₃₁ is Tyr or Phe, and X₃₂ is Trp, His or Ala;
(vi) X₃₃YYWSWIX₃₄QX₃₅PX₃₆X₃₇GX₃₈EWIG (SEQ ID NO:16) wherein X₃₃ is Gly or Thr Gly; X₃₄ is Arg or Lys; X₃₅ is Pro or Ser; X₃₆ is Gly or Glu; X₃₇ is Lys or Asp; and X₃₈ is Glu, Leu or Ser;
(viii) FSGYYWS (SEQ ID NO:8);
(ix) EINHSGSTNYKTSLKS (SEQ ID NO:9); or
(x) GLLRGGWNDVDYYYGMDV (SEQ ID NO:10), and
a substituted β-cyclodextrin as a solubility enhancer selected, and,
wherein both the peptide and the solubility enhancer are dissolved in the aqueous carrier; and
wherein the composition has a pH between 4 and 9.

2. The pharmaceutical composition of claim 1, wherein at least 0.5 mg/ml of the composition is the pharmaceutically acceptable salt of the peptide.

3. The pharmaceutical composition of claim 1 or 2, wherein the peptide has a sequence selected from the group consisting of:
NH₂- Thr Gly Tyr Tyr Met Gln Trp Val Lys Gln Ser Pro Glu Lys Ser Leu Glu-Trp Ile Gly-COOH (SEQ ID NO:1) ;
NH₂- Glu Ile Asn Pro Ser Thr Gly Gly Thr Thr Tyr Asn Gln Lys Phe Lys Ala Lys Ala Thr-COOH (SEQ ID NO:2) ;
NH₂- Tyr Tyr Cys Ala Arg Phe Leu Tip Glu Pro Tyr Ala Met Asp Tyr Trp Gly Gln Gly Ser-COOH (SEQ ID NO:3) ;
NH₂- Gly Tyr Asn Met Asn Trp Val Lys Gln Ser His Gly Lys Ser Leu Glu Trp Ile Gly-COOH (SEQ ID NO:4) ;
NH₂- Tyr Tyr Cys Ala Arg Ser Gly Arg Tyr Gly Asn Tyr Trp Gly Gln Thr Leu - COOH (SEQ ID NO:5) ;
NH₂-Gly Tyr Tyr Trp Ser Trp Ile Arg Gln Pro Pro Gly Lys Gly Glu Glu Trp Ile Gly-COOH (SEQ ID NO:6);
NH₂- Phe Ser Gly Tyr Tyr Trp Ser-COOH (SEQ I D NO:8) ;
NH₂- Glu Ile Asn His Ser Gly Ser Thr Asn Tyr Lys Thr Ser Leu Lys Ser-COOH (SEQ ID NO:9); and
NH₂- Gly Leu Leu Arg Gly Gly Trp Asn Asp Val Asp Tyr Tyr Tyr Gly Met Asp Val-COOH (SEQ ID NO:10).

4. The pharmaceutical composition of claim 1, wherein the peptide comprises consecutive amino acids having the sequence
X₃₃YYWSWIX₃₄QX₃₅PX₃₆X₃₇GX₃₈EWIG (SEQ ID NO:16)
wherein X₃₃ is Gly or Thr Gly; X₃₄ is Arg or Lys; X₃₅ is Pro or Ser; X₃₆ is Gly or Glu; X₃₇ is Lys or Asp; and X₃₈ is Glu, Leu or Ser.

5. The pharmaceutical composition of any one of claims 1-4, wherein the peptide is SEQ ID NO.6.

6. The pharmaceutical composition of any one of claims 1-5, wherein the substituted β-cyclodextrin is a hydroxypropyl, a sulfobutyl ether, or a sulfopropyl ether substituted β-cyclodextrin.

7. The pharmaceutical composition of claim 6, wherein the substituted β-cyclodextrin is a substituted sulfobutyl ether β-cyclodextrin.

8. The pharmaceutical composition of any one of claims 1-7, wherein the concentration of peptide in solution is at least 1 mg/ml.

9. The pharmaceutical composition of any one of claims 1-7, wherein the concentration of peptide in solution is at least 2.5 mg/ml.

10. The pharmaceutical composition of any one of claims 1-9, wherein the composition has a pH between 6.5 and 8.5.

11. The pharmaceutical composition of claim 10, wherein the composition has a pH between 7.5 and 8.5.

12. The pharmaceutical composition of any one of claims 1-11, wherein the pharmaceutically acceptable salt is an acetate salt.

13. The pharmaceutical composition of claim 1, wherein the pharmaceutically acceptable salt is an acetate salt, and the substituted β-cyclodextrin is hepta-(sulfobutyl ether)-β-cyclodextrin.

14. A process for manufacturing the pharmaceutical composition of any one of claims 1-13 comprising the steps of:
a) preparing a solution of a substituted β-cyclodextrin in an aqueous carrier at a predetermined concentration;
b) adding a predetermined amount of a pharmaceutically acceptable salt of
a peptide comprising amino acids having the sequence
(i) TGYYX₁X₂X₃X₄X₅QSPEKSLEWIG (SEQ ID NO:11) wherein X₁ is Met, Ala or Val; X₂ is Gln, Asp, Glu or Arg; X₃ is Trp or Ala; X₄ is Val or Ser; and X₅ is Lys, Glu or Ala;
(ii) EINPSTGGX₆X₇X₈X₉X₁₀X₁₁X₁₂KAKAT (SEQ ID NO:12) wherein X₆ and X₇ are each Thr, Val or Ala; X₈ is Tyr or Phe; X₉ is Asn or Asp; X₁₀ is Gln or Glu; X₁₁ is Lys or Glu, and X₁₂ is Phe or Tyr;
(iii) YYCARX₁₃X₁₄X₁₅X₁₆PYAX₁₇X₁₈YWGQGS (SEQ ID NO:13) wherein X₁₃ is Phe, Thr or Gly; X₁₄ is Leu, Ala or Ser; X₁₅ is Trp or Ala; X₁₆ is Glu or Lys; X₁₇ is Met or Ala, and X₁₈ is Asp, Lys or Ser;
(iv) GYNX₁₉X₂₀X₂₁X₂₂X₂₃X₂₄SHGX₂₅X₂₆LEWIG (SEQ ID NO:14) wherein X₁₉ is Met or Ala; X₂₀ is Asn, Asp or Arg; X₂₁ is Trp or Ala; X₂₂ is Val or Ser; X₂₃ is Lys or Glu; X₂₄ is Gln or Ala; X₂₅ is Lys or Glu, and X₂₆ is Ser or Ala;
(v) YYCARX₂₇X₂₈X₂₉YGX₃₀X₃₁X₃₂GQTL (SEQ ID NO:15) wherein X₂₇ is Ser or Phe; X₂₈ is Gly or Ala; X₂₉ is Arg, Ala or Glu; X₃₀ is Asn or Asp; X₃₁ is Tyr or Phe, and X₃₂ is Trp, His or Ala;
(vi) X₃₃YYWSWIX₃₄QX₃₅PX₃₆X₃₇GX₃₈EWIG (SEQ ID NO:16) wherein X₃₃ is Gly or Thr Gly; X₃₄ is Arg or Lys; X₃₅ is Pro or Ser; X₃₆ is Gly or Glu; X₃₇ is Lys or Asp; and X₃₈ is Glu, Leu or Ser;
(viii) FSGYYWS (SEQ ID NO:8);
(ix) EINHSGSTNYKTSLKS (SEQ ID NO:9); or
(x) GLLRGGWNDVDYYYGMDV (SEQ ID NO:10),
c) adjusting the pH of the solution of step b) until the peptide dissolves in the solution; and
d) if necessary, adjusting the pH of the solution of step c) to a pH of 4-9, thereby manufacturing the pharmaceutical composition.

15. The process of claim 14, wherein the predetermined amount of peptide is such which results in a final concentration of peptide in the pharmaceutical composition of 2.5mg/ml, 2.0mg/ml, 1.0mg/ml, 0.5 mg/ml or 0.1 mg/ml.

16. The process of any one of claims 14 or 15, wherein step b) further comprises mixing the solution for 1 hour.

17. The process of any one of claims 14-16, wherein in step c) the pH is adjusted using HCl or NaOH 1.0N.

18. The process of any one of claims 14-17, further comprising filtering the solution of step d) through a cellulose acetate filter.

19. A process of lyophilizing the pharmaceutical composition of any one of claims 1-13, comprising the steps of:
a) lowering the temperature of the pharmaceutical composition to -40°C;
b) holding the temperature at -40°C for a predetermined time;
c) raising the temperature of the solution to 20°C;
d) holding the temperature at 20°C for a predetermined time; and
e) reducing the pressure and holding the temperature at 20°C for a predetermined time, thereby lyophilizing the pharmaceutical composition.

20. The process of claim 19, wherein
step a) is performed within 2 hours;
step b) is performed within 3 hours;
step c) is performed over 13 hours and at a pressure of 110µbar;
step d) is performed over 13 hours and at a pressure of 110µbar; and
step e) is performed over 5 hours and the pressure is reduced to 10µbar.

21. A process of lyophilizing the pharmaceutical composition of any one of claims 1-13, comprising the steps of:
a) lowering the temperature of the pharmaceutical composition to -45°C;
b) holding the temperature at -45°C for a predetermined time;
c) raising the temperature of the solution to -20°C;
d) raising the temperature of the solution to 25°C; and
e) holding the temperature at 25°C for a predetermined time, thereby lyophilizing the pharmaceutical composition.

22. The process of claim 21, wherein
step a) is performed within 6 hours;
step b) is performed within 3 hours;
step c) is performed over 19 hours and at a pressure of 150µbar;
step d) is performed over 13 hours and at a pressure of 150µbar; and
step e) is performed over 8 hours and at a pressure of 150µbar.

23. A lyophilized pharmaceutical composition prepared by the process of any one of claims 19-22.

24. The lyophilized pharmaceutical composition of claim 23, wherein the water content of the composition is less than 5%.

25. The lyophilized pharmaceutical composition of claim 24, wherein the water content of the composition is less than 4.0%.

26. The lyophilized pharmaceutical composition of claim 25, wherein the water content of the composition is less then 3.5%.

27. Use of the pharmaceutical composition of any of claims 1-13 or 23-26 in the manufacture of a medicament for alleviating symptoms of systemic lupus erythematosus (SLE) in a human subject.

28. The pharmaceutical composition of any one of claims 1-13 or 23-26 for use in treating SLE in a human subject.

29. A packaged pharmaceutical composition comprised of:
a packaging material; and
a predetermined amount of the lyophilized pharmaceutical composition of claim 23.

## Patentansprüche

1. Pharmazeutische Zusammensetzung, umfassend:
einen wässrigen Träger;
von 0,1 mg/ml bis 20 mg/ml der Zusammensetzung eines pharmazeutisch akzeptablen Salzes von
einem Peptid, das aufeinanderfolgende Aminosäuren umfasst, mit der Sequenz
(i) TGYYX₁X₂X₃X₄X₅QSPEKSLEWIG (SEQ ID NO: 11),
worin X₁ Met, Ala oder Val ist; X₂ Gln, Asp, Glu oder Arg ist; X₃ Trp oder Ala ist; X₄ Val oder Ser ist; und X₅ Lys, Glu oder Ala ist;
(ii) EINPSTGGX₆X₇X₈X₉X₁₀X₁₁X₁₂KAKAT (SEQ ID NO: 12),
worin X₆ und X₇ jeweils Thr, Val oder Ala sind; X₈ Tyr oder Phe ist; X₉ Asn oder Asp ist; X₁₀ Gln oder Glu ist; X₁₁ Lys oder Glu ist und X₁₂ Phe oder Tyr ist;
(iii) YYCARX₁₃X₁₄X₁₅X₁₆PYAX₁₇X₁₈YWGQGS (SEQ ID NO: 13), worin X₁₃ Phe, Thr oder Gly ist; X₁₄ Leu, Ala oder Ser ist; X₁₅ Trp oder Ala ist; X₁₆ Glu oder Lys ist; X₁₇ Met oder Ala ist und X₁₈ Asp, Lys oder Ser ist;
(iv) GYNX₁₉X₂₀X₂₁X₂₂X₂₃X₂₄SHGX₂₅X₂₆LEWIG (SEQ ID NO: 14), wobei X₁₉ Met oder Ala ist; X₂₀ Asn, Asp oder Arg ist; X₂₁ Trp oder Ala ist; X₂₂ Val oder Ser ist; X₂₃ Lys oder Glu ist; X₂₄ Gln oder Ala ist; X₂₅ Lys oder Glu ist und X₂₆ Ser oder Ala ist;
(v) YYCARX₂₇X₂₈X₂₉YGX₃₀X₃₁X₃₂GQTL (SEQ ID NO: 15),
wobei X₂₇ Ser oder Phe ist; X₂₈ Gly oder Ala ist; X₂₉ Arg, Ala oder Glu ist; X₃₀ Asn oder Asp ist; X₃₁ Tyr oder Phe ist und X₃₂ Trp, His oder Ala ist;
(vi) X₃₃YYWSWIX₃₄QX₃₅PX₃₆X₃₇GX₃₈EWIG (SEQ ID NO: 16),
wobei X₃₃ Gly oder Thr Gly ist; X₃₄ Arg oder Lys ist; X₃₅ Pro oder Ser ist; X₃₆ Gly oder Glu ist; X₃₇ Lys oder Asp ist; und X₃₈ Glu, Leu oder Ser ist;
(viii) FSGYYWS (SEQ ID NO: 8);
(ix) EINHSGSTNYKTSLKS (SEQ ID NO: 9); oder
(x) GLLRGGWNDVDYYYGMDV (SEQ ID NO: 10) und
ein substituiertes β-Cyclodextrin als ausgewählter Löslichkeitsverbesserer; und
wobei sowohl das Peptid als auch der Löslichkeitsverbesserer in dem wässrigen Träger gelöst sind; und
wobei die Zusammensetzung einen pH-Wert zwischen 4 und 9 hat.

2. Pharmazeutische Zusammensetzung nach Anspruch 1, wobei mindestens 0,5 mg/ml der Zusammensetzung das pharmazeutisch akzeptable Salz des Peptids ist.

3. Pharmazeutische Zusammensetzung nach Anspruch 1 oder 2, wobei das Peptid eine Sequenz hat, ausgewählt aus der Gruppe, bestehend aus:
NH₂- Thr Gly Tyr Tyr Met Gin Trp Val Lys Gln Ser Pro Glu Lys Ser Leu Glu-Trp Ile Gly-COOH (SEQ ID NO:1);
NH₂- Glu Ile Asn Pro Ser Thr Gly Gly Thr Thr Tyr Asn Gln Lys Phe Lys Ala Lys Ala Thr-COOH (SEQ ID NO.2);
NH₂- Tyr Tyr Cys Ala Arg Phe Leu Trp Glu Pro Tyr Ala Met Asp Tyr Trp Gly Gln Gly Ser-COOH (SEQ ID NO:3) ;
NH₂- Gly Tyr Asn Met Asn Trp Val Lys Gln Ser His Gly Lys Ser Leu Glu Trp Ile Gly-COOH (SEQ ID NO:4) ;
NH₂- Tyr Tyr Cys Ala Arg Ser Gly Arg Tyr Gly Asn Tyr Trp Gly Gin Thr Leu - COOH (SEQ ID NO: 5) ;
NH₂-Gly Tyr Tyr Trp Ser Trp Ile Arg Gln Pro Pro Gly Lys Gly Glu Glu Trp Ile Gly-COOH (SEQ ID NO:6) ;
NH₂- Phe Ser Gly Tyr Tyr Trp Ser-COOH (SEQ ID NO:8) ;
NH₂- Glu Ile Asn His Ser Gly Ser Thr Asn Tyr Lys Thr Ser Leu Lys Ser-COOH (SEQ ID NO:9); und
NH₂- Gly Leu Leu Arg Gly Gly Trp Asn Asp Val Asp Tyr Tyr Tyr Gly Met Asp Val-COOH (SEQ ID NO:10).

4. Pharmazeutische Zusammensetzung nach Anspruch 1, wobei das Peptid aufeinanderfolgende Aminosäuren umfasst mit der Sequenz:
X₃₃YYWSWIX₃₄QX₃₅PX₃₆X₃₇GX₃₈EWIG (SEQ ID NO: 16),
wobei X₃₃ Gly oder Thr Gly ist; X₃₄ Arg oder Lys ist; X₃₅ Pro oder Ser ist; X₃₆ Gly oder Glu ist; X₃₇ Lys oder Asp ist; und X₃₈ Glu, Leu oder Ser ist.

5. Pharmazeutische Zusammensetzung nach einem der Ansprüche 1 bis 4, wobei das Peptid SEQ ID NO: 6 ist.

6. Pharmazeutische Zusammensetzung nach einem der Ansprüche 1 bis 5, wobei das substituierte β-Cyclodextrin ein Hydroxypropyl-, ein Sulfobutylether- oder ein Sulfopropylether-substituiertes β-Cyclodextrin ist.

7. Pharmazeutische Zusammensetzung nach Anspruch 6, wobei das substituierte β-Cyclodextrin ein substituiertes Sulfobutylether-β-cyclodextrin ist.

8. Pharmazeutische Zusammensetzung nach einem der Ansprüche 1 bis 7, wobei die Konzentration des Peptids in Lösung mindestens 1 mg/ml ist.

9. Pharmazeutische Zusammensetzung nach einem der Ansprüche 1 bis 7, wobei die Konzentration des Peptids in Lösung mindestens 2,5 mg/ml ist.

10. Pharmazeutische Zusammensetzung nach einem der Ansprüche 1 bis 9, wobei die Zusammensetzung einen pH-Wert zwischen 6,5 und 8,5 aufweist.

11. Pharmazeutische Zusammensetzung nach Anspruch 10, wobei die Zusammensetzung einen pH-Wert zwischen 7,5 und 8,5 aufweist.

12. Pharmazeutische Zusammensetzung nach einem der Ansprüche 1 bis 11, wobei das pharmazeutisch akzeptable Salz ein Acetatsalz ist.

13. Pharmazeutische Zusammensetzung nach Anspruch 1, wobei das pharmazeutisch akzeptable Salz ein Acetatsalz ist, und das substituierte β-Cyclodextrin Hepta-(sulfobutylether)-β-cyclodextrin ist.

14. Verfahren zur Herstellung der pharmazeutischen Zusammensetzung nach einem der Ansprüche 1 bis 13, umfassend die folgenden Schritte:
a) Herstellen einer Lösung eines substituierten β-Cyclodextrins in einem wässrigen Träger in einer festgelegten Konzentration,
b) Zugeben einer festgelegten Menge eines pharmazeutisch akzeptablen Salzes von
einem Peptid, umfassend Aminosäuren mit der Sequenz
(i) TGYYX₁X₂X₃X₄X₅QSPEKSLEWIG (SEQ ID NO: 11),
worin X₁ Met, Ala oder Val ist; X₂ Gln, Asp, Glu oder Arg ist; X₃ Trp oder Ala ist; X₄ Val oder Ser ist; und X₅ Lys, Glu oder Ala ist;
(ii) EINPSTGGX₆X₇X₈X₉X₁₀X₁₁X₁₂KAKAT (SEQ ID NO: 12),
worin X₆ und X₇ jeweils Thr, Val oder Ala sind; X₈ Tyr oder Phe ist; X₉ Asn oder Asp ist; X₁₀ Gln oder Glu ist; X₁₁ Lys oder Glu ist und X₁₂ Phe oder Tyr ist;
(iii) YYCARX₁₃X₁₄X₁₅X₁₆PYAX₁₇X₁₈YWGQGS (SEQ ID NO: 13), worin X₁₃ Phe, Thr oder Gly ist; X₁₄ Leu, Ala oder Ser ist; X₁₅ Trp oder Ala ist; X₁₆ Glu oder Lys ist; X₁₇ Met oder Ala ist und X₁₈ Asp, Lys oder Ser ist;
(iv) GYNX₁₉X₂₀X₂₁X₂₂X₂₃X₂₄SHGX₂₅X₂₆LEWIG (SEQ ID NO: 14), wobei X₁₉ Met oder Ala ist; X₂₀ Asn, Asp oder Arg ist; X₂₁ Trp oder Ala ist; X₂₂ Val oder Ser ist; X₂₃ Lys oder Glu ist; X₂₄ Gln oder Ala ist; X₂₅ Lys oder Glu ist und X₂₆ Ser oder Ala ist;
(v) YYCARX₂₇X₂₈X₂₉YGX₃₀X₃₁X₃₂GQTL (SEQ ID NO: 15),
wobei X₂₇ Ser oder Phe ist; X₂₈ Gly oder Ala ist; X₂₉ Arg, Ala oder Glu ist; X₃₀ Asn oder Asp ist; X₃₁ Tyr oder Phe ist und X₃₂ Trp, His oder Ala ist;
(vi) X₃₃YYWSWIX₃₄QX₃₅PX₃₆X₃₇GX₃₈EWIG (SEQ ID NO: 16),
wobei X₃₃ Gly oder Thr Gly ist; X₃₄ Arg oder Lys ist; X₃₅ Pro oder Ser ist; X₃₆ Gly oder Glu ist; X₃₇ Lys oder Asp ist; und X₃₈ Glu, Leu oder Ser ist;
(viii) FSGYYWS (SEQ ID NO: 8);
(ix) EINHSGSTNYKTSLKS (SEQ ID NO: 9); oder
(x) GLLRGGWNDVDYYYGMDV (SEQ ID NO: 10);
c) Einstellen des pH-Werts der Lösung von Schritt b), bis sich das Peptid in der Lösung löst; und
d) gegebenenfalls Einstellen des pH-Werts der Lösung von Schritt c) auf einen pH-Wert von 4-9, wodurch die pharmazeutische Zusammensetzung hergestellt wird.

15. Verfahren nach Anspruch 14, wobei die festgelegte Menge an Peptid derart ist, dass eine Endkonzentration des Peptids in der pharmazeutischen Zusammensetzung von 2,5 mg/ml, 2,0 mg/ml, 1,0 mg/ml, 0,5 mg/ml oder 0,1 mg/ml erhalten wird.

16. Verfahren nach einem der Ansprüche 14 oder 15, wobei Schritt b) ferner das Mischen der Lösung für 1 Stunde umfasst.

17. Verfahren nach einem der Ansprüche 14 bis 16, wobei in Schritt c) der pH-Wert unter Verwendung von HCl oder NaOH 1,0N eingestellt wird.

18. Verfahren nach einem der Ansprüche 14 bis 17, ferner umfassend das Filtrieren der Lösung aus Schritt d) durch ein Celluloseacetat-Filter.

19. Verfahren zum Lyophilisieren der pharmazeutischen Zusammensetzung nach einem der Ansprüche 1-13, umfassend die folgenden Schritte:
a) Senken der Temperatur der pharmazeutischen Zusammensetzung auf -40°C;
b) Halten der Temperatur auf -40°C für eine festgelegte Zeit;
c) Erhöhen der Temperatur der Lösung auf 20°C;
d) Halten der Temperatur auf 20°C für eine festgelegte Zeit; und
e) Reduzieren des Drucks und Halten der Temperatur auf 20°C für eine festgelegte Zeit, wodurch die pharmazeutische Zusammensetzung lyophilisiert wird.

20. Verfahren nach Anspruch 19, wobei
Schritt a) innerhalb von 2 Stunden durchgeführt wird;
Schritt b) innerhalb von 3 Stunden durchgeführt wird;
Schritt c) über 13 Stunden und bei einem Druck von 110 µbar durchgeführt wird;
Schritt d) über 13 Stunden und bei einem Druck von 110 µbar durchgeführt wird und
Schritt e) über 5 Stunden durchgeführt wird und der Druck auf 10 µbar reduziert wird.

21. Verfahren zum Lyophilisieren der pharmazeutischen Zusammensetzung nach einem der Ansprüche 1 bis 13, umfassend die folgenden Schritte:
a) Senken der Temperatur der pharmazeutischen Zusammensetzung auf -45°C;
b) Halten der Temperatur auf -45°C für eine festgelegte Zeit;
c) Erhöhen der Temperatur der Lösung auf -20°C;
d) Erhöhen der Temperatur der Lösung auf 25°C; und
e) Halten der Temperatur auf 25°C für eine festgelegte Zeit, wodurch die pharmazeutische Zusammensetzung lyophilisiert wird.

22. Verfahren nach Anspruch 21, wobei
Schritt a) innerhalb von 6 Stunden durchgeführt wird;
Schritt b) innerhalb von 3 Stunden durchgeführt wird;
Schritt c) über 19 Stunden und bei einem Druck von 150 µbar durchgeführt wird;
Schritt d) über 13 Stunden und bei einem Druck von 150 µbar durchgeführt wird.
Schritt e) über 8 Stunden und bei einem Druck von 150 µbar durchgeführt wird.

23. Lyophilisierte pharmazeutische Zusammensetzung, die durch das Verfahren nach einem der Ansprüche 19 bis 22 hergestellt wird.

24. Lyophilisierte pharmazeutische Zusammensetzung nach Anspruch 23, wobei der Wassergehalt der Zusammensetzung weniger als 5% beträgt.

25. Lyophilisierte pharmazeutische Zusammensetzung nach Anspruch 24, wobei der Wassergehalt der Zusammensetzung weniger als 4,0% beträgt.

26. Lyophilisierte pharmazeutische Zusammensetzung nach Anspruch 25, wobei der Wassergehalt der Zusammensetzung weniger als 3,5% beträgt.

27. Verwendung der pharmazeutischen Zusammensetzung nach einem der Ansprüche 1 bis 13 oder 23 bis 26 bei der Herstellung eines Arzneimittels zur Linderung der Symptome von systemischem Lupus erythematodes (SLE) bei einem menschlichen Individuum.

28. Pharmazeutische Zusammensetzung nach einem der Ansprüche 1 bis 13 oder 23 bis 26 zur Verwendung bei der Behandlung von SLE bei einem menschlichen Individuum.

29. Verpackte pharmazeutische Zusammensetzung, umfassend:
ein Verpackungsmaterial; und
eine festgelegte Menge der lyophilisierten pharmazeutischen Zusammensetzung von Anspruch 23.

## Revendications

1. Composition pharmaceutique comprenant :
un véhicule aqueux ;
de 0,1 mg/ml à 20 mg/ml de la composition d'un sel pharmaceutiquement acceptable d'un peptide comprenant des acides aminés consécutifs ayant la séquence
(i) TGYYX₁X₂X₃X₄X₅QSPEKSLEWIG (SEQ ID NO: 11) dans laquelle X₁ est Met, Ala ou Val ; X₂ est Gln, Asp, Glu ou Arg ; X₃ est Trp ou Ala ; X₄ est Val ou Ser ; et X₅ est Lys, Glu ou Ala ;
(ii) EINPSTGGX₆X₇X₈X₉X₁₀X₁₁X₁₂KAKAT (SEQ ID NO: 12) dans laquelle X₆ et X₇ sont chacun Thr, Val ou Ala ; X₈ est Tyr ou Phe ; X₉ est Asn ou Asp ; X₁₀ est Gln ou Glu ; X₁₁ est Lys ou Glu, et X₁₂ est Phe ou Tyr ;
(iii) YYCARX₁₃X₁₄X₁₅X₁₆PYAX₁₇X₁₈YWGQGS (SEQ ID NO: 13) dans laquelle X₁₃ est Phe, Thr ou Gly ; X₁₄ est Leu, Ala ou Ser ; X₁₅ est Trp ou Ala ; X₁₆ est Glu ou Lys ; X₁₇ est Met ou Ala, et X₁₈ est Asp, Lys ou Ser ;
(iv) GYNX₁₉X₂₀X₂₁X₂₂X₂₃X₂₄SHGX₂₅X₂₆LEWIG (SEQ ID NO: 14) dans laquelle X₁₉ est Met ou Ala ; X₂₀ est Asn, Asp ou Arg ; X₂₁ est Trp ou Ala ; X₂₂ est Val ou Ser ; X₂₃ est Lys ou Glu ; X₂₄ est Gln ou Ala ; X₂₅ est Lys ou Glu, et X₂₆ est Ser ou Ala ;
(v) YYCARX₂₇X₂₈X₂₉YGX₃₀X₃₁X₃₂GQTL (SEQ ID NO: 15) dans laquelle X₂₇ est Ser ou Phe ; X₂₈ est Gly ou Ala ; X₂₉ est Arg, Ala ou Glu ; X₃₀ est Asn ou Asp ; X₃₁ est Tyr ou Phe, et X₃₂ est Trp, His ou Ala ;
(vi) X₃₃YYWSWIX₃₄QX₃₅PX₃₆X₃₇GX₃₈EWIG (SEQ ID NO: 16) dans laquelle X₃₃ est Gly ou Thr Gly ; X₃₄ est Arg ou Lys ; X₃₅ est Pro ou Ser ; X₃₆ est Gly ou Glu ; X₃₇ est Lys ou Asp ; et X₃₈ est Glu, Leu ou Ser ;
(viii) FSGYYWS (SEQ ID NO: 8) ;
(ix) EINHSGSTNYKTSLKS (SEQ ID NO: 9) ; ou
(x) GLLRGGWNDVDYYYGMDV (SEQ ID NO: 10), et
une β-cyclodextrine en tant qu'adjuvant de solubilité sélectionné, et,
dans laquelle le peptide et l'adjuvant de solubilité sont dissous dans le véhicule aqueux ; et
la composition ayant un pH compris entre 4 et 9.

2. Composition pharmaceutique de la revendication 1, dans lequel au moins 0,5 mg/ml de la composition est le sel pharmaceutiquement acceptable du peptide.

3. Composition pharmaceutique de la revendication 1 ou 2, dans laquelle le peptide a une séquence choisie dans le groupe constitué de :
NH₂- Thr Gly Tyr Tyr Met Gln Trp Val Lys Gln Ser Pro Glu Lys Ser Leu Glu-Trp
Ile Gly-COOH (SEQ ID NO: 1) ;
NH₂- Glu Ile Asn Pro Ser Thr Gly Gly Thr Thr Tyr Asn Gln Lys Phe Lys Ala Lys Ala Thr-COOH (SEQ ID NO: 2) ;
NH₂- Tyr Tyr Cys Ala Arg Phe Leu Trp Glu Pro Tyr Ala Met Asp Tyr Trp Gly Gln Gly Ser-COOH (SEQ ID NO: 3) ;
NH₂- Gly Tyr Asn Met Asn Trp Val Lys Gln Ser His Gly Lys Ser Leu Glu Trp Ile Gly-COOH (SEQ ID NO: 4) ;
NH₂- Tyr Tyr Cys Ala Arg Ser Gly Arg Tyr Gly Asn Tyr Trp Gly Gln Thr Leu - COOH (SEQ ID NO: 5) ;
NH₂-Gly Tyr Tyr Trp Ser Trp Ile Arg Gln Pro Pro Gly Lys Gly Glu Glu Trp Ile Gly-COOH (SEQ ID NO: 6) ;
NH₂- Phe Ser Gly Tyr Tyr Trp Ser-COOH (SEQ ID NO: 8) ;
NH₂- Glu Ile Asn His Ser Gly Ser Thr Asn Tyr Lys Thr Ser Leu Lys Ser-COOH (SEQ ID NO: 9) ; et
NH₂- Gly Leu Leu Arg Gly Gly Trp Asn Asp Val Asp Tyr Tyr Tyr Gly Met Asp Val-COOH (SEQ ID NO: 10).

4. Composition pharmaceutique de la revendication 1, dans laquelle le peptide comprend des acides aminés consécutifs ayant la séquence X₃₃YYWSWIX₃₄QX₃₅PX₃₆X₃₇GX₃₈EWIG (SEQ ID NO: 16) dans laquelle X₃₃ est Gly ou Thr Gly ; X₃₄ est Arg ou Lys ; X₃₅ est Pro ou Ser ; X₃₆ est Gly ou Glu ; X₃₇ est Lys ou Asp ; et X₃₈ est Glu, Leu ou Ser.

5. Composition pharmaceutique de l'une quelconque des revendications 1 à 4, dans laquelle le peptide est SEQ ID NO: 6.

6. Composition pharmaceutique de l'une quelconque des revendications 1 à 5, dans laquelle la β-cyclodextrine substituée est une β-cyclodextrine substituée par hydroxypropyle, éther de sulfobutyle, ou éther de sulfopropyle.

7. Composition pharmaceutique de la revendication 6, dans laquelle la β-cyclodextrine substituée est une β-cyclodextrine substituée par éther de sulfobutyle.

8. Composition pharmaceutique de l'une quelconque des revendications 1 à 7, dans laquelle la concentration de peptide en solution est d'au moins 1 mg/ml.

9. Composition pharmaceutique de l'une quelconque des revendications 1 à 7, dans laquelle la concentration de peptide en solution est d'au moins 2,5 mg/ml.

10. Composition pharmaceutique de l'une quelconque des revendications 1 à 9, la composition ayant un pH compris entre 6,5 et 8,5.

11. Composition pharmaceutique de la revendication 10, la composition ayant un pH compris entre 7,5 et 8,5.

12. Composition pharmaceutique de l'une quelconque des revendications 1 à 11, dans laquelle le sel pharmaceutiquement acceptable est un sel d'acétate.

13. Composition pharmaceutique de la revendication 1, dans laquelle le sel pharmaceutiquement acceptable est un sel d'acétate, et la β-cyclodextrine substituée est l'hepta-(éther de sulfobutyle) -β-cyclodextrine.

14. Procédé de fabrication de la composition pharmaceutique de l'une quelconque des revendications 1 à 13 comprenant les étapes de :
a) préparation d'une solution d'une β-cyclodextrine substituée dans un véhicule aqueux à une concentration prédéterminée ;
b) ajout d'une quantité prédéterminée d'un sel pharmaceutiquement acceptable d'un peptide comprenant les acides aminés ayant la séquence
(i) TGYYX₁X₂X₃X₄X₅QSPEKSLEWIG (SEQ ID NO: 11) dans laquelle X₁ est Met, Ala ou Val ; X₂ est Gln, Asp, Glu ou Arg ; X₃ est Trp ou Ala ; X₄ est Val ou Ser ; et X₅ est Lys, Glu ou Ala ;
(ii) EINPSTGGX₆X₇X₈X₉X₁₀X₁₁X₁₂KAKAT (SEQ ID NO: 12) dans laquelle X₆ et X₇ sont chacun Thr, Val ou Ala ; X₈ est Tyr ou Phe ; X₉ est Asn ou Asp ; X₁₀ est Gln ou Glu ; Xl1 est Lys ou Glu, et X₁₂ est Phe ou Tyr ;
(iii) YYCARX₁₃X₁₄X₁₅X₁₆PYAX₁₇X₁₈YWGQGS (SEQ ID NO: 13) dans laquelle X₁₃ est Phe, Thr ou Gly ; X₁₄ est Leu, Ala ou Ser ; X₁₅ est Trp ou Ala ; X₁₆ est Glu ou Lys ; X₁₇ est Met ou Ala, et X₁₈ est Asp, Lys ou Ser ;
(iv) GYNX₁₉X₂₀X₂₁X₂₂X₂₃X₂₄SHGX₂₅X₂₆LEWIG (SEQ ID NO: 14) dans laquelle X₁₉ est Met ou Ala ; X₂₀ est Asn, Asp ou Arg ; X₂₁ est Trp ou Ala ; X₂₂ est Val ou Ser ; X₂₃ est Lys ou Glu ; X₂₄ est Gln ou Ala ; X₂s est Lys ou Glu, et X₂₆ est Ser ou Ala ;
(v) YYCARX₂₇X₂₈X₂₉YGX₃₀X₃₁X₃₂GQTL (SEQ ID NO: 15) dans laquelle X₂₇ est Ser ou Phe ; X₂₈ est Gly ou Ala ; X₂₉ est Arg, Ala ou Glu ; X₃₀ est Asn ou Asp ; X₃₁ est Tyr ou Phe, et X₃₂ est Trp, His ou Ala ;
(vi) X₃₃YYWSWIX₃₄QX₃₅PX₃₆X₃₇GX₃₈EWIG (SEQ ID NO: 16) dans laquelle X₃₃ est Gly ou Thr Gly ; X₃₄ est Arg ou Lys ; X₃₅ est Pro ou Ser ; X₃₆ est Gly ou Glu ; X₃₇ est Lys ou Asp ; et X₃₈ est Glu, Leu ou Ser ;
(viii) FSGYYWS (SEQ ID NO: 8) ;
(ix) EINHSGSTNYKTSLKS (SEQ ID NO: 9) ; ou
(x) GLLRGGWNDVDYYYGMDV (SEQ ID NO: 10),
c) l'ajustement du pH de la solution de l'étape b) jusqu'à ce que le peptide se dissolve dans la solution ; et
d) si nécessaire, l'ajustement du pH de la solution de l'étape c) à un pH de 4 à 9, de manière à fabriquer la composition pharmaceutique.

15. Procédé de la revendication 14, dans lequel la quantité prédéterminée de peptide est telle qu'elle conduise à une concentration finale de peptide dans la composition pharmaceutique de 2,5 mg/ml, 2,0 mg/ml, 1,0 mg/ml, 0,5 mg/ml ou 0,1 mg/ml.

16. Procédé de l'une quelconque des revendications 14 ou 15, dans lequel l'étape b) comprend en outre le mélange de la solution pendant 1 heure.

17. Procédé de l'une quelconque des revendications 14 à 16, dans lequel, dans l'étape c), le pH est ajusté avec HCl ou NaOH 1,0 N.

18. Procédé de l'une quelconque des revendications 14 à 17, comprenant en outre la filtration de la solution de l'étape d) à travers un filtre d'acétate de cellulose.

19. Procédé de lyophilisation de la composition pharmaceutique de l'une quelconque des revendications 1 à 13, comprenant les étapes de :
a) diminution de la température de la composition pharmaceutique à -40 °C ;
b) maintien de la température à -40 °C pendant une durée prédéterminée ;
c) augmentation de la température de la solution à 20 °C ;
d) maintien de la température à 20 °C pendant une durée prédéterminée ; et
e) réduction de la pression et maintien de la température à 20 °C pendant une durée prédéterminée, de manière à lyophiliser la composition pharmaceutique.

20. Procédé de la revendication 19, dans lequel
l'étape a) est conduite en 2 heures ;
l'étape b) est conduite en 3 heures ;
l'étape c) est conduite en 13 heures et à une pression de 110 µbar ;
l'étape d) est conduite en 13 heures et à une pression de 110 µbar ; et
l'étape e) est conduite en plus de 5 heures et la pression est réduite à 10 µbar.

21. Procédé de lyophilisation de la composition pharmaceutique de l'une quelconque des revendications 1 à 13, comprenant les étapes de :
a) diminution de la température de la composition pharmaceutique à -45 °C ;
b) maintien de la température à -45 °C pendant une durée prédéterminée ;
c) augmentation de la température de la solution à -20 °C ;
d) augmentation de la température de la solution à 25 °C ; et
e) maintien de la température à 25 °C pendant une durée prédéterminée, de manière à lyophiliser la composition pharmaceutique.

22. Procédé de la revendication 21, dans lequel
l'étape a) est conduite en 6 heures ;
l'étape b) est conduite en 3 heures ;
l'étape c) est conduite en 19 heures et à une pression de 150 µbar ;
l'étape d) est conduite en 13 heures et à une pression de 150 µbar ; et
l'étape e) est conduite en 8 heures et à une pression de 150 µbar.

23. Composition pharmaceutique lyophilisée préparée par le procédé de l'une quelconque des revendications 19 à 22.

24. Composition pharmaceutique lyophilisée de la revendication 23, dans laquelle la teneur en eau de la composition est inférieure à 5 %.

25. Composition pharmaceutique lyophilisée de la revendication 24, dans laquelle la teneur en eau de la composition est inférieure à 4,0 %.

26. Composition pharmaceutique lyophilisée de la revendication 25, dans laquelle la teneur en eau de la composition est inférieure à 3,5 %.

27. Utilisation de la composition pharmaceutique de l'une quelconque des revendications 1 à 13 ou 23 à 26 dans la fabrication d'un médicament pour soulager les symptômes de lupus érythémateux disséminé (LED) chez un sujet humain.

28. Composition pharmaceutique de l'une quelconque des revendications 1 à 13 ou 23 à 26 pour utilisation dans le traitement de LED chez un sujet humain.

29. Composition pharmaceutique conditionnée constituée de :
un matériau d'emballage ; et
une quantité prédéterminée de la composition pharmaceutique lyophilisée de la revendication 23.
